Europäisches Patentamt

European Patent Office

Office européen des brevets

(11) Publication number: **0 159 123 B1**

# EUROPEAN PATENT SPECIFICATION

(45) Date of publication of patent specification: **15.01.92**

(51) Int. Cl.⁵: **C12N 15/62**, C12N 15/69, C07K 15/00

(21) Application number: **85301468.6**

(22) Date of filing: **04.03.85**

(54) **Vectors for expressing bovine growth hormone derivatives.**

(30) Priority: **06.03.84 US 586582**
**26.07.84 US 634920**
**31.01.85 US 697090**

(43) Date of publication of application:
**23.10.85 Bulletin 85/43**

(45) Publication of the grant of the patent:
**15.01.92 Bulletin 92/03**

(84) Designated Contracting States:
**AT BE CH DE FR GB IT LI LU NL SE**

(56) References cited:
**EP-A- 0 003 062      EP-A- 0 075 444**
**EP-A- 0 095 361      EP-A- 0 111 814**
**EP-A- 0 131 843      EP-A- 0 154 539**

**Biotechnology, vol, 3, n 2, February 1985,
pages 151-154, London, GB. R. Schoner et al
"Isolation and purification of protein gran-
ules from Escherichia coli cells overproduc-
ing bovine growth hormone"**

**proceedings of the National Academy of
Sciences USA, Vol. 81, n 17, September
1984, pages 5403-5407, US. R. Schoner et al**

**"Role of mRNA translational efficiency in
bovine growth hormone expression in Es-
cherichia coli"**

(73) Proprietor: **ELI LILLY AND COMPANY
Lilly Corporate Center
Indianapolis Indiana 46285(US)**

(72) Inventor: **Hsiung, Hansen Maxwell
108 West 88th Street
Indianapolis Indiana 46260(US)**
Inventor: **Schoner, Ronald George
274 Larkspur Court
Zionsville Indiana 46077(US)**
Inventor: **Schoner, Brigitte Elisabeth
274 Larkspur Court
Zionsville Indiana 46077(US)**

(74) Representative: **Hudson, Christopher Mark et
al
Erl Wood Manor
Windlesham Surrey GU20 6PH(GB)**

**Description**

The present invention relates to a novel selectable and autonomously replicating recombinant DNA expression vector which comprises 1) a transcriptional and translational activating nucleotide sequence which is in the reading frame of a sequence that codes for a bioactive derivative of bovine growth hormone (bGH) and 2) a replicon which, under induced conditions, loses copy number control. The invention further relates to novel transformants of the aforementioned vectors, bovine growth hormone derivatives and methods of use.

The development and exploitation of recombinant DNA technology for the production of bGH has been severely handicapped by problems of gene expression. Some of these problems, detailed in European Patent Application Publication No. 0075444, involve the transcription of functionally suboptimal mRNA. Such mRNA has stem and loop secondary configurations that interfere with normal ribosome binding and thus drastically reduce or even prevent bGH expression. The present invention circumvents this problem by providing vectors for the high level expression of bGH derivatives.

For purposes of the present invention as disclosed and claimed herein, the following terms are as defined below.

Recombinant DNA Cloning Vector - any autonomously replicating agent, including but not limited to plasmids, comprising a DNA molecule to which one or more additional DNA segments can be or have been added.

Recombinant DNA Expression Vector - any recombinant DNA cloning vector into which one or more transcriptional and translational activator sequence(s) have been incorporated.

Transcriptional Activating Sequence - any DNA sequence that directs or provides for the transcription of DNA into a mRNA transcript.

Translational Activating Sequence - any DNA sequence that directs or provides for the translation of a mRNA transcript into a peptide or polypeptide.

Translational Start Signal - any DNA triplet that codes for a translational start codon.

Translational Stop Signal - any DNA triplet that codes for a translational stop codon.

Transformation - the introduction of DNA into a recipient host cell that changes the genotype.

Transformant - a recipient host cell that has undergone transformation.

Restriction Fragment - any linear DNA sequence generated by the action of one or more restricton enzymes.

Replicon - any DNA sequence that controls the replication of recombinant DNA cloning and expression vectors.

Runaway Replicon - a replicon which lacks or can be induced to lose copy number control, such loss resulting in the uncontrolled replication and an extreme increase in the copy number of DNA into which such replicon has been incorporated.

Functional Polypeptide - a recoverable bioactive heterologous polypeptide or precursor, a recoverable bioactive polypeptide comprising a heterologous polypeptide and a portion or whole of a homologous polypeptide, or a recoverable bioinactive fusion polypeptide comprising a heterologous polypeptide and a bioinactivating polypeptide which can be specifically cleaved.

Fused Gene Product - a recoverable heterologous polypeptide which comprises a portion or whole of a homologous polypeptide.

Description Of The Figures

Figures 1-4 - Schematic illustration of the construction protocol for plasmid pNM575.
Figures 5-8 - Schematic illustration of the construction protocol for plasmid pNM789B.
Figure 9 - Restriction site map of plasmids pCZ1920 and pJR1.
Figure 10 - Restriction site map of plasmid pCZ112.
Figure 11 - Thymosin alpha 1 synthetic gene.
Figure 12 - Synthesis protocol for nucleotide fragment T15.
Figure 13 - Construction protocol for plasmid pThαI.
Figure 14 - Proinsulin synthetic gene.
Figure 15 - Construction protocol for plasmid pHI7△4△1.
Figure 16 - Construction protocol for plasmid pHI104.

Detailed Description Of The Invention

The present invention provides a selectable and autonomously replicating recombinant DNA expression vector which comprises

a) a runaway replicon and

b) a transcriptional and translational activating sequence which is in the reading frame of a sequence that codes for a bioactive bovine growth hormone derivative, said sequence being

```
(1)   5'   ATG AAA GGG AAT TCT ATG GCC TTC
            ||| ||| ||| ||| ||| ||| ||| |||
      3'   TAC TTT CCC TTA AGA TAC CGG AAG

           CCA GCC ATG TCC TTG TCC GGC           3'
           ||| ||| ||| ||| ||| ||| |||-R-R¹
           GGT CGG TAC AGG AAC AGG CCG           5',

(2)   5'   ATG GAT GAT AAG TTT CCG GCT ATG
            ||| ||| ||| ||| ||| ||| ||| |||
      3'   TAC CTA CTA TTC AAA GGC CGA TAC

           TCT CTG TCC GGC           3'
           ||| ||| ||| |||-R-R¹
           AGA GAC AGG CCG           5',

(3)   5'   ATG TTT CCA GCC ATG GCT CTA
            ||| ||| ||| ||| ||| ||| |||
      3'   TAC AAA GGT CGG TAC CGA GAT

           TCT GGT           3'
           ||| |||-R-R¹
           AGA CCA           5',

(4)   5'   ATG TTC CCA GCT ATG TCT CTA
            ||| ||| ||| ||| ||| ||| |||
      3'   TAC AAG GGT CGA TAC AGA GAT

           TCT GGT           3'
           ||| |||-R-R¹
           AGA CCA           5',

(5)   5'   ATG GAT TTT CCG GCT ATG TCT
            ||| ||| ||| ||| ||| ||| |||
      3'   TAC CTA AAA GGC CGA TAC AGA

           CTG TCC GGC           3'
           ||| ||| |||-R-R¹
           GAC AGG CCG           5',

(6)   5'   ATG TTT CCA GCT ATG TCT CTA
            ||| ||| ||| ||| ||| ||| |||
      3'   TAC AAA GGT CGA TAC AGA GAT

           TCT GGT           3'
           ||| |||-R-R¹
           AGA CCA           5'
```

3

```
(7)   5'  ATG GTT TTT CCG GCT ATG TCT
          ''' ''' ''' ''' ''' ''' '''
      3'  TAC CAA AAA GGC CGA TAC AGA

          CTG TCC GGC            3'
          ''' ''' '''-R-R¹
          GAC AGG CCG            5'
```

or

```
(8)   5'  ATG GCT TTT CCG GCT ATG TCT
          ''' ''' ''' ''' ''' ''' '''
      3'  TAC CGA AAA GGC CGA TAC AGA

          CTG TCC GTC            3'
          ''' ''' '''-R-R¹
          GAC AGG CAG            5'
```

wherein

A is deoxyadenyl,
G is deoxyguanyl,
C is deoxycytidyl,
T is thymidyl,
R is a DNA sequence that codes for amino acids 9 (leucine) through 191 (phenylalanine) of bovine growth hormone, and
R¹ is a DNA sequence that codes for a translational stop signal which is

```
          5' TAA  3'    5' TAG  3'
             '''          '''
          3' ATT  5',   3' ATC  5',
```

or

```
          5' TGA  3'
             '''
          3' ACT  5'    .
```

The invention further relates novel transformants of the aforementioned vectors, and methods of use.

The present invention can be constructed by independently ligating the following XbaI-HgiAI DNA linker sequences

```
a)  5'   CTAGAGGGTATTAATA ATG AAA GGG AAT TCT ATG
         !!!!!!!!!!!!!!! !!! !!! !!! !!! !!!
             TCCCATAATTAT TAC TTT CCC TTA AGA TAC


         GCC TTC CCA GCC ATG TCC TTG TCC GGC CTG
         !!! !!! !!! !!! !!! !!! !!! !!! !!! !!!
         CGG AAG GGT CGG TAC AGG AAC AGG CCG GAC


         TTT GCC AAC GCT GTGCT      3'
         !!! !!! !!! !!! !
         AAA CGG TTG CGA C          5',
                                              .

b)  5'   CTAGAGGGTATTAATA ATG TTT CCA GCC ATG GCT
         !!!!!!!!!!!!!!! !!! !!! !!! !!! !!!
    3'       TCCCATAATTAT TAC AAA GGT CGG TAC CGA


         CTA TCT GGT CTG TTT GCC AAC GCT GTGCT    3'
         !!! !!! !!! !!! !!! !!! !!! !!! !
         GAT AGA CCA GAC AAA CGG TTG CGA C         5'


c)  5'   CTAGAGGGTATTAATA ATG TTT CCA GCT ATG TCT
         !!!!!!!!!!!!!!! !!! !!! !!! !!! !!!
    3'       TCCCATAATTAT TAC AAA GGT CGA TAC AGA


         CTA TCT GGT CTG TTT GCC AAC GCT GTGCT    3'
         !!! !!! !!! !!! !!! !!! !!! !!! !
         GAT AGA CCA GAC AAA CGG TTG CGA C         5',
```

into the ~10.2 kb BamHI-XbaI and ~0.6 kb BamHI-HgiAI fragments of plasmid pCZ101. The resultant plasmids, respectively designated as plasmids pCZ1920, pJR1 and pAT2, contain 1) the E. coli lipoprotein gene transcriptional and translational activating sequence (Nakamura and Inouye, 1979, Cell 18:1109) in the reading frame of a coding sequence for a bioactive bovine growth hormone derivative; 2) an appropriately placed translational stop signal; and 3) a runaway replicon. Cells transformed by plasmids pCZ1920, pJR1 and pAT2 respectively express MET-LYS-GLY-ASPN-SER-MET-ALA-bGH, MET-PHE-PRO-ALA-MET-ALA-$R^2$ and MET-bGH wherein

MET is methionine,
LYS is lysine,
GLY is glycine,
ASPN is asparagine,
SER is serine,
ALA is alanine,
PHE is phenylalanine,
PRO is proline,

bGH is the natural amino acid sequence of bovine growth hormone beginning with the N-terminal (first) phenylalanine

$R^2$ is the natural amino acid sequence of bovine growth hormone beginning with the sixth amino acid (leucine) from the N-terminus.

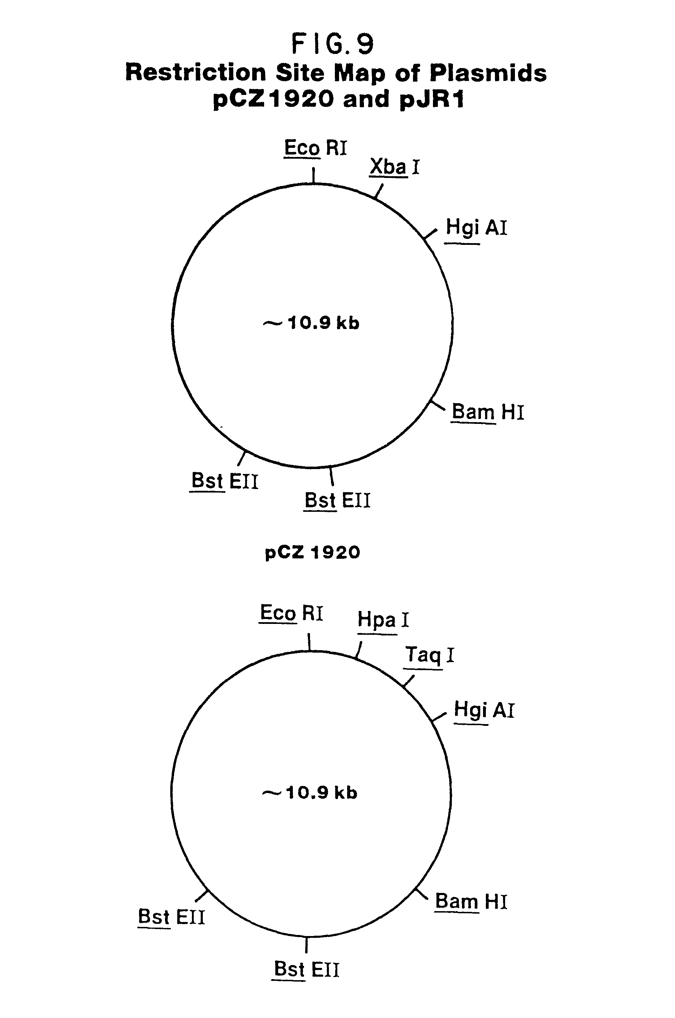

A restriction site map of each of plasmids pCZ1920 and pJR1 is presented in Figure 9 of the accompanying drawings.

Additional plasmids that are also within the scope of the present invention can be constructed by independently ligating the following TaqI-HgiAI DNA linker sequences

5

```
a)  5'   CGACC ATG GAT GAT AAG TTT CCG GCT ATG TCT
    3'        TGG TAC CTA CTA TTC AAA GGC CGA TAC AGA

         CTG TCC GGC CTG TTT GCC AAC GCT GTGCT   3'
         GAC AGG CCG GAC AAA CGG TTG CGA C       5'


b)  5'   CGACA ATG TTC CCA GCT ATG TCT CTA TCT GGT
    3'        TGT TAC AAG GGT CGA TAC AGA GAT AGA CCA

         CTG TTT GCC AAC GCT GTGCT    3'
         GAC AAA CGG TTG CGA C        5',


c)  5'   CGACC ATG GAT TTT CCG GCT ATG TCT CTG TCC
    3'        TGG TAC CTA AAA GGC CGA TAC AGA GAC AGG

         GGC CTG TTT GCC AAC GCT GTGCT     3'
         CCG GAC AAA CGG TTG CGA C         5',


d)  5'   CGATC ATG GAT TTT CCG GCT ATG TCT CTG TCC
             TAG TAC CTA AAA GGC CGA TAC AGA GAC AGG

         GGC CTG TTT GCC AAC GCT GTGCT     3'
         CCG GAC AAA CGG TTG CGA C         5',


e)  5'   CGACC ATG GTT TTT CCG GCT ATG
    3'        TGG TAC CAA AAA GGC CGA TAC

         TCT CTG TCC GGC CTG TTT GCC
         AGA GAC AGG CCG GAC AAA CGG

         AAC GCT GTGCT        3'
         TTG CGA C           5'


f)  5'   CGACC ATG GCT TTT CCG GCT ATG
    3'        TGG TAC CGA AAA GGC CGA TAC

         TCT CTG TCC GTC CTG TTT GCC
         AGA GAC AGG CAG GAC AAA CGG

         AAC GCT GTGCT        3'
         TTG CGA C           5'
```

and

g)    5'    CGATA ATG GAT TTT CCG GCT ATG
      3'          TAT TAC CTA AAA GGC CGA TAC

           TCT CTG TCC GGC CTG TTT GCC
           AGA GAC AGG CCG GAC AAA CGG

           AAC GCT GTGCT          3'
           TTG CGA C              5'

wherein

A is deoxyadenyl,

G is deoxyguanyl,

C is deoxycytidyl and

T is thymidyl,

into the ~10 kb EcoRI-BamHI and ~0.6 kb BamHI-HgiAI fragments of plasmid pCZ101 and the ~0.29 kb EcoRI-ClaI fragment of plasmid pNM608. The resultant plasmids, respectively designated as plasmids pCZ112, pAT1, pASP1, pASP2, pCZ154, pCZ155 and pCZ156, contain 1) the E. coli tryptophan gene transcriptional and translational activating sequence (Hallewell and Emtage, 1980, Gene 9:27) in the reading frame of a coding sequence for a bioactive bovine growth hormone derivative; 2) an appropriately placed translational stop signal; and 3) a runaway replicon. Cells transformed by plasmids pCZ112, pAT1, pASP1, pASP2, pCZ154, pCZ155 and pCZ156 respectively express MET-ASP-ASP-LYS-bGH, MET-bGH, MET-ASP-bGH, MET-ASP-bGH, MET-VAL-bGH, MET-ALA-PHE-PRO-ALA-MET-SER-LEU-SER-VAL-b'GH, and MET-ASP-bGH, wherein

MET is methionine,

ASP is aspartic acid,

LEU is leucine,

SER is serine,

PRO is proline,

PHE is phenylalanine,

LYS is lysine

VAL is valine,

ALA is alanine,

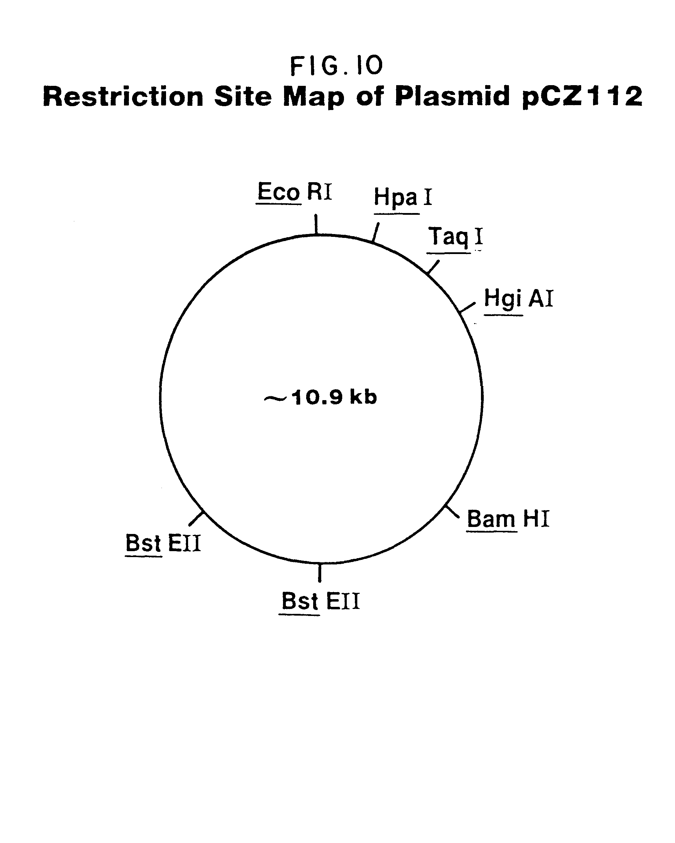

b'GH is the natural amino acid sequence of bovine growth hormone beginning with amino acid 9, leucine, and

bGH is the natural amino acid sequence of bovine growth hormone beginning with the N-terminal (first) phenylalanine. A restriction site map of illustrative plasmid pCZ112 is presented in Figure 10 of the accompanying drawings.

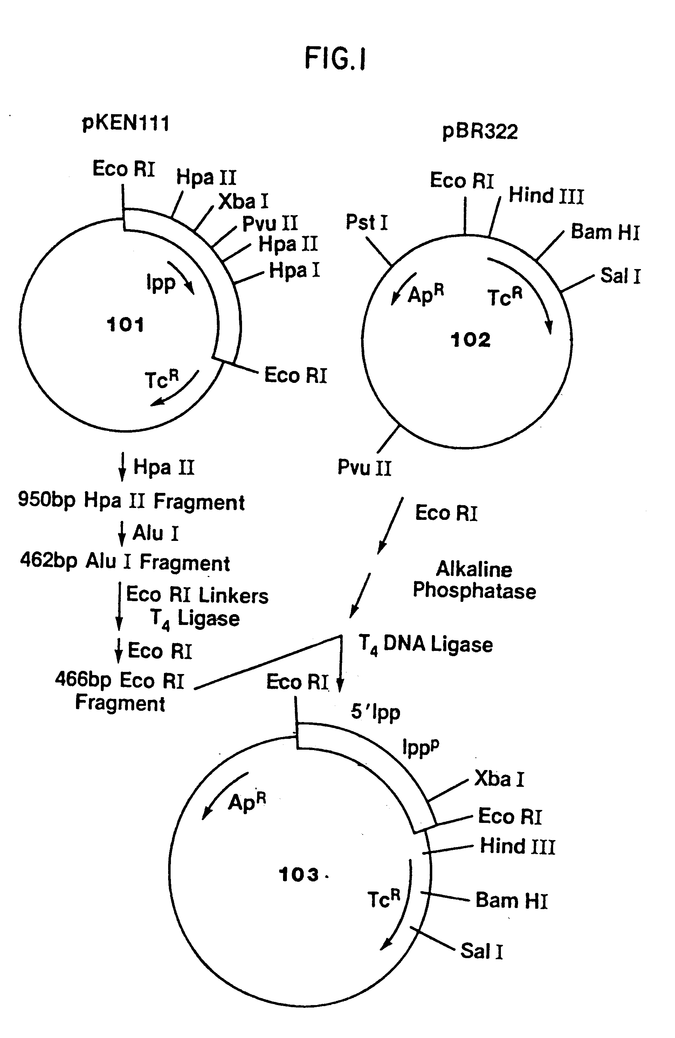

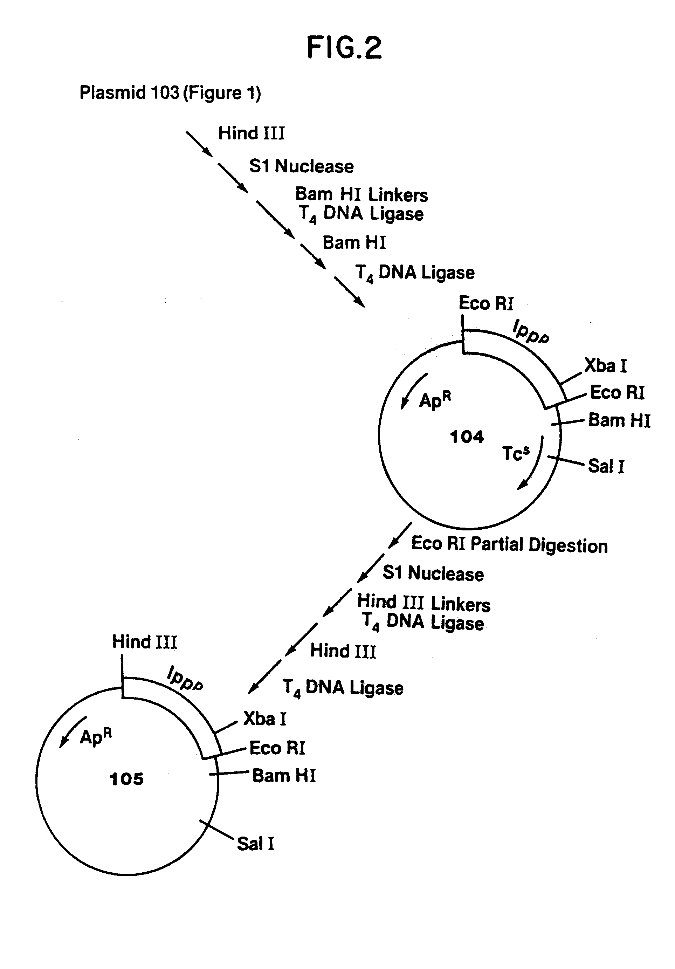

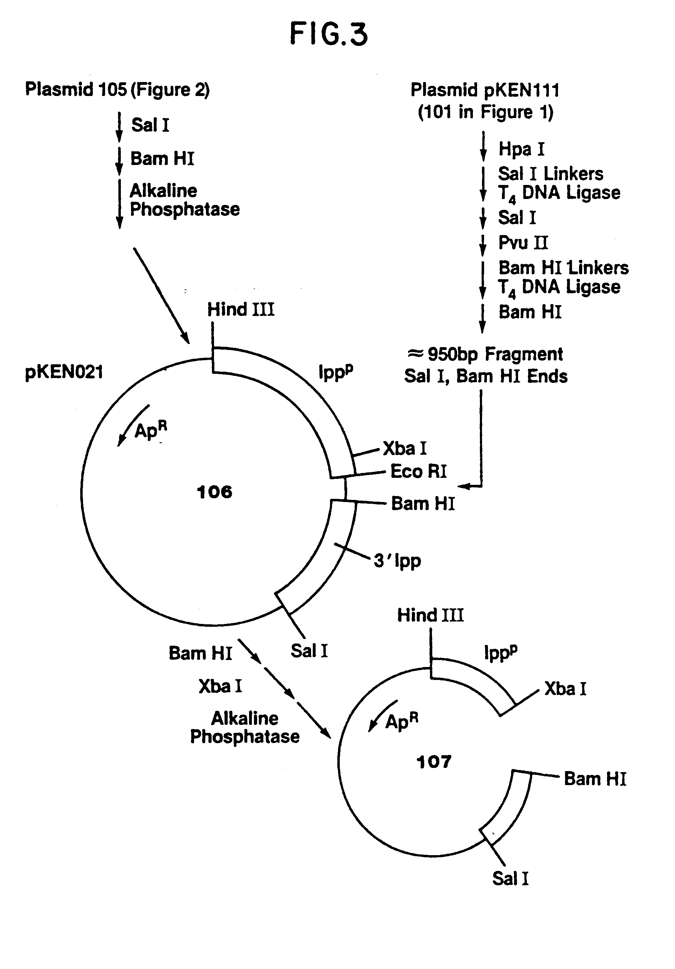

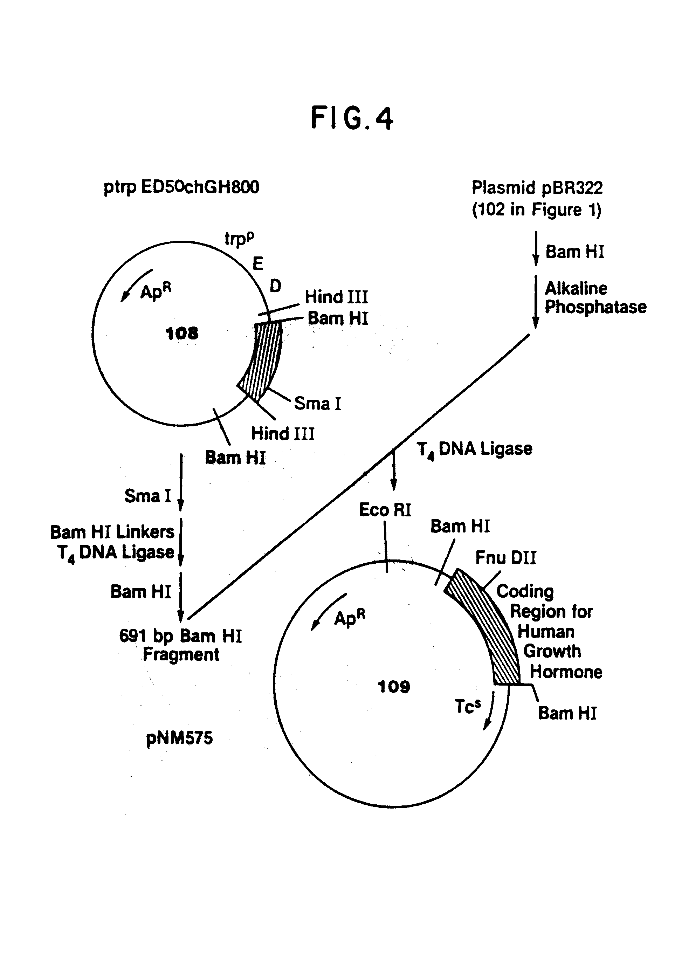

The plasmid pCZ101 starting material is ~10.8 kb and is constructed by ligating the ~0.6 kb XbaI-BamHI fragment of plasmid pNM789B into similarly digested plasmid pIM-I'-A3. The latter plasmid, which contains the transcriptional and translational activating sequence of the E. coli lipoprotein gene as well as a runaway replicon, can be obtained from E. coli K12 RV308/pIM-I'-A3, a strain deposited and made part of the permanent stock culture collection of the Northern Regional Research Laboratory, Peoria, Illinois. The strain is available as a preferred source and stock reservoir of the plasmid under the accession number NRRL B-15733. The plasmid pNM789B starting material is derived from plasmid pKEN111 in accordance with the steps illustrated and described in Figures 1-8 and Example 1 below. Plasmid pKEN111 can be obtained from E. coli K12 CC620/pKEN111, a strain deposited and made part of the permanent stock culture collection of the Northern Regional Research Laboratory, Peoria, Illinois. The strain is available as a preferred source and stock reservoir of the plasmid under the accession number NRRL B-15011. Plasmid pNM789B also contains the transcriptional and translational activating sequence of the E. coli lipoprotein gene and, in addition, the coding sequence, including an appropriately positioned translational stop signal, for a fusion protein comprising bGH and a nine member polypeptide at the bGH N-terminus. Ligation of the fusion protein-coding sequence, contained in the XbaI-BamHI fragment, to appropriately cleaved plasmid pIM-I'-A3 results in the aforementioned plasmid pCZ101 starting material.

The plasmid pNM608 starting material is ~4.6 kb and is constructed by ligating the EcoRI-ClaI fragment of plasmid pHI7Δ4Δ1 into EcoRI-ClaI-digested plasmid pBR322. Plasmid pHI7Δ4Δ1 can be constructed in

7

accordance with the teaching of Example 5 below. Because of the multiplicity of TaqI restriction sites in plasmid pHI7Δ4Δ1, the desired EcoRI-TaqI fragment can best be generated by subcloning the pHI7Δ4Δ1 EcoRI-HpaI and HpaI-TaqI fragments. Plasmid pNM608 contains the E. coli tryptophan transcriptional activating sequence and is thus useful for constructing the present invention.

Those skilled in the art will recognize that the various DNA linkers described above are important components of the present invention. These sequences can be conventionally synthesized by the modified phosphotriester method using fully protected dideoxyribonucleotide building blocks. Such synthetic methods are well known in the art and can be carried out in substantial accordance with the procedure of Itakura et al., 1977, Science 198:1056 and Crea et al., 1978, Proc. Nat. Acad. Sci. USA 75:5765. In addition, an especially preferred method is disclosed in Hsiung et al., 1983, Nucleic Acid Research 11:3227 and Narang et al., 1980, Methods in Enzymology 68:90. The linkers code for a translational activating sequence and also amino acids comprising the first portion (N-terminus region) of the aforementioned bGH derivative compounds. The remainder of the bGH coding sequence (including an appropriately positioned translational stop signal) and also a transcriptional activating sequence can be provided by ligation of the appropriate fragment of plasmid pCZ101. Such ligations result in the illustrative bovine growth hormone derivative expression plasmids of the present invention.

Deletion of the ~900 bp BstEII restriction fragment of plasmid pCZ101, followed by recircularization, results in plasmid pCZ103. The BstEII deletion does not affect the bGH coding region in plasmid pCZ101. Using plasmid pCZ103 in place of plasmid pCZ101 in the above-described constructions thus results in similar, but 900 bp smaller, plasmids. The bGH derivatives expressed by these pCZ103-derived plasmids are therefore identical to the derivatives expressed by their pCZ101-derived counterparts.

Thus, using the ~9.3 kb BamHI-XbaI and ~0.6 kb BamHI-HgiA1 restriction fragments of plasmid pCZ103, in place of the ~10.2 kb BamHI-XbaI and 0.6 kb BamHI-HgiAI restriction fragments of plasmid pCZ101, in the above-described constructions of plasmids pJR1 and pAT2 results in the construction of derivative plasmids pJR1.3 and pAT2.3, respectively. Using the ~9.3 kb BamHI-EcoRI and ~0.6 kb BamHI-HgiA1 restriction fragments of plasmid pCZ103, in place of the ~10.2 kb BamHI-EcoRI and ~0.6 kb BamHI-HgiA1 restriction fragments of plasmid pCZ101, in the above-described constructions of plasmids pAT1, pASP1, pASP2, pCZ154, pCZ155, and pCZ156 results in the construction of derivative plasmids pAT1.3, pASP2.3, pCZ154.3, pCZ155.3, and pCZ156.3, respectively.

The present invention is in no way limited to the use of a particular transcriptional activating sequence since the choice of a specific sequence is not critical to the operability of the present invention. Transcriptional activating sequences which can be substituted for the previously exemplified lipoprotein and tryptophan activating sequences include, but are not limited to, the E. coli lactose (lac), bacteriophage λ $P_LO_L$ and bacteriophage λ $P_RO_R$ transcriptional activating sequences. In addition, one or more transcriptional activating sequences, or parts of them, can be used in tandem, such as, for example, the trp and lac or tac transcriptional activating sequences. All of the aforementioned sequences have been previously characterized and can be constructed either synthetically or from known plasmids.

In the specific embodiments herein described, plasmid replication is determined by a thermoinducible runaway replicon disclosed in both GB Patent Publication Number 1,557,774 and Uhlin et al., 1979, Gene 6:91. At temperatures below 30°C, especially 25°C, the replicon maintains a relatively low copy number of about 10-15 copies per cell. When the temperature is raised to 37°C, copy number control is lost and plasmids containing the replicon amplify to 1000-2000 copies per cell. The particular runaway replicon exemplified herein is contained in the previously described plasmid pIM-I'-A3 starting material. Skilled artisans will understand that the present invention is not limited to the use of any particular runaway replicon or copy number mutant. Other inducible runaway or high copy number replicons can be obtained by appropriate selection or can be constructed in accordance with the procedure disclosed in International Publication Number WO82/02901. Such replicons can be used to construct expression vectors that are also within the scope of the present invention.

The cloning of foreign genes into vectors containing a runaway replicon results, upon induction and loss of copy number control, in a greatly increased rate of protein synthesis and the concomitant formation of a heretofore unknown and unrecognized species of intracellular proteinaceous granule. Such granules, are highly homogeneous in their protein composition and are thus distinguishable over the known high molecular weight aggregates and inclusions that sometimes occur in recombinant DNA-containing host cells. The latter inclusions are heterogeneous, commonly containing an assortment of cellular components, such as nucleic acids, carbohydrates, lipids and peptides, and comprise only 10-20% of a particular protein product. The granules are highly homogeneous with the desired protein product comprising at least 50% and often exceeding 80% of the granule.

The granule can be readily isolated from cell lysates and is stable to washing in low concentrations of urea or detergents. Washing removes proteins that bind non-specifically to the granule. Isolation, which generates high specific activity material, constitutes a useful first step in the purification of foreign proteins. All subsequent steps in purification are therefore simplified. Particularly helpful is the fact that cell wall components can be readily separated from the granules.

It is believed that the formation of the granules sequesters a foreign protein such that disruption of normal cell metabolism and premature cell death does not occur. Some proteins, such as human proinsulin, are rapidly degraded in E. coli and do not accumulate. However, when such proteins are coded for in vectors containing a runaway replicon, the proinsulin protein forms insoluble granules that protect the ordinarily unstable protein from proteolytic degradation. The formation of the present species of granule is thus useful not only for accumulating unstable proteins, but also for simplifying procedures for isolating and purifying gene products.

Transformed cells which exemplify the production of the present species of granule include, but are not limited to, E. coli K12 RV308/pCZ1920, E. coli K12 RV308/pJR1, E. coli K12 RV308/pASP1, E. coli K12 RV308/pCZ112, E. coli K12 RV308/pASP2, E. coli K12 RV308/pAT1, E. coli K12 RV308/pAT2, E. coli K12 RV308/pCZ154, E. coli K12 RV308/pCZ156, E. coli K12 RV308/pJR1.3, E. coli K12 RV308/pASP1.3, E. coli K12 RV308/pASP2.3, E. coli K12 RV308/pAT1.3, E. coli K12 RV308/pAT2.3, E. coli K12 RV308/pCZ154.3, and E. coli K12 RV308/pCZ156.3.

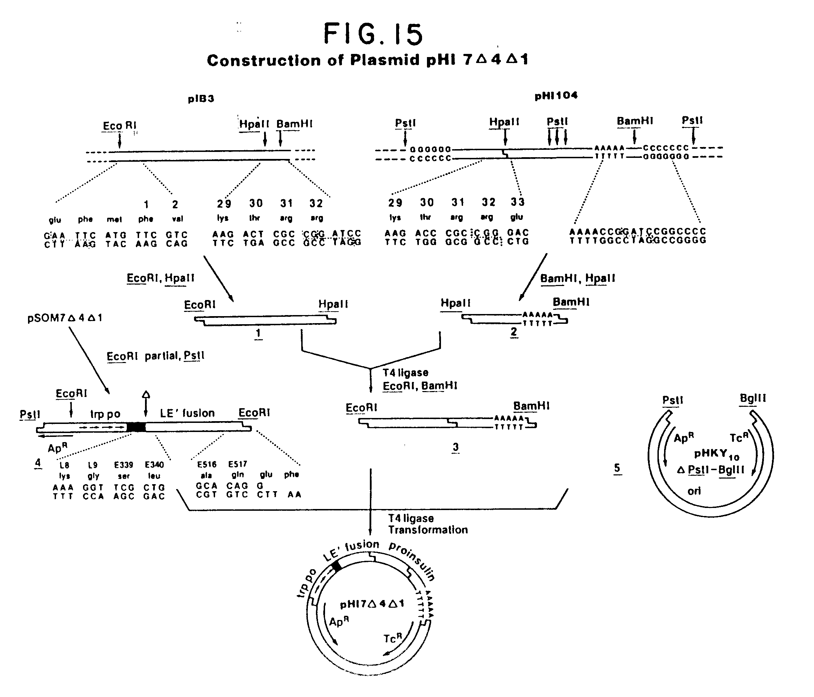

The present method for producing a highly homogeneous species of granule is not limited to the use of the above plasmids coding for bovine or human growth hormone. Human proinsulin granules can also be produced by culturing cells, in accordance with the aforementioned method, that contain vectors comprising the human proinsulin coding sequence. Such a vector can be constructed by ligating a trpLE'-proinsulin fusion sequence-containing EcoRI-BamHI fragment, as depicted in Figure 15, into the large EcoRI-BamHI fragment of plasmid pCZ101. The resultant plasmid, designated as plasmid pCZ201, can transform E. coli, such as E. coli K12 RV308, in accordance with conventional transformation methods. The present granules, which in this case comprise human proinsulin, can then be produced by culturing the E. coli K12 RV308/pCZ201 transformants at 37°C.

Many modifications and variations of the present illustrative DNA sequences and plasmids are possible. For example, the degeneracy of the genetic code allows for the substitution of nucleotides throughout polypeptide coding regions as well as for the substitution of the

$$\begin{array}{ccc} TAG & or & TGA \\ ||| & - & ||| \\ ACT & & ACT \end{array}$$

translational stop signals for the

$$\begin{array}{c} TAA \\ ||| \\ ATT \end{array}$$

translational stop signal specifically exemplified. Thus R, as defined hereinabove, can be one of any number of possible DNA sequences that code for amino acids 9 (leucine) through 191 (phenylalanine) of bGH. Such sequences can be deduced from the known amino acid sequence of bGH and can be constructed by following conventional synthetic procedures. However, nucleotide triplets should be chosen in accordance with known principles and extrapolations reviewed in Zuker and Stiegler, 1981, Nucleic Acids Research 9(1):133, to avoid generating complementary bases in mRNA. Hydrogen bonding (pairing) between such complementary bases results in stem and loop configurations and folding that reduce the efficiency of translation. Skilled artisans will understand that all of the modifications and variations disclosed above can be conventionally synthesized in substantial accordance with the synthetic methods previously cited. Therefore, the present invention is in no way limited to the DNA sequences and plasmids specifically exemplified.

The expression vectors and method of this invention can be applied to a wide range of host organisms, for example, gram-negative prokaryotic organisms such as Escherichia coli, E. coli K12, E. coli K12 RV308, E. coli K12 HB101, E. coli K12 C600, E. coli K12 C600 $R_k$-$M_k$-, E. coli K12 RR1, E. coli K12 MM294 and the like. Although all of the embodiments of the present invention are useful, some of the vectors and transformants are preferred. Preferred vectors include pCZ1920, pJR1, pJR1.3, pCZ112, pAT1, pAT1.3, pAT2, pAT2.3, pASP1, pASP1.3, pCZ154, pCZ154.3, pCZ156, pCZ156.3, pASP2.3, and pASP2 and preferred transformants include E. coli K12 RV308/pCZ1920, E. coli K12 RV308/pJR1, E. coli K12 RV308/pJR1.3, E. coli K12 RV308/pCZ112, E. coli K12 RV308/pAT1, E. coli K12 RV308/pAT1.3, E. coli K12 RV308/pAT2, E. coli K12 RV308/pAT2.3, E. coli K12 RV308/pASP1, E. coli K12 RV308/pASP1.3, E. coli K12 RV308/pCZ154, E. coli K12 RV308/pCZ154.3, E. coli K12 RV308/pCZ156, E. coli K12 RV308/pCZ156.3, E. coli K12 RV308/pASP2.3, and E. coli K12 RV308/pASP2. Of this preferred group, plasmids pCZ112, pCZ154, pCZ154.3, pCZ156, pCZ156.3, pASP2.3, and pASP2 and transformants E. coli K12 RV308/pCZ112, E. coli K12 RV308/pCZ154, E. coli K12 RV308/pCZ154.3, E. coli K12 RV308/pCZ156, E. coli K12 RV308/pCZ156.3, E. coli K12 RV308/pASP2.3, and E. coli K12 RV308/pASP2 are especially preferred.

Those skilled in the art will recognize that the expression vectors of this invention are used to transform suitable host organisms such that a bovine growth hormone derivative product is expressed using standard fermentation conditions. The product, expressed as a highly homogeneous granule and isolated by routine methods from the resulting cell lysates is useful for increasing milk production and for generally stimulating growth in cattle. Modes of use, administration and suitable dosages for cattle are known and disclosed in European Patent Office Publication 0085036, pages 7-9, incorporated herein by reference. The following examples further illustrate the invention disclosed herein. Both an explanation of and the actual procedures for constructing the invention are described where appropriate.

Example 1

Construction of Plasmid pNM789B

## A. Construction of Plasmid pKEN021 and the XbaI-BamHI Fragment Thereof

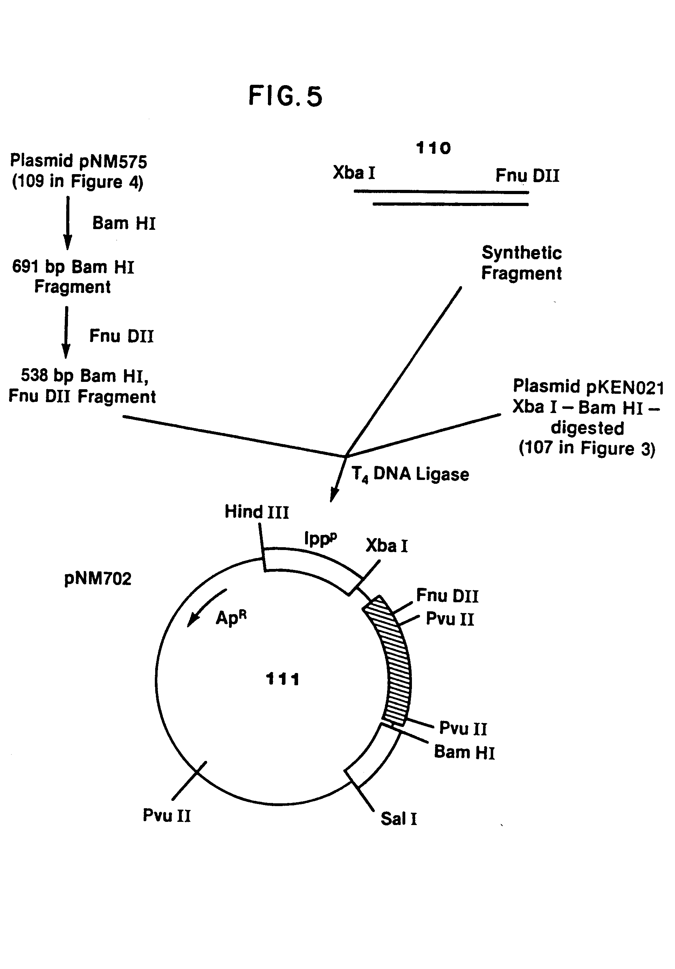

The ~5.1 kb fragment produced by XbaI-BamHI cleavage of plasmid pKEN021 (106 in Figure 3) was used as starting material. Plasmid pKEN021 is a derivative of pKEN111, (101 in Figure 1 and further described in Lee, et al., 1981, J. Bact. 146: 861-866 and Zwiebel, et al., 1981, J. Bact. 145: 654-656), which is on deposit in E. coli CC620 (NRRL Deposit No. 15011) and which has a ~2.8kb fragment which contains the lipoprotein gene of E. coli. A description of this fragment is provided in Nakamura and Inouye, 1979, Cell 18: 1109-1117. In pKEN021, the 650 bp (base pair) sequence between the unique EcoRI and SalI restriction sites of pBR322 has been replaced by sequences taken from the lipoprotein gene of E. coli. The lipoprotein gene sequence (Nakamura and Inouye, 1979) includes a 462 bp AluI fragment, upstream from the first triplet (methionine) of the lipoprotein gene that contains the promoter, the 5' untranslated region and the ribosome binding site. A unique XbaI restriction site is located within the ribosome binding site 16 bp before the translation initiating methionine signal. A PvuII restriction site located 105 bp upstream from the translation termination codon of the structural gene was changed to a BamHI restriction site by the addition of a synthetic DNA linker (5'CCGGATCCGG3', obtained from Collaborative Research). The coding sequence for the last thirty-five amino acids of lipoprotein, the translation termination signal, and the sequence corresponding to the 3' Untranslated region of the messenger RNA follow the BamHI site. Plasmid pKEN021 also includes some 850 bp of extraneous sequences unrelated to the lipoprotein gene and located downstream of it in the E. coli chromosome. These sequences were included as a consequence of the methods and restriction enzyme sites used in the original isolation of the gene.

Referring to Figures 1, 2, and 3, plasmid pKEN021 is derived from pKEN111 in the following manner: About 50 $\mu$g of pKEN111 (101 in Figure 1) are digested with 25 units of HpaII restriction enzyme in 300 $\mu$l of a buffer containing 20mM Tris•HCl, pH 7.4, 10mM MgCl$_2$, and 6mM $\beta$-mercaptoethanol at 37°C for 2 hours. The mixture is extracted twice with 300 $\mu$l of a 50:50 mixture of phenol and chloroform and the recovered aqueous phase is then precipitated with 2.5 volumes of ethanol and 0.1 volumes of 3M sodium acetate. The DNA pellet is dissolved in 100 $\mu$l of electrophoresis buffer and fractionated on a 5 percent polyacrylamide gel (acrylamide:bis ratio is 29:1 in all gels except where noted). The gel is stained in a solution containing 0.5 $\mu$g/ml of ethidium bromide and bands are visualized under long wave-length ultraviolet light. A 950 bp band is isolated and recovered from the gel by electroelution into a dialysis bag. After phenol/CHCl$_3$ extraction and ethanol precipitation, the recovered DNA (approximately 2.5 $\mu$g) is dissolved in 25 $\mu$l of TEN (10mM NaCl, 10mM Tris•HCl pH 7.4 and 1mM sodium

ethylenedinitrilotetraacetate (EDTA), pH 8.0).

About 2 $\mu$g of the 950 bp HpaII fragment are digested with AluI restriction enzyme in 200 $\mu$l of a buffer containing 50mM NaCl, 6mM Tris•HCl (pH 7.6), 6mM MgCl$_2$, and 6mM $\beta$-mercaptoethanol for 2 hours at 37°C. The DNA is fractionated on a 6 percent polyacrylamide gel and the 462 bp AluI fragment generated is recovered and purified by the method previously described. The 462 bp AluI fragment (approximately 1 $\mu$g) is dissolved in 10 $\mu$l of T4 DNA ligase buffer (66mM Tris•HCl pH 7.6, 10mM MgCl$_2$, 10mM dithiothreitol, 0.4mM ATP) containing 150 picomoles of phosphorylated EcoRI linker (5'GGAATTCC3' from Collaborative Research) and 2 units T4 DNA ligase. After incubation at 4°C. for 16 hours, the mixture is heated at 65°C. for 10 minutes and diluted to 100 $\mu$l with the addition of EcoRI buffer (100mM Tris•HCl pH 7.2, 50mM NaCl, 10mM MgCl$_2$, 6mM $\beta$-mercaptoethanol) and 40 units EcoRI enzyme. After 2 hours at 37°C., the sample is conventionally phenol/CHCl$_3$ extracted and ethanol precipitated. The DNA is then dissolved in 20 $\mu$l of T4 DNA ligase buffer containing 0.1 unit T4 DNA ligase and 0.1 $\mu$g pBR322 (102 in Figure 1) which has been linearized with EcoRI and then treated with alkaline phosphatase. After ligation at 4°C for 16 hours, the resultant DNA is used to conventionally transform E. coli strain K12 HB101. Transformants are selected on agar plates containing 12 $\mu$g/ml of tetracycline and plasmids isolated from resistant colonies by the rapid alkaline extraction procedure described in Birnboim and Doly, 1979, Nucleic Acids Research 7: 1513-1523. A plasmid (103 in Figure 1) containing a 466 bp XbaI-BamHI fragment is selected and used as the starting material for the step next described.

About two $\mu$g of this plasmid (103 in Figure 2) are digested with 2 units of HindIII enzyme in 50 $\mu$l HindIII buffer (60mM NaCl, 10mM Tris•HCl, pH 7.4, 10mM MgCl$_2$ and 6mM $\beta$-mercaptoethanol) for 1 hour at 37°C. After phenol/CHCl$_3$ extraction and ethanol precipitation, the DNA is dissolved in 200 $\mu$l of a buffer containing 300mM NaCl, 30mM sodium acetate pH 4.25, 1mM ZnCl$_2$ and 200 units of S1 nuclease (Miles Laboratories). After 1 hour at 15°C., the reaction is stopped by phenol/CHCl$_3$ extraction and ethanol precipitation. The resultant DNA is dissolved in 10 $\mu$l T4 DNA ligase buffer containing 20 picomoles phosphorylated BamHI linkers (5'CCGGATCCGG3', from Collaborative Research) and 2 units T4 DNA ligase. After 16 hours at 4°C, the reaction mixture is heated at 65°C for 10 minutes to inactivate the ligase and then diluted to 100 $\mu$l in BamHI buffer (150mM NaCl, 20mM Tris•HCl, pH 8.0, 10mM MgCl$_2$, 6mM $\beta$-mercaptoethanol) containing 20 units BamHI enzyme. After 2 hours at 37°C, the mixture is purified on a 1 percent agarose gel. The gel is stained and the larger fragment (~4.5 kb) is recovered by elution after freezing and then purified by phenol/CHCl$_3$ extraction and ethanol precipitation. The recovered fragment with BamHI cohesive ends is dissolved in 20 $\mu$l of T4 DNA ligase buffer containing 0.1 unit T4 DNA ligase. After 16 hours at 4°C, the DNA is used to transform E. coli HB101. Transformants are selected by resistance to ampicillin (Ap$^r$) at 100 $\mu$g/ml and screened for sensitivity to 10 $\mu$g/ml tetracycline (Tc$^s$). Several plasmids, prepared by the previously described Birnboim procedure from colonies which are Ap$^r$Tc$^s$, are examined for the absence of a HindIII site and the presence of a single BamHI site. EcoRI, SalI sequential digestion yields a 466 bp and a 305 bp fragment. A plasmid (104 in Figure 2) with these characteristics is selected and then modified to convert the EcoRI site, located up-stream of the lpp promoter, to a HindIII restriction site.

Two micrograms of plasmid (104 in Figure 2) are digested in 100 $\mu$l of EcoRI buffer with 0.2 units of EcoRI for 10 minutes at 37°C. The reaction is stopped by heating for 10 minutes at 65°C and then, after phenol/CHCl$_3$ extraction, the DNA is ethanol precipitated, dissolved in 200 $\mu$l of S1 nuclease buffer containing S1 nuclease at 1000 units/ml and reacted at 12°C for 1 hour. The reaction is stopped by phenol/CHCl$_3$ extraction and ethanol precipitation. The resultant DNA is resuspended in 10 $\mu$l of T4 DNA ligase buffer containing 20 picomoles phosphorylated HindIII linker (5'CCAAGCTTGG3', from Collaborative Research) and 2 units of T4 DNA ligase. After 16 hours at 4°C, the mixture is heated for 10 minutes at 65°C, diluted to 150 $\mu$l in HindIII buffer containing 10 units HindIII enzyme, incubated for 2 hours at 37°C and then fractionated on a 1 percent agarose gel. The largest band (equivalent to single cut plasmid) is conventionally recovered and purified, dissolved in 20 $\mu$l T4 ligase buffer containing 0.2 units T4 ligase, incubated 16 hours at 4°C. and then used to transform E. coli HB101. Transformants are selected for ampicillin resistance and plasmid isolates conventionally analyzed by restriction enzyme analysis. A plasmid (105 in Figure 2) with an EcoRI-HindIII fragment of 500 bp is selected and used as the cloning vector for addition of the 3' region of the lpp gene.

About two $\mu$g of plasmid (105 in Figure 3) are digested in 50 $\mu$l of SalI restriction buffer (150mM NaCl, 6mM Tris•HCl, pH 7.9, 6mM MgCl$_2$, 6mM $\beta$-mercaptoethanol) with 2 units of SalI for 1 hour at 37°C and then diluted to 150 $\mu$l in BamHI buffer containing 2 units BamHI. After 1 hour at 37°C, 2.5 units of alkaline phosphatase are added and then incubation is continued for 1 hour at 65°C. The material is phenol/CHCl$_3$ extracted, ethanol precipitated, dissolved in TEN, and used as a cloning vector for the lpp 3' fragment.

To obtain the fragment containing the lpp 3' region, 10 $\mu$g of pKEN111 (101 in Figure 3) are digested in

200 $\mu$l of HpaI buffer (20mM KCl, 10mM Tris•HCl, pH 7.4, 10mM MgCl$_2$ and 6mM $\beta$-mercaptoethanol) with 10 units of HpaI for 2 hours at 37°C. After phenol/CHCl$_3$ extraction and ethanol precipitation, the DNA is dissolved in 10 $\mu$l T4 DNA ligase buffer containing 20 picomoles phosphorylated SalI linker (5'GGTCGACC3', from Collaborative Research) and 2 units T4 DNA ligase and then incubated for 16 hours at 4°C. The ligase is inactivated by heating at 65°C for 10 minutes. The resultant material is diluted to 100 $\mu$l in SalI buffer containing 10 units of SalI and incubated 1 hour at 37°C, and then diluted to 300 $\mu$l in PvuII buffer (60mM NaCl, 6mM Tris•HCl, pH 7.5, 6mM MgCl$_2$, 6mM $\beta$-mercaptoethanol) containing 10 units PvuII restriction enzyme. After 1 hour at 37°C, the DNA is fractionated on a 5 percent polyacrylamide gel. Approximately 0.5 $\mu$g of a 950 bp fragment is recovered, purified and dissolved in TEN. Two-tenths microgram of the fragment is diluted into 20 $\mu$l T4 DNA ligase buffer containing 20 picomoles phosphorylated BamHI linker (5'CCGGATCCGG3', from Collaborative Research) and 2 units T4 DNA ligase and then incubated for 16 hours at 4°C. The resultant DNA is then heated for 10 minutes at 65°C, diluted to 100 $\mu$l in BamHI buffer containing 20 units BamHI, incubated at 37°C for 2 hours and then fractionated on a 5 percent polyacrylamide gel to remove excess linker molecules. The resultant 950 bp fragment having BamHI and SalI cohesive ends is conventionally purified and dissolved in 20 $\mu$l of T4 DNA ligase buffer containing both 0.2 $\mu$g of the cloning vector described previously and 0.2 units T4 DNA ligase. After incubation for 16 hours at 4°C, the DNA is used to transform E. coli K12 HB101. Plasmids are prepared from ampicillin resistant transformants and conventionally analyzed for the SalI-BamHI fragment. The desired plasmid (~5.2 kb) is designated pKEN021 (106 in Figure 3).

Ten micrograms of pKEN021 were digested at 37°C in 200 $\mu$l of XbaI/BamHI buffer (150mM NaCl, 10mM Tris•HCl, pH 8, 10mM MgCl$_2$, 6mM $\beta$-mercaptoethanol) using 10 units of BamHI for 1 hour followed by 10 units of XbaI for an additional hour at 37°C. The desired XbaI-BamHI-digested DNA was then treated with 2.5 units of alkaline phosphatase for 1.5 hours at 65°C, phenol/CHCl$_3$ extracted, collected by ethanol precipitation, and dissolved in 50 $\mu$l of TEN for future use (107 in Figure 3).

## B. Construction of Plasmid pNM575

Plasmid ptrpED50chGH800 (108 in Figure 4), described in Martial et al., 1979, Science 205: 602-607 was used as the source of a DNA fragment containing the coding sequence for a portion of the human growth hormone gene. This fragment can also be constructed synthetically (Itakura et al., 1977 and Crea et al., 1978) or can be obtained using recognized methodology described by Goodman et al., 1979, Methods in Enzymology 68:75-90, by isolating mRNA coding for human growth hormone from human pituitaries. The human growth hormone gene portion of plasmid ptrpED50chGH800 contains a unique SmaI restriction site 6 bp downstream from the translation termination codon of the gene. This site was changed to a BamHI site using the following procedure: 6 $\mu$g of the plasmid were digested with 6 units of SmaI in 200 $\mu$l of SmaI restriction buffer (15mM Tris•HCl, pH 8.0, 6mM MgCl$_2$, 15mM KCl and 6mM $\beta$-mercaptoethanol) for 1.5 hours at 37°C. After digestion was complete, phenol/CHCl$_3$ extraction was performed and the DNA was recovered by ethanol precipitation and then dissolved in 24 $\mu$l of TEN. Forty picomoles of phosphorylated BamHI adapter fragment (Collaborative Research) were added to 0.5 $\mu$g (0.2 picomole ends) of the above-digested plasmid in 16 $\mu$l of ligase buffer containing 2 units T4 DNA ligase. The mixture was incubated 2 hours at 22°C, 16 hours at 4°C and then 10 minutes at 65°C. BamHI cohesive termini were generated by conventional digestion with BamHI restriction enzyme. The enzyme cleaved the linker sequence as well as the BamHI site located at the beginning of the cloned human growth hormone cDNA sequence. This yielded a 691 bp fragment with cohesive BamHI ends which was separated on a 6 percent polyacrylamide gel and then conventionally recovered. The recovered DNA fragment was ligated (using 0.2 unit T4 DNA ligase in 20 $\mu$l of buffer under previously described conditions) with 0.2 $\mu$g of BamHI-digested and alkaline phosphatase-treated pBR322 (102 in Figure 4). After 16 hours at 4°C, the material was used to transform E. coli strain JA221 (NRRL No. B-15014) in substantial accordance with the transformation procedure of Wensink et al., 1974, Cell 3:315-325. Transformants were selected on agar plates containing 100 $\mu$g/ml ampicillin and then plasmids were conventionally isolated and identified by restriction enzyme and gel electrophoretic analysis. Desired plasmids, designated as pNM575 (109 in Figure 4), contain a BamHI fragment of approximately 700 bp and were conventionally amplified for future use.

## C. Construction of Plasmid pNM702

The DNA sequence of mature human growth hormone contains one FnuDII site which is 47 bp from the first nucleotide. Twenty-five micrograms of pNM575 were digested in 250 $\mu$l of BamHI buffer with 25 units of BamHI at 37°C for 1 hour. The 691 bp fragment with BamHI cohesive termini was conventionally isolated from a 6 percent polyacrylamide gel and purified. After purification of the fragment, one third of it (equivalent to 8 $\mu$g of plasmid) was digested in 100 $\mu$l of FnuDII buffer (6mM NaCl, 6mM Tris•HCl, pH 7.4, 6mM $MgCl_2$, 6mM $\beta$-mercaptoethanol) with 2.5 units FnuDII for 1.5 hours at 37°C. Electrophoresis on a 6 percent polyacrylamide gel and standard recovery procedures were used to isolate a 538 bp DNA fragment containing the coding sequence for the last 175 amino acids of the gene followed by a translation stop signal.

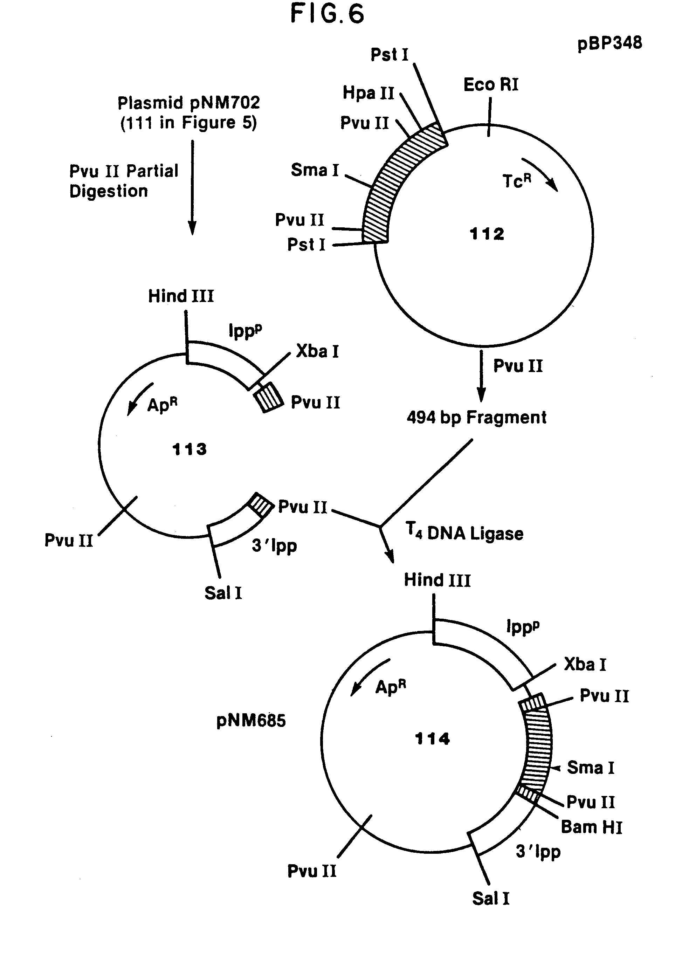

A double stranded DNA fragment (110 in Figure 5) was synthesized by the phosphotriester method to join the lpp promoter region with the human growth hormone coding region. The double stranded DNA fragment (110 in Figure 5) has the following sequence:

<u>**Xba**</u>I

```
    5'  CTAGAGGGTATTAATAATGTTCCCATTGGATGATGATGATAAGTTCCCAA-
        !!!!!!!!!!!!!!!!!!!!!!!!!!!!!!!!!!!!!!!!!!
    3'      TCCCATAATTATTACAAGGGTAACCTACTACTACTATTCAAGGGTT-


    CCATTCCCTTATCCAGGCTTTTTGACAACGCTATGCTCCG 3'  FnuDII
    !!!!!!!!!!!!!!!!!!!!!!!!!!!!!!!!!!!!!!!!
    GGTAAGGGAATAGGTCCGAAAAACTGTTGCGATACGAGGC 5'
```

The fragment was prepared by recognized phosphotriester methodology by which the following segments were prepared:

1) CTAGAGGGTAT
2) TAATAATGTTCC
3) CATTGGATGAT
4) GATGATAAGTTCC
5) CAACCATTCCC
6) TTATCCAGGC
7) TTTTTGACAACG
8) CTATGCTCCG
9) CATTATTAATACCCT
10) ATGGGAA
11) CTTATCATCATCATCCA
12) GGTTGGGAA
13) GGATAAGGGAAT
14) GTCAAAAAGCCT
15) CGGAGCATAGCGTT

Using the above-prepared segments, the T4 ligase catalyzed joining reactions were performed stepwise as described below:

a) 5'-Unphosphorylated segment 1 was joined to 5'-phosphorylated segment 2 in the presence of 5'-phosphorylated segment 9 using T4 ligase to form DNA duplex 1 (Brown et al., 1979, Methods in Enzymology 68:109-151). The duplex was isolated by preparative gel electrophoresis on 15% polyacrylamide.

b) 5'-Phosphorylated segment 3 was joined to 5'-phosphorylated segment 4 in the presence of 5'-phosphorylated segment 11 using T4 ligase to form DNA duplex 2 which was purified by 15% polyacrylamide gel electrophoresis.

c) 5'-Phosphorylated segment 5 was joined to 5'-phosphorylated segment 6 in the presence of 5'-phosphorylated segments 12 and 13 using T4 ligase to form DNA duplex 3 which was purified by 15% polyacrylamide gel electrophoresis.

d) 5'-Phosphorylated segment 7 was joined to 5'-phosphorylated segment 8 in the presence of 5'-phosphorylated segment 14 and 5'-unphosphorylated segment 15 using T4 ligase to form DNA duplex 4 which was purified by 15% polyacrylamide gel electrophoresis.

e) The DNA duplexes 2, 3 and 4 then were joined together by T4 ligase to form DNA duplex 5 which was purified by 15% polyacrylamide gel electrophoresis.

f) 5'-phosphorylated segment 10 and DNA duplex 5 were added, in the presence of T4 ligase, to the DNA duplex 1. The resulting DNA duplex (110 in Figure 5) was purified by 10% polyacrylamide gel electrophoresis. This DNA duplex then was enzymatically phosphorylated using T4 polynucleotide kinase and [$\gamma$-$^{32}$P]ATP by following established procedure.

13

EP 0 159 123 B1

The expression plasmid pNM702 was constructed by enzymatically joining 0.1 picomole (0.4 $\mu$g) of the Xbal-BamHI fragment of plasmid pKEN021 (107 in Figure 5), 0.025 picomoles synthetic DNA fragment (110 in Figure 5) and 0.3 picomoles (0.08 $\mu$g) of 538 bp fragment (from 109 in Figure 5) in 24 $\mu$l of ligation buffer using 1.5 units T4 DNA ligase. After incubation for 16 hours at 4° C, the mixture was used to transform E. coli JA221 as previously described. Transformants were selected on agar plates containing 100 $\mu$g/ml ampicillin and were conventionally cultured as a preferred source of the desired expression plasmid.

Expression of human growth hormone was detected by a standard radioimmunoassay procedure (Twomey et al., 1974, Clin. Chem. 20:389-391) and was determined to be at least 2 million molecules per cell.

D. Construction of Plasmid pNM789

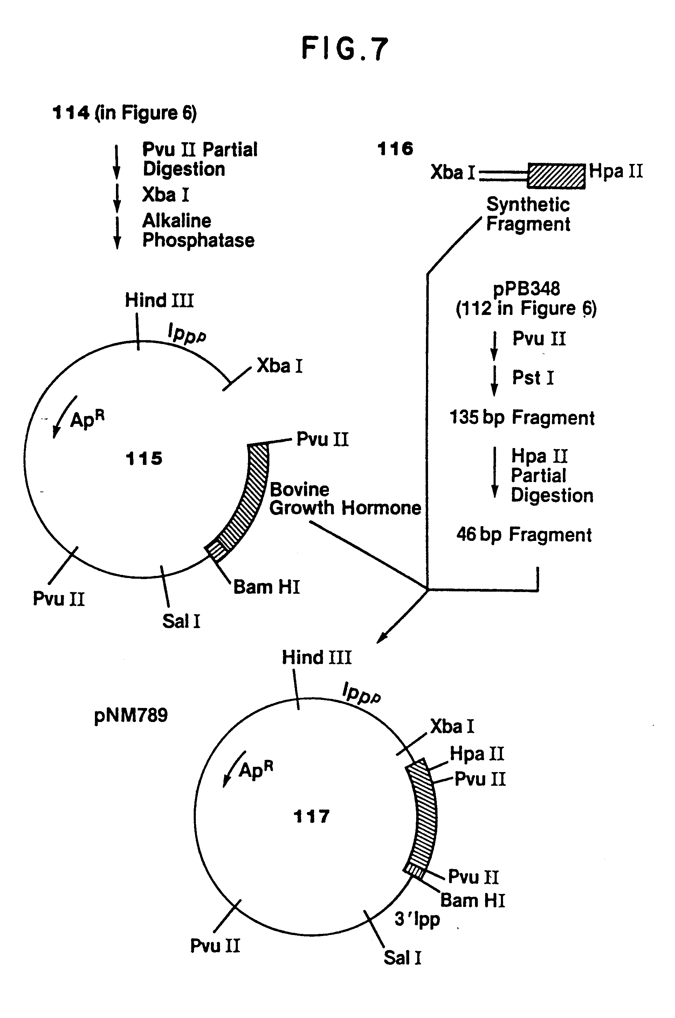

Plasmid pNM702 (111 in Figure 6), the expression plasmid for human growth hormone, was used as the starting material for construction of a plasmid expressing Met-Phe-Pro-Leu-Asp-Asp-Asp-Asp-Lys-bGH.

Plasmid pBP348 (112 in Figure 6), described in Miller et al., 1980, J. Biol. Chem. 255:7521-7524, was used as the source of two DNA fragments containing the coding sequence for a portion of the bovine growth hormone gene. The plasmid contains an 831 bp bovine growth hormone-encoding sequence cloned in the Pstl restriction site of pBR322. As an alternative to the method described in Miller et al., 1980, the sequence for bovine growth hormone can be constructed synthetically (Itakura et al., 1977 and Crea et al., 1978) or can also be obtained from messenger RNA isolated from bovine pituitaries by the now routine procedures described by Goodman et al., 1979.

The coding sequences for human growth hormone and bovine growth hormone are very similar and show much homology. Particularly useful in the construction of the expression plasmid for bovine growth hormone were the fragments generated by digestion with the restriction enzyme PvuII. The size of the fragments produced are 497 bp in human growth hormone and 494 bp in bovine growth hormone and the corresponding restriction sites occur in the same reading frames in both sequences.

Ten micrograms of pNM702 (111 in Figure 6) were partially digested with 1 unit of PvuII in 200 $\mu$l of PvuII restriction buffer (60mM NaCl, 6mM Tris•HCl, pH 7.5, 6mM $MgCl_2$, 6mM $\beta$-mercaptoethanol) for 10 minutes at 37° C. After the reaction was stopped by heating at 65° C for 10 minutes, the DNA was treated with alkaline phosphatase and the fragments separated on a one percent agarose gel. The linear DNA fragment (113 in Figure 6) of the size that corresponded to DNA with the 497 bp PvuII fragment missing (runs slightly faster than single cut plasmid) was conventionally excised, purified and used in the construction of an intermediate plasmid (114 in Figure 6).

A 494 bp PvuII fragment of plasmid pBP348 was prepared by digesting 10 $\mu$g of the plasmid in 200 $\mu$l PvuII buffer containing 10 units of PvuII for 1 hour at 37° C. The fragments were separated on a 6 percent polyacrylamide gel and the desired 494 bp fragment (from 112 in Figure 6) was conventionally visualized and purified.

Intermediate plasmid (114 in Figure 6) was constructed by reacting 0.2 $\mu$g of the plasmid pNM702 PvuII fragment with 0.05 $\mu$g of 494 bp fragment in 20 $\mu$l of T4 DNA ligase buffer containing 2 units T4 DNA ligase for 16 hours at 4° C. After transformation and selection of transformants for ampicillin resistance, the plasmids were conventionally analyzed for the presence and proper orientation of the 494 bp PvuII fragment. Plasmids with a 494 bp PvuII fragment and a 440 bp Xbal-Smal fragment are selected for use in further constructions.

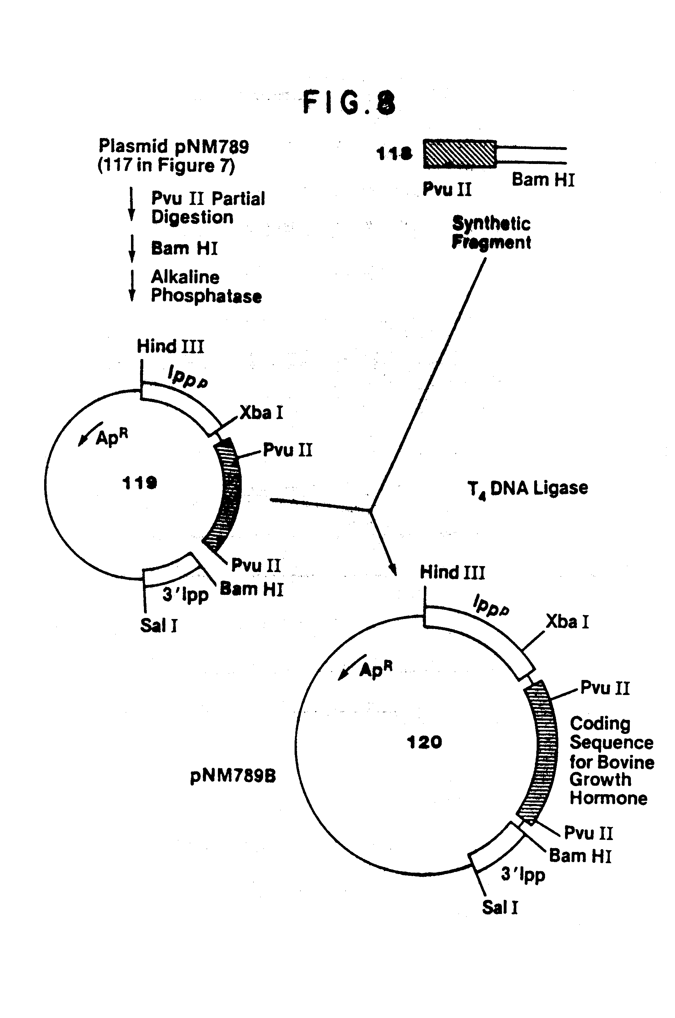

Ten micrograms of the intermediate plasmid (114 in Figure 7) were digested with 1 unit PvuII in 200 $\mu$l PvuII buffer for 5 minutes at 37° C. After the heating at 65° C for 10 minutes, the mixture was spread on a 1 percent agarose gel and linear DNA having only a single PvuII cut per molecule was recovered and purified. This recovered material (approximately 3 $\mu$g) was digested completely with 5 units of Xbal and treated with alkaline phosphatase. The fragments were spread on a 1 percent agarose gel and the largest fragment (missing the 109 bp fragment between the Xbal and the first PvuII site in human and bovine growth hormone) was conventionally recovered (115 in Figure 7).

The DNA sequence for the first 23 amino acids (69 bp) of bovine growth hormone to the first PvuII site contains 2 HpaII restriction sites, the first of which is 23 bp from the first nucleotide of the coding sequence. A 63 bp fragment (116 in Figure 7) was synthesized by the phosphotriester method. This fragment corresponds to the 19 bp sequence from the Xbal site in the Ipp ribosome binding site through the ATG translational Start signal followed by the coding sequence for Phe-Pro-Leu-Asp-Asp-Asp-Asp-Lys (24 bp) and 20 nucleotides of the coding sequence of bovine growth hormone (from Phe to the first HpaII site). The fragment has the following sequence:

14

<u>Xba</u>I

```
5' CTAGAGGGTATTAATAATGTTCCCATTGGATGATGATGATAAG-
   !!!!!!!!!!!!!!!!!!!!!!!!!!!!!!!!!!!!!!!!!!!!!
3'      TCCCATAATTATTACAAGGGTAACCTACTACTACTATTC-
```

```
TTCCCAGCCATGTCCTTGTC      3' Hpa II
!!!!!!!!!!!!!!!!!!!!
AAGGGTCGGTACAGGAACAGGC      5'
```

In producing the 63 bp fragment, the following nine segments were prepared:
1) CTAGAGGGTAT
2) TAATAATGTTCC
3) CATTGGATGAT
4) GATGATAAGTTCC
5) CAGCCATGTCCTTGTC
6) ATGGGAACATTATTAATACCCT
7) TTATCATCATCATCCA
8) ATGGCTGGGAAC
9) CGGACAAGGAC

Using the above-prepared segments, the T4 ligase catalyzed joining reactions were performed stepwise as described below:

a) 5'-Unphosphorylated segment 1 was joined to 5'-phosphorylated segment 2 in the presence of 5'-phosphorylated segment 6 using T4 ligase to form DNA duplex 1 which was purified by 15% polyacrylamide gel electrophoresis.

b) 5'-Phosphorylated segments 3, 4 and 5 were joined in the presence of 5'-phosphorylated segments 7 and 8 and 5'-unphosphorylated segment 9 using T4 ligase to form DNA duplex 2 which was purified by 15% polyacrylamide gel electrophoresis.

c) Duplexes 1 and 2 then were joined by T4 ligase to form DNA duplex (116 in Figure 7) which was purified by 15% polyacrylamide gel electrophoresis. This DNA duplex then was enzymatically phosphorylated using T4 polynucleotide kinase and $[\gamma\text{-}^{P32}]$ATP following established procedure.

The DNA fragment of 46 bp which runs from the above-described HpaII site to the PvuII site can either be constructed synthetically or obtained from the original pBP348 plasmid. Accordingly, one hundred micrograms of plasmid pBP348 were digested in 400 $\mu$l of PvuII buffer with 50 units of PvuII for 2 hours at 37°C. After phenol extraction and ethanol precipitation, the DNA was dissolved in 400 $\mu$l of PstI buffer (50mM NaCl, 6mM Tris•HCl, pH 7.4, 6mM MgCl$_2$, 6mM $\beta$-mercaptoethanol) with 50 units of PstI for 2 hours at 37°C. The DNA fragments were spread on a 6 percent polyacrylamide gel and the 135 bp fragment containing the desired 46 bp sequence was recovered and purified by standard procedures. One-third of the recovered DNA (equivalent to 33 $\mu$g) was subjected to limited digestion by 1 unit of HpaII restriction enzyme in 100 $\mu$l HpaII buffer (20mM Tris•HCl, pH 7.4, 7mM MgCl$_2$, 6mM $\beta$-mercaptoethanol) for 40 minutes at 37°C. After heating at 65°C for 10 minutes, the DNA fragments were run on a 5 percent acrylamide gel (acrylamide:bis ratio 19:1) along with an appropriate size marker. The desired 46 bp fragment yielded by HpaII partial digestion of the 135 bp fragment (from 112 in Figure 7) was purified by standard procedures.

Two-tenths microgram of the XbaI-PvuII fragment of plasmid vector (115 in Figure 7), 3.2 picomoles of synthetic 63 bp fragment (116 in Figure 7) and 0.5 picomoles 46 bp fragment (from 112 in Figure 7) were incubated in 10 $\mu$l ligation buffer with 2 units of T4 DNA ligase for 16 hours at 4°C. The ligation mixture was used to transform E. coli JA221 and the resultant transformants, which thus contained the desired plasmid pNM789, were selected by ampicillin resistance. The identity of plasmid pNM789 (117 in Figure 7) was confirmed by conventionally screening for the presence of both the 494 bp PvuII the 109 bp XbaI-PvuII fragments.

E. Final Construction of Plasmid pNM789B

Plasmid pNM789 (117 in Figure 8) requires one amino acid codon change for complete conversion to bovine growth hormone. This was accomplished by the removal of the 28 bp PvuII to BamHI fragment of pNM789 and replacement with a synthetic double stranded fragment having the following sequence (118 in Figure 8):

$$5' \text{ CTGTGCCTTCTAG } 3'$$
$$\text{|||||||||||||}$$
$$3' \text{ GACACGGAAGATCCTAG } 5'$$

Ten micrograms of pNM789 were digested with 1 unit of PvuII in 200 $\mu$l PvuII buffer for 5 minutes at 37°C. After heating 10 minutes at 65°C, the mixture was diluted to 300 $\mu$l with the addition of BamHI buffer, digested to completion with 10 units of BamHI for 1 hour at 37°C, treated with 5 units of alkaline phosphatase and incubated for 1 hour at 65°C. The DNA fragments were separated on a 1 percent agarose gel and a DNA fragment (119 in Figure 8) about the size of single cut plasmid pNM789 was conventionally purified. Two-tenths microgram of this fragment was ligated with 5 picomoles of synthetic fragment using 2 units of T4 ligase in 20 $\mu$l ligase buffer. The ligation was carried out overnight at 4°C. Following transformation, several plasmids were isolated and screened for the appropriate PvuII (494bp) and XbaI-BamHI (628bp) fragments. Plasmids comprising the aforementioned fragments constituted the desired plasmid pNM789B (120 in Figure 8).

Example 2

## Construction of Plasmid pCZ101 and E. coli K12 RV308/- pCZ101

A. Isolation of Plasmid pIM-I'-A3

The bacterium E. coli K12/pIM-I'-A3 (NRRL B-15733) was cultured in TY broth (1% tryptone, 0.5% yeast extract, 0.5% sodium chloride, pH 7.4) with 50 $\mu$g/ml of kanamycin at 25°C according to conventional microbiological procedures. After the culture was diluted 1:10 into fresh broth and after 3 hours incubation at 37°C, about .5 ml of the culture was transferred to a 1.5 ml Eppendorf tube and centrifuged for about 15 seconds. Unless otherwise indicated, all the manipulations were done at ambient temperature. The resultant supernatant was carefully removed with a fine-tip aspirator and the cell pellet was resuspended in about 100 $\mu$l of freshly prepared lysozyme solution (2 mg/ml) which contained 2 mg/ml lysozyme, 50mM glucose, 10 mM EDTA (diaminetetracetate) and 25 mM Tris•HCl, pH 8. About 200 $\mu$l. of alkaline SDS (sodium dodecyl sulfate) solution (0.2N NaOH, 1% SDS) were added and the tube was gently inverted and then kept at 0°C until lysis was complete (~5 minutes). Next, about 150 $\mu$l of 3M sodium acetate were added and the contents of the tube mixed gently by inversion for a few seconds.

The tube was maintained at 0°C for at least 60 minutes and then centrifuged for 15 minutes to yield an almost clear supernatant. The supernatant was transferred to a second centrifuge tube to which 3 volumes of cold 100% ethanol were added. After the tube was held on dry ice ethanol for 5 minutes, the resultant precipitate was collected by centrifugation (5 minutes) and the supernatant was removed by aspiration. The collected pellet was dissolved in 100 $\mu$l of TE (10mM Tris•HCl, pH 8.0, 1mM EDTA) and constituted the desired pIM-I'-A3 plasmid DNA.

## B.  XbaI-BamHI Digestion of Plasmid pNM789B and generation of the ~0.6 kb XbaI-BamHI Fragment

About 5 $\mu$g of plasmid pNM789B DNA in 50 $\mu$l Hi Salt buffer *were incubated with 10 units each of

*Hi Salt buffer was conventionally prepared with the following composition:
100 mM NaCl
20 mM Tris HCl, pH 8.0
10 mM MgCl$_2$
5 mM $\beta$-mercaptoethanol

BamHI and XbaI restriction enzymes at 37°C for about 1 hour. After the addition of 5 μl of 3M sodium acetate pH 7.0, the DNA was precipitated with 2 volumes of 100% ethanol. The desired DNA digest was dissolved in 100 μl of TE buffer and stored at 0°C for future use.

## C.  XbaI-BamHI Digestion of Plasmid pIM-I'-A3

The desired digestion was carried out in substantial accordance with the procedure of Example 2B except that plasmid pIM-I'-A3, rather than plasmid pNM789B, was used. The desired DNA was dissolved in about 100 μl of TE buffer and stored at 0°C. for future use.

D. Ligation and Transformation

About 1 μg of the plasmid pIM-I'-A3 digest, 1 μg of the plasmid pNM789B Xbal-BamHI digest, 40 μl water, 5 μl (5mM) ATP, 5 μl ligation mix *and 5 units T4 DNA ligase were incubated at 20°C for about 2 hours. After incubation at 65°C for 2 minutes followed by cooling on ice, the resultant ligation mixture was used to transform, in substantial accordance with the transformation procedure of Wensink, 1974, Cell 3:315, E. coli K12 RV308 on TY plates (1% tryptone, 0.5% yeast extract, 0.5% sodium chloride, 1.5% agar, pH 7.4) containing 50 μg/ml of kanamycin. Bacterial strain E. coli K12 RV308 has been deposited and made part of the permanent stock culture collection of the Northern Regional Research Laboratory, Peoria, Illinois, from which it is available to the public under the accession number NRRL B-15624.

Some of the resultant transformants, as conventionally shown by agarose gel electrophoresis (Maniatis et al., 1982) and other tests, contained only the desired ~10.8 kb plasmid. Such a transformant, herein designated as E. coli K12 RV308/pCZ101, was selected, plated on TY agar containing appropriate antibiotics and then cultured using conventional microbiological techniques. The resultant cells were used to isolate plasmid pCZ101 in substantial accordance with the procedure of Example 2A.

Example 3

## Construction of Plasmid pCZ1920 and E. coli K12 RV308/- pCZ1920

## A.  Construction of the ~10.2 kb BamHI-XbaI Fragment of Plasmid pCZ101

The desired fragment was constructed in substantial accordance with the teaching of Example 2B except that plasmid pCZ101, rather than plasmid pNM789B, was used. The desired ~10.2 kb BamHI-Xbal restriction fragments were conventionally separated and isolated by agarose gel electrophoresis (Maniatis et al., 1982) and then dissolved in about 100 μl of TE buffer and stored at 0°C for future use.

## B.  Construction of the ~0.6 kb BamHI-HgiAI Fragment of Plasmid pCZ101

The desired fragment was constructed in substantial accordance with the teaching of Example 2B

*Ligation mix can be prepared with the following composition:
500 mM Tris HCl, pH 7.8
200 mM Dithiothreitol
100 mM MgCl₂

except that plasmid pCZ101 and HgiAI restriction enzyme, rather than plasmid pNM789B and XbaI restriction enzyme, were respectively used. The desired ~0.6 kb BamHI-HgiAI restriction fragments were conventionally separated and isolated by agarose gel electrophoresis (Maniatis et al., 1982) and then dissolved in about 100 µl of TE buffer and stored at 0°C for future use.

C. Construction of the DNA Linker Sequence

```
5' CTAGAGGGTATTAATA ATG AAA GGG AAT TCT ATG GCC TTC CCA GCC
   ¦¦¦¦¦¦¦¦¦¦¦¦ ¦¦¦ ¦¦¦ ¦¦¦ ¦¦¦ ¦¦¦ ¦¦¦ ¦¦¦ ¦¦¦ ¦¦¦
3'      TCCCATAATTAT TAC TTT CCC TTA AGA TAC CGG AAG GGT CGG

ATG TCC TTG TCC GGC CTG TTT GCC AAC GCT GTGCT  3'
¦¦¦ ¦¦¦ ¦¦¦ ¦¦¦ ¦¦¦ ¦¦¦ ¦¦¦ ¦¦¦ ¦¦¦ ¦¦¦ ¦
TAC AGG AAC AGG CCG GAC AAA CGG TTG CGA C        5'
```

The desired linker sequence was conventionally synthesized by the modified phosphotriester method in substantial accordance with the teaching of Itakura et al., 1977 and Crea et al., 1978. The aforementioned synthesis method is also specifically illustrated in Example 1.

D. Ligation and Transformation

About 20 picomoles of the DNA linker of Example 3C, 1 µg of the plasmid pCZ101 ~10.2 kb BamHI-XbaI fragment and 0.5 µg of the plasmid pCZ101 ~0.6 kb BamHI-HgiAI fragment were ligated and the resultant plasmid used to transform E. coli K12 RV308 in substantial accordance with the teaching of Example 2D.

Some of the resultant transformants, as conventionally shown by agarose gel electrophoresis (Maniatis et al., 1982) and other tests, contained only the desired ~10.8 kb plasmid. Such a transformant, herein designated as E. coli K12 RV308/pCZ1920, was selected, plated on TY agar containing appropriate antibiotics and then cultured using conventional microbiological techniques. The resultant cells were shown, by SDS gel electrophoresis, RIA and other tests, to express the aforedefined MET-LYS-GLY-ASPN-SER-MET-ALA-bGH derivative at high levels. Because plasmid pCZ1920 contains a thermoinducible runaway replicon, maximum expression of the desired bGH derivative product occurs at culture temperatures of about 37°C.

Example 4

## Construction of Plasmid pJR1 and E. coli K12 RV308/-pJR1

The desired constructions are made in substantial accordance with the teaching of Example 3 except that the DNA linker sequence

```
5' CTAGAGGGTATTAATA ATG TTT CCA GCC ATG GCT CTA TCT GGT
   ¦¦¦¦¦¦¦¦¦¦¦¦ ¦¦¦ ¦¦¦ ¦¦¦ ¦¦¦ ¦¦¦ ¦¦¦ ¦¦¦ ¦¦¦ ¦¦¦
3'      TCCCATAATTAT TAC AAA GGT CGG TAC CGA GAT AGA CCA

CTG TTT GCC AAC GCT GTGCT  3'
¦¦¦ ¦¦¦ ¦¦¦ ¦¦¦ ¦¦¦ ¦
GAC AAA CGG TTG CGA C        5'
```

is substituted for the linker sequence of Example 3C. The above-specified linker sequence can be constructed in substantial accordance with the conventional procedure of Itakura et al., 1977 and Crea et al., 1978. The aforementioned synthesis method is also specifically illustrated in Example 1.

The desired transformants, herein designated as E. coli K12 RV308/pJR1, are plated on TY agar containing appropriate antibiotics and then conventionally cultured for subsequent production and isolation of plasmid pJR1. The transformants are also shown, by SDS gel electrophoresis, RIA and other tests, to express the aforedefined MET-PHE-PRO-ALA-MET-ALA-R[2] bovine growth hormone derivative at high levels. Because plasmid pJR1 contains a thermoinducible runaway replicon, maximum expression of the desired bGH derivative product occurs at culture temperatures of about $37^\circ$ C.

## Example 5

### Construction of Plasmid pHI7△4△1

A partial scheme for the construction of plasmid pHI7△4△1 is presented in Figure 15 of the accompanying drawings.

### A. Construction of Plasmid pBRHtrp

Plasmid pGM1 carries the E. coli tryptophan operon containing the deletion △LE1413 (Miozzari, et al., 1978, J. Bacteriology, 1457-1466) and hence expresses a fusion protein comprising the first 6 amino acids of the trp leader and approximately the last third of the trp E polypeptide (hereinafter referred to in conjunction as LE'), as well as the trp D polypeptide in its entirety, all under the control of the trp promoteroperator system. E. coli K12 W3110tna2-trp-△102/pGM1 has been deposited with the American Type Culture Collection (ATCC No. 31622) and pGM1 may be conventionally removed from the strain for use in the procedures described below.

About 20 µg of the plasmid were digested with the restriction enzyme PvuII which cleaves the plasmid at five sites. The gene fragments were next combined with EcoRI linkers (consisting of a self complementary oligonucleotide of the sequence: pCATGAATTCATG) providing an EcoRI cleavage site for later cloning into a plasmid containing an EcoRI site. The 20 µg of DNA fragments obtained from pGM1 were treated with 10 units T4 DNA ligase in the presence of 200 picomoles of the 5'-phosphorylated synthetic oligonucleotide pCATGAATTCATG and in 20 µl T4 DNA ligase buffer (20 mM Tris, pH 7.6, 0.5 mM ATP, 10 mM MgCl$_2$, 5 mM dithiothreitol) at $4^\circ$ C overnight. The solution was then heated 10 minutes at $70^\circ$ C to halt ligation. The linkers were cleaved by EcoRI digestion and the fragments, now with EcoRI ends, were separated using 5 percent polyacrylamide gel electrophoresis (hereinafter "PAGE"). The three largest fragments were isolated from the gel by first staining with ethidium bromide and then locating the fragments with ultraviolet light and cutting from the gel the portions of interest. Each gel fragment, with 300 µl 0.1xTBE, was placed in a dialysis bag and subjected to electrophoresis at 100 v for one hour in 0.1xTBE buffer (TBE buffer contains: 10.8 gm Tris base, 5.5 gm boric acid, .09 gm Na$_2$EDTA in 1 liter H$_2$O). The aqueous solution was collected from the dialysis bag, phenol extracted, chloroform extracted, and made 0.2M with respect to sodium chloride. The DNA was then recovered in water after ethanol precipitation. The trp promoter/operator-containing fragments with EcoRI sticky ends were identified by insertion into a tetracycline sensitive plasmid which, upon promoter/operator insertion, becomes tetracycline resistant. All DNA fragment isolations hereinafter described in this example are performed using PAGE followed by the electroelution method described above.

### B. Construction of Plasmid pBRHtrp Expressing Tetracycline Resistance Under the Control of the trp Promoter/Operator and Identification and Amplification of the trp Promoter/Operator-Containing DNA Fragment Isolated in 'A' above.

Plasmid pBRH1, (Rodriguez, et al., 1979, Nucleic Acids Research 6, 3267-3287 and ATCC No. 37070) expresses ampicillin resistance and contains the gene for tetracycline resistance but, there being no associated promoter, does not express that resistance. Cells harboring the plasmid are accordingly tetracycline sensitive. By introducing a promoter/operator system in the EcoRI site, the plasmid will express tetracycline resistance.

Plasmid pBRH1 was digested with EcoRI. The enzyme was removed by phenol extraction followed by chloroform extraction and then the DNA was resuspended in water after ethanol precipitation. The resulting DNA was, in separate reaction mixtures, combined with each of the three DNA fragments obtained in Example 5A above and ligated with T4 DNA ligase as previously described. The DNA present in the reaction mixture was used to transform competent E. coli K12 294 (NRRL B-15625) by standard techniques (Hershfield et al., 1974, Proc. Nat. Acad. Sci. USA 71:3455-3459) and the bacteria were then plated on LB plates (Maniatis et al., 1982) containing 20 μg/ml ampicillin and 5 μg/ml tetracycline.

Several tetracycline-resistant colonies were selected and the plasmid DNA was isolated and designated pBRHtrp. The presence of the desired fragment was confirmed by restriction enzyme analysis. Plasmid pBRHtrp expresses β-lactamase, imparting ampicillin resistance, and contains a DNA fragment which includes the trp promoter/operator. The DNA fragment also codes for a first protein, (designated LE'), comprising a fusion of the first six amino acids of the trp leader and approximately the last third of the trp E polypeptide, a second protein (designated D'), corresponding to approximately the first half of the trp D polypeptide, and a third protein, coded for by the tetracycline resistance gene.

C. Construction of Plasmid pSOM7Δ2

Plasmid pBRHtrp was digested with EcoRI restriction enzyme and the resulting fragment, isolated by PAGE and electroelution, was combined with EcoRI-digested plasmid pSOM11 (Itakura et al., 1977, Sci. 198:1056, U.S. Patent No. 4,366,246, G.B. Patent Publication No. 2,007,676A). The mixture was ligated with T4 DNA ligase and the resulting DNA transformed into E. coli K12 294 as previously described. Transformant bacteria were selected on ampicillin-containing plates and the resulting ampicillin-resistant colonies were screened by colony hybridization (Gruenstein et al., 1975, Proc. Nat. Acad. Sci. USA 72:3951-3965). The trp promoter/operator-containing fragment, isolated from pBRHtrp and then radioactively labelled with $^{32}$P, was used as a probe in the above procedure. Several colonies were shown to be positive by colony hybridization and were therefore selected. Plasmid DNA was isolated and the orientation of the inserted fragments was determined by restriction analysis using enzymes BglII and BamHI in double digestion. Colonies containing the desired plasmid with the trp promoter/operator fragment in the proper orientation were grown in LB medium containing 10 μg/ml ampicillin. The desired plasmid was designated pSOM7Δ2 and was used for subsequent constructions described below.

D. Construction of Plasmid ptrp24

1. **Construction of a Gene Fragment Coding for the Distal Regions of the LE' Polypeptide With BglII and EcoRI Restriction Sites Respectively at the 5' and 3' Ends of the Coding Strand**

Plasmid pSOM7Δ2 was HindIII digested followed by digestion with lambda exonuclease (a 5' to 3' exonuclease) under conditions chosen so as to digest beyond the BglII restriction site within the LE' encoding region. About 20 μg of HindIII-digested pSOM7Δ2 was dissolved in buffer (20mM glycine buffer, pH 9.6, 1mM MgCl$_2$, 1mM β-mercaptoethanol). The resulting mixture was treated with 5 units of lambda exonuclease for 60 minutes at room temperature. The reaction mixture obtained was then phenol extracted, chloroform extracted, and ethanol precipitated.

To create an EcoRI residue at the distal end of the LE' gene fragment, a primer $^{32}$pCCTGTGCATGAT was synthesized by the improved phosphotriester method (Crea et al., 1978) and hybridized to the single stranded end of the LE' gene fragment resulting from lambda exonuclease digestion. The hybridization was performed by dissolving 20 μg of the lambda exonuclease-treated HindIII digestion product of plasmid pSCM7Δ2 in 20 μl H$_2$O and combining with 6 μl of a solution containing approximately 80 picomoles of the 5'-phosphorylated oligonucleotide described above. The synthetic fragment was hybridized to the 3' end of the LE' coding sequence and the remaining single strand portion of the LE' fragment was filled in by Klenow Polymerase I using dATP, dTTP, dGTP and dCTP. Klenow Polymerase I is the fragment obtained by proteolytic cleavage of DNA Polymerase I. It contains the 5' → 3' polymerizing activity, the 3' → 5'

20

exonucleolytic activity, but not the 5' → 3' exonucleolytic activity of the parental enzyme (Kornberg, 1974, W. H. Freeman and Co., SFO, 98).

The reaction mixture was thus heated to 50°C and let cool slowly to 10°C, whereafter 4 μl of Klenow enzyme were added. After 15 minutes incubation at room temperature, followed by 30 minutes incubation at 37°C, the reaction was stopped by the addition of 5 μl of 0.25 molar EDTA. The reaction mixture was phenol extracted, chloroform extracted, and ethanol precipitated. The DNA was subsequently cleaved with the restriction enzyme BglII and the fragments were separated by PAGE. An autoradiogram obtained from the gel revealed a $^{32}P$ labelled fragment of the expected length of approximately 470 bp, which was recovered by electroelution. As outlined, this fragment LE'(d) has a BglII terminus and a blunt end coinciding with the beginning of the primer.

### 2. Construction of Plasmid pThα1

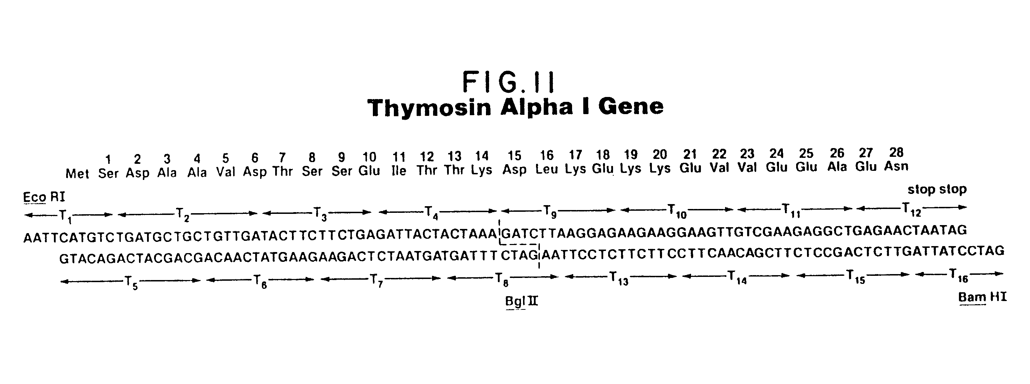

Plasmid pThα1 was constructed by inserting a synthesized gene for thymosin alpha 1 into plasmid pBR322. The synthesis of the thymosin alpha 1 coding DNA involves the synthesis and subsequent ligation of the 16 oligonucleotides ($T_1$ through $T_{16}$) that are indicated by the double headed arrows in Figure 11 of the accompanying drawings. A met ATG was inserted at the N-terminus and the 5' ends were designed with single-stranded cohesive termini to facilitate joining to plasmids cleaved with EcoRI and BamHI. As can be readily appreciated, the BglII site in the center of the gene assists in the analysis of recombinant plasmids.

Oligodeoxyribonucleotides $T_1$ to $T_{16}$ were synthesized by the modified phosphotriester method of Itakura et al., 1977 and Crea et al., 1978. The various oligodeoxyribonucleotides are shown below in Table 1.

Table 1

| SYNTHETIC OLIGONUCLEOTIDES FOR THYMOSINα1 GENE | | | |
|---|---|---|---|
| Compound | Sequence | Length | HPLC Analysis Retention Time (min)[*] |
| $T_1$ | A-A-T-T-C-A-T-G-T-C | 10 | 17.4 |
| $T_2$ | T-G-A-T-G-C-T-G-C-T-G-T-T-G-A | 15 | 24.3 |
| $T_3$ | T-A-C-T-T-C-T-T-C-T-G-A | 12 | 20.3 |
| $T_4$ | G-A-T-T-A-C-T-A-C-T-A-A-A | 13 | 22.0 |
| $T_5$ | G-C-A-G-C-A-T-C-A-G-A-C-A-T-G | 15 | 24.8 |
| $T_6$ | G-A-A-G-T-A-T-C-A-A-C-A | 12 | 20.1 |
| $T_7$ | A-G-T-A-A-T-C-T-C-A-G-A-A | 13 | 22.6 |
| $T_8$ | A-A-G-A-T-C-T-T-T-A-G-T | 12 | 20.2 |
| $T_9$ | G-A-T-C-T-T-A-A-G-G-A-G | 12 | 20.4 |
| $T_{10}$ | A-A-G-A-A-G-G-A-A-G-T-T | 12 | 21.1 |
| $T_{11}$ | G-T-C-G-A-A-G-A-G-G-C-T | 12 | 20.5 |
| $T_{12}$ | G-A-G-A-A-C-T-A-A-T-A-G | 12 | 20.4 |
| $T_{13}$ | C-T-T-C-T-T-C-T-C-C-T-T | 12 | 19.9 |
| $T_{14}$ | T-T-C-G-A-C-A-A-C-T-T-C | 12 | 20.5 |
| $T_{15}$ | G-T-T-C-T-C-A-G-C-C-T-C | 12 | 20.2 |
| $T_{16}$ | G-A-T-C-C-T-A-T-T-A | 10 | 17.2 |

*at ambient temperature

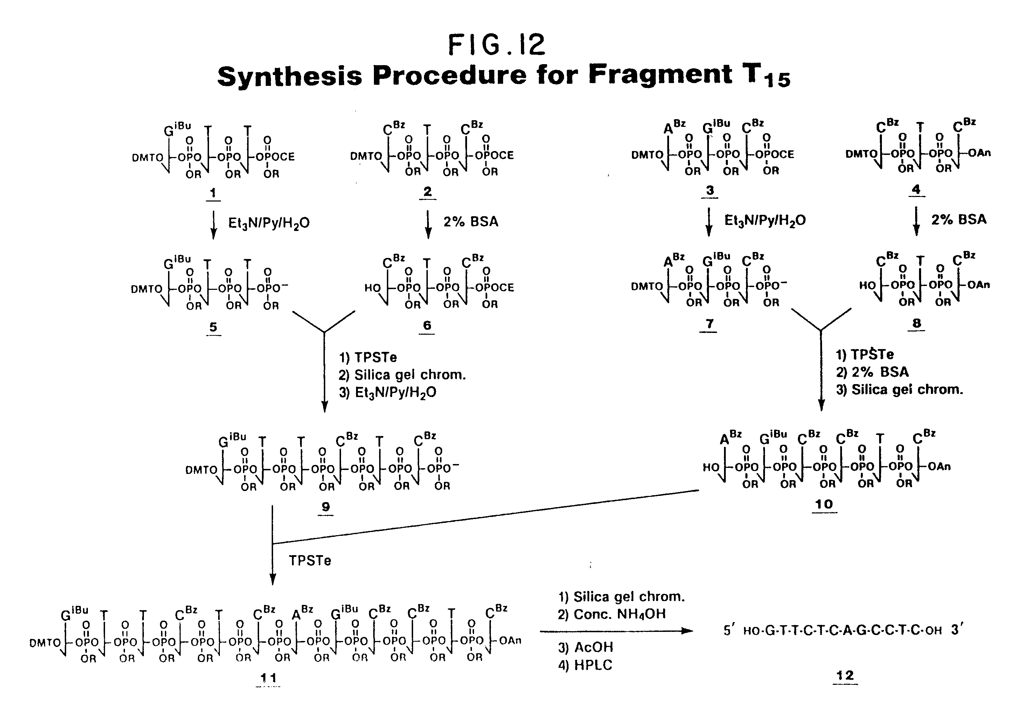

The above synthesis is typified by the following procedure for fragment $T_{15}$ as summarized in Figure 12 of the accompanying drawings. Various nucleotide fragments that are used in the synthesis of T15 are numerically designated in the Figure. The abbreviations employed are as follows: TPSTe, 2,4,6-triisopropyl-benzenesulfonyltetrazole; BSA, benzene sulfonic acid; TLC, thin layer chromatography; HPLC, high performance liquid chromatography; DMT, 4,4'-dimethoxytrityl; CE, 2-cyanoethyl; R, p-chlorophenyl; Bz, benzoyl; An, anisoyl: iBu, isobutryl; Py, pyridine; AcOH, acetic acid; $Et_3N$, triethylamine.

The fully protected trideoxyribonucleotides 4 (85 mg, 0.05 mmol) and 2 (180 mg, 0.1 mmol) were deblocked at the 5' hydroxyls by treatment with 2% BSA in 7:3 (v/v) chloroform/methanol (10 and 20 ml, respectively) for 10 minutes at 0°C. Reactions were stopped by addition of saturated aqueous ammonium bicarbonate (2 ml), extracted with chloroform (25 ml) and washed with water (2 x 10 ml). The organic layers were dried (magnesium sulfate), concentrated to small volumes (about 5 ml) and precipitated by addition of petroleum ether (35°-60°C fraction). The colorless precipitates were collected by centrifugation and dried in a dessicator in vacuo to give 6 and 8, respectively, each homogeneous by silica gel tlc (Merck 60 F254, chloroform/methanol, 9:1).

Trimers 1 and 3 (270 mg, 0.15 mmol; 145 mg, 0.075 mmol) were converted into their phosphodiesters (5 and 7) by treatment with triethylamine/pyridine/water (1:3:1, v/v, 10 ml) for 25 minutes at ambient temperature. Reagents were removed by rotary evaporation and the residues dried by repeated evaporations with anhydrous pyridine (3x 10 ml). Trimer 8 (0.05 mmol) and trimer 7 were combined with TPSTe (50 mg, 0.15 mmol) in anhydrous pyridine (3 ml) and the reaction mixture left in vacuo at ambient temperature for two hours. TLC analysis showed that 95% of the trimer 8 had been converted into hexamer product (visualized by detection of the DMT group by spraying with 10% aqueous sulfuric acid and heating at 60°C). The reaction was quenched by addition of water (1 ml) and the solvent evaporated under reduced pressure. After removal of pyridine by coevaporations with toluene, the hexamer was deblocked at the 5' position with 2% BSA (8 ml) as described above for trimers 4 and 2. The product (10) was purified on a silica gel column (Merck 60 H, 3.5 x 5 cm) by step gradient elution with chloroform/methanol (98:2 to 95:5, v/v). Fractions containing product 10 were evaporated to dryness. Similarly, trimer 5 was coupled to 6 and the fully protected product directly purified on silica gel. This latter compound was deblocked at the 3' end by triethylamine/pyridine/water as described above to give fragment 9.

Finally, hexamers 9 and 10 were coupled in anhydrous pyridine (2 ml) with TPSTe (75 mg, 0.225 mmol) as the condensing agent. Upon completion (4 hours, ambient temperature) the mixture was rotary evaporated and the residue chromatographed on silica gel. Product 11 (160 mg) was obtained by precipitation with petroleum ether and appeared homogeneous on TLC. A portion of compound 11 (20 mg) in pyridine (0.5 ml) was completely deblocked by treatment with concentrated ammonium hydroxide (7 ml, 8 hours, 60°C) and subsequent treatment in 80% acetic acid (15 minutes, ambient temperature). After evaporation of acetic acid, the solid residue was dissolved in 4% aqueous ammonium hydroxide (v/v, 4 ml) and extracted with ethyl ether (3x2 ml). The aqueous phase was concentrated to 1-2 ml and a portion applied to HPLC for purification of 12. The fractions corresponding to the major peak were pooled (ca. 2 $A_{254}$ units) and concentrated to about 5 ml. The final product 12 was desalted on Bio-gel P-2 (1.5 x 100 cm) by elution with 20% aqueous ethanol, reduced to dryness and resuspended in water (200 $\mu$l) to give a solution of $A_{254}$ = 10. The sequence of 12 was confirmed by two-dimensional sequence analysis.

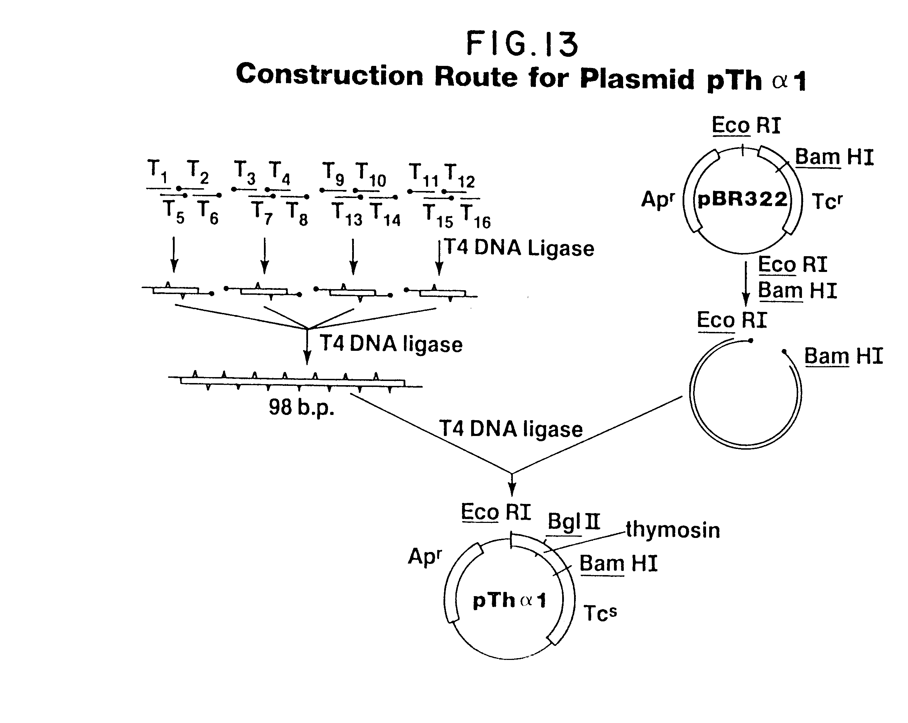

The complete thymosin alpha 1 gene was assembled from the 16 synthetic oligo-nucleotides by methods previously described in detail for somatostatin (Itakura et al., 1977), insulin (Goeddel et al., 1979), and growth hormone (Goeddel et al., 1979, Nature 281:544). Ten microgram quantities of oligonucleotides $T_2$ through $T_{15}$ were quantitatively phosphorylated with [$\gamma$-$P^{32}$]-ATP (New England Nuclear) in the presence of T4 polynucleotide kinase (Goeddel et al, 1979), to give specific activities of approximately 1 Ci/mmol. Radiolabelled fragments were purified by 20% polyacrylamide/7 M urea gel electrophoresis and sequences of the eluted fragments were verified by two-dimensional electrophoresis/homochromatography (Jay et al., 1974, Nucleic Acids Res. 1:331) of partial snake venom digests. Fragments $T_1$ and $T_{16}$ were left unphosphorylated to minimize undesired polymerization during subsequent ligation reactions. These oligonucleotides (2 $\mu$g each) were assembled in four groups of four fragments (see figure 13 of the accompanying drawings), by T4 DNA ligase using published procedures (Goeddel et al., 1979). The reaction products were purified by gel electrophoresis on a 15% polyacrylamide gel containing 7 M urea (Maxam and Gilbert, 1977, Proc. Nat. Acad. Sci. USA 71:3455). The four isolated products were ligated together and the reaction mixture resolved by 10% polyacrylamide gel electrophoresis. DNA in the size range of the thymosin alpha 1 gene (90-105 base pairs) was electroeluted.

Plasmid pBR322 (0.5 $\mu$g) was treated with BamHI and EcoRI restriction endonucleases and the fragments separated by polyacrylamide gel electrophoresis. The large fragment was recovered from the gel by electro-elution and subsequently ligated to the assembled synthetic DNA (Goeddel et al., Nature 281:544 1979). This mixture was used to transform E. coli K12 294. Five percent of the transformation mixture was plated on LB plates containing 20 $\mu$g/ml ampicillin. The four ampicillin resistant colonies obtained were sensitive to tetracycline, suggesting insertion into the tetracycline resistance gene. Analysis of the plasmids from these four colonies showed that in each case the plasmid, designated pThα1, contained (a) a BglII site not found in pBR322 itself, thus indicating the presence of the thymosin alpha 1 gene as shown in Figure 11, and (b) a fragment of approximately 105 base pairs generated by BamHI/EcoRI cleavage. The construction route for plasmid pThα1 (not drawn to scale), is presented in Figure 13 of the accompanying drawings wherein the heavy dots indicate 5'-phosphate groups.

3. Reaction of Treated pThα1 and LE'(d) Fragment

The plasmid pThα1 contains a gene specifying ampicillin resistance and a structural gene specifying thymosin alpha 1 cloned at its 5' coding strand end into an EcoRI site and at its 3' end into a BamHI site. The thymosin gene contains a BglII site as well. To create a plasmid capable of accepting the LE'(d) fragment prepared above, pTHα1 was EcoRI digested followed by Klenow polymerase I reaction with dTTP and dATP to blunt the EcoRI residues. BglII digestion of the resulting product created a linear DNA fragment containing the gene for ampicillin resistance and, at its opposite ends, a sticky BglII residue and a blunt end. The resulting product could be recircularized by reaction with the LE'(d) fragment containing a BglII sticky end and a blunt end in the presence of T4 ligase to form the plasmid ptrp24. In doing so, an EcoRI site is recreated at the position where blunt end ligation occurred.

E. Construction of Plasmid pSOM7△2△4

Successive digestion of ptrp24 with BglII and EcoRI, followed by PAGE and electroelution, yields a fragment having codons for the LE'(d) polypeptide with a BglII sticky end and an EcoRI sticky end adjacent to its 3' coding terminus. The LE'(d) fragment can be cloned into the BglII site of plasmid pSOM7△2 to form an LE' polypeptide/somatostatin fusion protein expressed under the control of the tryptophan promoter/operator. To do so requires (1) partial EcoRI digestion of pSOM7△2 in order to cleave the EcoRI site distal to the tryptophan promoter/operator, and (2) proper choice of the primer sequence in order to properly maintain the codon reading frame, and to recreate an EcoRI cleavage site.

Thus, 16 μg of plasmid pSOM7△2 were diluted into 200 μl of buffer containing 20 mM Tris, pH 7.5, 5 mM MgCl₂, 0.02% NP40 detergent, and 100 mM NaCl, and treated with 0.5 units EcoRI. After 15 minutes at 37°C, the reaction mixture was phenol extracted, chloroform extracted, ethanol precipitated, and subsequently digested with BglII. The larger resulting fragment was isolated by the PAGE procedure followed by electroelution. This fragment contains the codons "LE'(p)" for the proximal end of the LE' polypeptide, i.e., those up-stream from the BglII site. This fragment was next ligated to the above LE'(d) fragment in the presence of T4 DNA ligase to form the plasmid pSOM7△2△4, which upon transformation into E. coli K12 294, efficiently produced a fusion protein consisting of the fully reconstituted LE polypeptide and somatostatin under the control of the tryptophan promoter/operator.

## F.  Construction of Linear DNA Having a PstI Residue at the 3' end and a BglII Residue at its 5' End Bounding a Gene Specifying Tetracycline Resistance

Plasmid pBR322 was HindIII digested and the protruding HindIII ends were digested with S1 nuclease. The S1 nuclease digestion involved treatment of 10 μg of HindIII-cleaved pBR322 in 30 μl S1 buffer (0.3M NaCl, 1 mM ZnCl₂, 25 mM sodium acetate, pH 4.5) with 300 units S1 nuclease for 30 minutes at 15°C. The reaction was stopped by the addition of 1 μl of 30X S1 nuclease stop solution (0.8M tris base, 50 mM EDTA). The mixture was phenol extracted, chloroform extracted, ethanol precipitated, and then EcoRI digested as previously described. The resulting fragment, obtained by the PAGE procedure followed by electroelution, has an EcoRI sticky end and a blunt end whose coding strand begins with the nucleotide thymidine. The S1-digested HindIII residue beginning with thymidine can be joined to a Klenow Polymerase I-treated BglII residue so as to reconstitute the BglII restriction site upon ligation.

Therefore plasmid pSOM7△2, prepared in Example 5C, was BglII digested and the resulting BglII sticky ends were made double stranded by treatment with Klenow polymerase I using all four deoxynucleotide triphosphates. EcoRI cleavage of the resulting product, followed by PAGE and electroelution of the small fragment, yielded a linear piece of DNA containing the tryptophan promoter/operator and codons of the LE' "proximal" sequence upstream from the BglII site ("LE'(p)"). The product had an EcoRI end and a blunt end resulting from filling in the BglII site. However, the BglII site is reconstituted by ligation of the blunt end to the blunt end of the above S1-digested HindIII fragment. Thus, the two fragments were ligated in the presence of T4 DNA ligase to form the recircularized plasmid pHKY10 which was propagated by transformation into competent E. coli K12 294 cells. Tetracycline resistant cells bearing the recombinant plasmid pHKY10 were selected and the plasmid DNA extracted. Digestion with BglII and PstI, followed by isolation by the PAGE procedure and electroelution of the large fragment, yielded the desired linear piece of DNA having PstI and BglII sticky ends. This DNA fragment, thus produced from pHKY10, contains the origin of replication and, therefore, is useful as a component in the construction of plasmid pHI△4△1 in which both

23

the genes coding for the trp LE' polypeptide fusion protein and the tetracycline resistance are controlled by the trp promoter/operator.

## G.  Construction of Linear DNA Having the trp Promoter/Operator

Plasmid pSOM7△2△4, prepared in Example 5E, was subjected to partial EcoRI digestion followed by PstI digestion. The resulting fragment contained the trp promoter/operator and was isolated by the PAGE procedure followed by electroelution. Partial EcoRI digestion was necessary to obtain a fragment which was cleaved adjacent to the 5' end of the somatostatin gene but not cleaved at the EcoRI site present between the ampicillin resistance gene and the trp promoter/operator. Ampicillin resistance lost by the PstI cut in the ampicillin resistance gene can be restored upon ligation with the final pHKY10 linear DNA derivative produced in Example 5F above.

H. Preparation of Synthetic Gene Coding for the 32 N-Terminal Amino Acids of Proinsulin

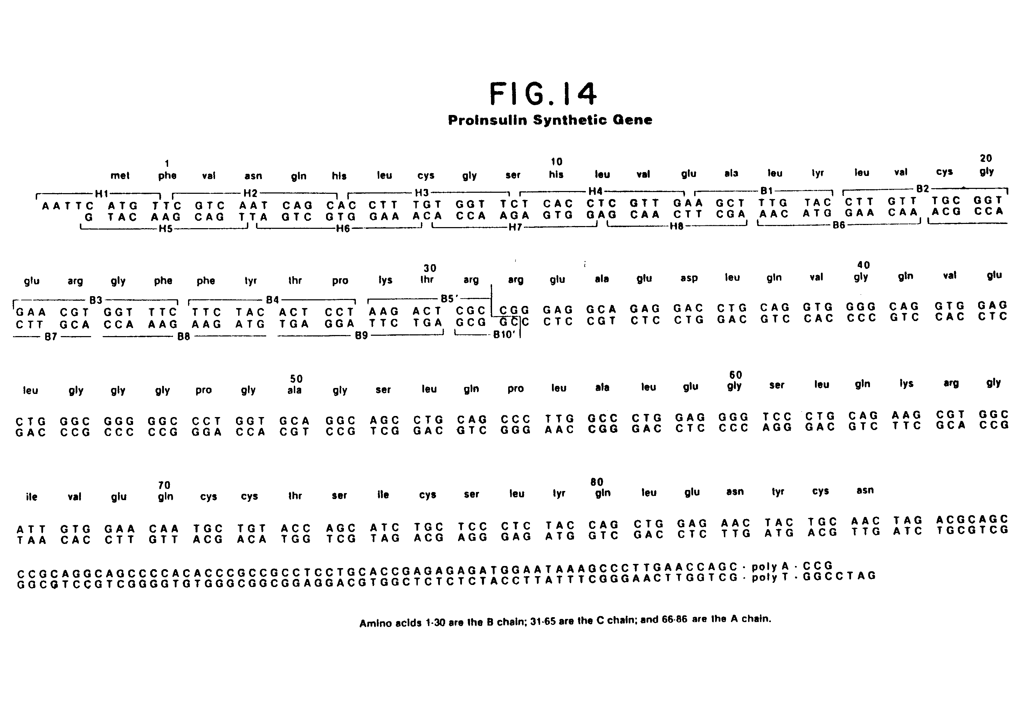

A series of 18 oligonucleotides, shown in Table 2, were prepared as a first step in constructing a gene coding for the first 32 amino acids of proinsulin. The nucleotide sequence of the entire gene ultimately constructed is shown in Figure 14.

Table 2

| Synthetic Oligonucleotides For Human Proinsulin | |
| --- | --- |
| Compound | Sequence |
| H1 | AATTCATGTT |
| H2 | CGTCAATCAGCA |
| H3 | CCTTTGTGGTTC |
| H4 | TCACCTCGTTGA |
| H5 | TTGACGAACATG |
| H6 | CAAAGGTGCTGA |
| H7 | AGGTGAGAACCA |
| H8 | AGCTTCAACG |
| B1 | AGCTTTGTAC |
| B2 | CTTGTTTGCGGT |
| B3 | GAACGTGGTTTC |
| B4 | TTCTACACTCCT |
| B5' | AAGACTCGCC |
| B6 | AACAACGTACAA |
| B7 | ACGTTCACCGCA |
| B8 | GTAGAAGAAACC |
| B9 | AGTCTTAGGAGT |
| B10' | GATCCGGCG |

The synthetic nucleotides are shown between brackets and are underlined in Figure 14. These olignucleotides were synthesized by the conventional method of Crea et al., 1978, J. Biol. Chem. 250:4592 and Itakura et al., 1975, J. Am. Chem. Soc. 97:7327. Some of the oligonucleotides were used for constructing a gene for the human insulin B chain previously described by Crea, et al., 1978 and Goeddel, et al, 1979. Two oligonucleotides (B5' and B10') incorporate HpaII and terminal BamHI and EcoRI restriction sites. The terminal sites are particularly useful for purposes of cloning.

The eight oligonucleotides H1-H8, used previously for constructing the left half of the human insulin B chain gene (Goeddel, et al., 1979), contain the coding sequence for the 1-13 amino acids of the B chain gene and an additional N-terminal methionine. The right half of the B chain gene was constructed from oligonucleotides $B_1$, $B_2$, $B_3$, $B_4$, $B_5'$, $B_6$, $B_7$, $B_8$, $B_9$ and $B_{10}$ by ligation using T4 DNA ligase in substantial accordance with the teaching of Goeddel, et al., 1979. The resultant gene fragment codes for the 14-30 amino acid units of the human insulin B chain and the first arginine of the bridging chain. A HpaII restriction site is incorporated into the gene sequence in the same reading frame and location as the HpaII site in the human insulin gene. After purification of the ligated gene fragment by polyacrylamide gel electrophoresis and after elution of the largest DNA band, the fragment was inserted into HindIII-BamHI-cleaved plasmid pBR322. The resultant plasmid, designated pB3, was inserted into E. coli K12 294 by transformation. The plasmid conferred resistance to antibiotics ampicillin and tetracycline and was found to contain the desired nucleotide sequence.

Two fragments, a 58 base pair HindIII-BamHI fragment of pB3 and a 46 base pair EcoRI-HindIII fragment of pBH1 (disclosed in Goeddel, et al., 1979), were ligated to produce a fragment having EcoRI and BamHI termini. This fragment was conventionally ligated into EcoRI and BamHI restricted plasmid pBR322 and the resultant plasmid, designated pIB3, was then cloned into E. coli K12 294. After conventional amplification and isolation, plasmid pIB3 was digested with EcoRI and HpaII to produce a synthetic gene fragment (Fragment 1, Figure 15) that codes for the N-terminal proinsulin amino acids preceded by a methionine. The synthetic gene was isolated conventionally by polyacrylamide gel electrophoresis.

## I. Isolation of cDNA Coding for the 50 C-Terminal Amino Acids of Human Proinsulin

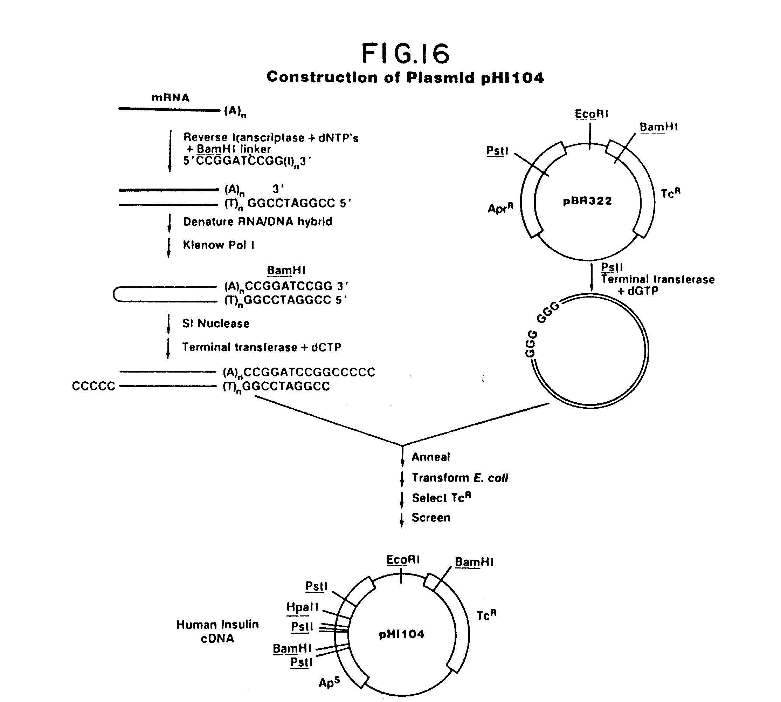

The scheme for obtaining the desired cDNA is presented in Figure 16 of the accompanying drawings. In accordance therewith, the DNA sequence $pCCGGATCCGGTTT_{18}T$, which contained both a BamHI recognition sequence and a 3' polythymidylic acid tract of approximately 20 residues, was synthesized and used to prime AMV reverse transcriptase for cDNA synthesis. The primer was prepared using terminal deoxynucleotidyl transferase (Enzo Biochem, 200 units) with 1 $\mu$mol of the BamHI decanucleotide in a reaction volume of 0.6 ml containing $1.5 \times 10^{-4}$ $\mu$mol TTP. The reaction was conducted at 37°C for 1 hour in the buffer system of Chang, et al., 1978, Nature 275:617.

Human insulinoma tissue was provided by the Institute für Diabetesforschung, Muenchen, West Germany. Those skilled in the art understand that human insulinoma tissue is readily available and can be obtained from a number of other sources in the medical community. Poly A mRNA (2.5 $\mu$g) of the human insulinoma tissue was isolated in substantial accordance with the procedure of Ullrich, et al., 1977, Science 196:1313 and then converted to double stranded cDNA in substantial accordance with the teaching of Wickens, et al., 1978, J. Biol. Chem. 253:2483. Thus, a reaction volume of 80 $\mu$l containing 15 mM Tris•HCl (pH 8.3 at 42°C), 21 mM KCl, 8 mM MgCl$_2$, 30 mM B-mercaptoethanol, 2 mM of the primer dCCGGATCCGGTT$_{18}$T, and 1 mM dNTPs was preincubated at 0°C. After addition of AMV reverse transcriptase, the mixture was incubated for 15 minutes at 42°C. The resultant RNA/DNA was then denatured using conventional procedures.

The complementary cDNA strand was synthesized in a reaction volume of 150 $\mu$l containing 25 mM Tris•HCl, pH 8.3, 35 mM KCl, 4 mM MgCl$_2$, 15 mM $\beta$-mercaptoethanol 1 mM dNTPs and 9 units of Klenow Polymerase I. The mixture was incubated at 15°C for 90 minutes followed by 15 hours at 4°C. S1 nuclease digestion was then performed for 2 hours at 37°C using 1000 units of S1 nuclease (Miles Laboratories, Elkhart, Indiana) in substantial accordance with the teaching of Wickens, et al., 1978. The double stranded cDNA (0.37 $\mu$g) was electrophoresed on an 8% polyacrylamide gel and DNA fragments larger than 500 base pairs were eluted. Oligodeoxycytidylic acid residues were added to the 3' ends of the fragments using terminal deoxynucleotidyl transferase in substantial accordance with the procedure of Maizel, 1971, Meth. Virol. 5:180. The dC tailed cDNA fragments were then annealed to pBR322 that had first been digested with PstI restriction enzyme and then tailed with deoxyguanidylic acid using terminal deoxynucleotidyl transferase. The resulting plasmids were used to transform E. coli K12 294 and the resultant cells plated on LB and TET medium. Colonies resistant to tetracycline but sensitive to ampicillin were isolated and screened for plasmids that had three PstI restriction sites. Such a restriction pattern is indicative of the gene for proinsulin, Sures, et al., 1980, Science 208:57. One plasmid, designated as pHI104, contained a 600 base pair insert, gave the anticipated PstI restriction pattern and contained a BamHI site between the 3' polyA and the polyGC introduced during the cDNA preparation. Some of the nucleotide sequence of the insert is shown in Figure 14.

## J. Assembly of a Gene Coding for Human Proinsulin

The scheme used for assembling a gene coding for human proinsulin is shown in Figure 15 of the accompanying drawings.

The synthetic gene segment coding for the first 31 amino acids of proinsulin, fragment 1 in Figure 15, was recovered from 50 $\mu$g of plasmid pIB3 using the restriction endonucleases EcoRI and HpaII as

described above. This fragment also contains the ATG sequence for methionine in place of the "presequence" of preproinsulin.

The cDNA gene segment coding for amino acids 32-86, as well as the translation stop signal and the 3' untranslated region of the mRNA, was recovered from 40 $\mu$g of plasmid pHI104 by treatment first with BamHI and then HpaII restriction enzymes. The two fragments were isolated by polyacrylamide gel electrophoresis followed by electroelution. The gene fragments were joined by treatment with T4 DNA ligase in 20 $\mu$l ligase buffer at 4°C for 24 hours. The mixture was diluted with 50 $\mu$l H$_2$O, conventionally extracted with each of phenol and chloroform and then precipitated with ethanol.

The resulting DNA was treated with BamHI and EcoRI restriction enzymes to regenerate these sites and remove gene polymers. The assembled proinsulin gene was isolated by polyacrylamide gel electrophoresis and ligated (using T4 DNA ligase) to EcoRI and BamHI digested plasmid pBR322. The resulting DNA was used to transform E. coli K12 294 and then the resultant colonies were screened for tetracycline sensitivity and ampicillin resistance. Plasmid pHI3, isolated from one such colony, contained the desired proinsulin gene which was subsequently characterized by nucleotide sequence analysis.

K. Construction of a Plasmid for Expression of a Chimeric Protein Containing Human Proinsulin

The complete human proinsulin gene, including the N-terminal codon that codes for methionine, was recovered from plasmid pHI3 by treatment with EcoRI and BamHI restriction enzymes. The desired fragment was purified by gel electrophoresis and then ligated (using T4 DNA ligase) to the plasmid pSOM7Δ2Δ4 PstI-EcoRI partial digest (prepared in Example 5G) and the larger of the PstI-BglII fragments of plasmid pHKY10 (prepared in Example 5F). Thus, about 1 $\mu$g of the complete human proinsulin gene with EcoRI and BamHI termini, 4 $\mu$g of PstI-EcoRI (partial) pSOM7Δ2Δ4 fragment, and about 1 $\mu$g of the PstI-BglII fragment of pHKY10 were ligated at 4°C. for 24 hours using T4 DNA ligase in ligation buffer. The ligated DNA mixture was used to transform E. coli K12 294. Colonies that grew on both ampicillin and tetracycline were selected and were found to contain the desired plasmid pHI7Δ4Δ1 and to express a protein of the molecular weight expected of the trp LE'- proinsulin fusion. Plasmid pHI7Δ4Δ1, which expressed the aforementioned protein, was completely characterized as to the DNA sequence and restriction sites of both the incorporated gene and also the vector. Plasmid pHI7Δ4Δ1 is depicted in Figure 15 of the accompanying drawings and can be selected readily because of the restoration of ampicillin and tetracycline resistance.

Example 6

## Construction of Plasmid pNM608 and E. coli K12 RV308/ pNM608

### A.   Construction of the ∿253 bp EcoRI-HpaI Fragment of Plasmid pHI7Δ4Δ1

About 5 $\mu$g of plasmid pHI7Δ4Δ1 DNA, 10 $\mu$l (10X) reaction buffer *, 80 $\mu$l water and 5 $\mu$l (5 units) of HpaI restriction enzyme were incubated at 370°C for about 1 hour. The reaction was terminated by incubation at 70°C for 5 minutes. After the mixture was cooled on ice, about 12 $\mu$l of 1M Tris•HCl, pH 7.2, 7 $\mu$l water and 1 $\mu$l (10 units) of EcoRI restriction enzyme were added. The resultant mixture was incubated first at 37°C for 1 hour and then at 70°C for 5 minutes. Following cooling on ice, the resultant DNA was extracted with each of phenol and chloroform:isoamyl alcohol (24:1) and then ethanol precipitated. The

* Reaction buffer (10X) for HpaI restriction enzyme was prepared with the following composition:
100 mM Tris HCl,   pH 7
100 mM MgCl$_2$
60 mM $\beta$-mercaptoethanol
200 mM KCl

26

desired ~253 bp EcoRI-HpaI fragment was conventionally separated by polyacrylamide gel electrophoresis, recovered by electroelution and ETOH precipitation and then dissolved in about 10 μl of TE buffer and stored at 0˚C for future use.

## B.   Construction of the ~34 bp HpaI-TaqI Fragment of Plasmid pHI7Δ4Δ1

About 20 μg of plasmid pHI7Δ4Δ1 DNA in 32 μl (5X) EcoRI reaction buffer [*], 124 μl water and 4 μl EcoRI restriction enzyme (20 units) were incubated at 37˚C for about 1 hour. The reaction was terminated by incubation at 70˚C for 5 minutes. The resulting 862 bp EcoRI fragment was isolated by electrophoresis on a 6% polyacrylamide gel followed by electroelution and ethanol precipitation. The fragment was then dissolved in about 100 μl of HpaI buffer containing 16 units of HpaI restriction enzyme. After 1.5 hours at 37˚C, 7.5 units of TaqI restriction enzyme were added, incubation was continued for an additional 1.5 hours and the resulting fragments conventionally separated on a 7½% polyacrylamide gel. The desired ~34 bp HpaI-TaqI fragment was recovered by electroelution and ethanol precipitation and was then dissolved in 5 μl of TE buffer.

250 mM NaCl
500 mM Tris•HCl, pH 7.2
25 mM MgCl₂
30 mM β-mercaptoethanol

## C.   EcoRI-ClaI Digestion of Plasmid pBR322

About 5 μg of plasmid pBR322 DNA, 10 μl of ClaI reaction mix [*], 77.5 μl water and 7.5 μl (15 units) of ClaI restriction enzyme were incubated at 37˚C for about 1 hour. The reaction was terminated by incubation at 70˚C for 5 minutes. After the mixture was cooled on ice, about 12.5 μl of 1M Tris•HCl, pH 7.2, 6.25 μl 1M NaCl, 2.5 μl 0.1M MgCl₂ and 1 μl (10 units) of EcoRI restriction enzyme were added. The resultant mixture was incubated first at 37˚C for 1 hour and then at 70˚C for 5 minutes. Following cooling on ice, the resultant DNA was subjected to electrophoresis on a 1% agarose gel. The large fragment was recovered by elution and ethanol precipitated and then dissolved in about 20 μl of TE buffer and stored at 0˚C for future use.

D. Ligation and Transformation

About 0.2 μg of the ~253 bp EcoRI-HpaI fragment of Example 6A, 0.5 μg of the HpaI-TaqI fragment of Example 6B and 0.1 μg of EcoRI-ClaI digest of Example 6C were ligated and used to transform E. coli K12 RV308 in substantial accordance with the teaching of Example 2D.

Some of the resultant transformants, as conventionally shown by agarose gel electrophoresis (Maniatis et al, 1982) and other tests, contained only the desired ~4.6 kb plasmid. Such a transformant, herein designated as E. coli K12 RV308/pNM608, was selected, plated on TY agar containing appropriate antibiotics and then cultured using conventional microbiological techniques. Plasmid pNM608 comprises and is a preferred source of the ~287 bp EcoRI-ClaI (TaqI) fragment of plasmid pHI7Δ4Δ1.

[*]Reaction buffer (5X) for Eco I restriction enzyme was prepared with the following composition:

[*]Reaction mix (10X) for Cla I restriction enzyme was prepared with the following composition:
100 mM Tris HCl,   pH 7.4
100 mM MgCl₂
60 mM β-mercaptoethanol

Example 7

## Construction of Plasmid pCZ112 and E. coli K12 RV308/pCZ112

### A. EcoRI-ClaI Digestion of Plasmid pNM608 and Generation of the ~0.288 kb EcoRI-TaqI Fragment (Same as the 0.288 kb EcoRI-TaqI Fragment of Plasmid pHI7Δ4Δ1)

About 5 $\mu$g of plasmid pNM608 DNA in 50 $\mu$l Medium Salt buffer *were incubated with 10 units each of EcoRI and ClaI restriction enzymes at 37° C for about 1 hour. After the addition of 5 $\mu$l of 3M sodium acetate, pH 7.0, the DNA was precipitated with 3 volumes of 100% ethanol. The desired DNA digest was dissolved in 100 $\mu$l of TE buffer and stored at 0° C for future use.

B. Construction of the DNA Linker Sequence

```
5'  CGACC ATG GAT GAT AAG TTT CCG GCT ATG TCT CTG
      ''' ''' ''' ''' ''' ''' ''' ''' ''' ''' '''
3'        TGG TAC CTA CTA TTC AAA GGC CGA TAC AGA GAC

    TCC GGC CTG TTT GCC AAC GCT GTGCT   3'
    ''' ''' ''' ''' ''' ''' ''' '''''
    AGG CCG GAC AAA CGG TTG CGA C        5'
```

The desired linker sequence is conventionally synthesized by the modified phosphotriester method in substantial accordance with the teaching of Itakura et al, 1977 and Crea et al., 1978. The aforementioned synthesis method is also specifically illustrated in Example 1.

C. Ligation and Transformation

About 20 picomoles of the DNA linker of Example 7B, 1 $\mu$g of the pNM608 digest of Example 7A, 0.5 $\mu$g of the plasmid pCZ101 ~10.2 kb BamHI-EcoRI fragment (prepared in accordance with the teaching of Example 2B except that EcoRI restriction enzyme and appropriate buffer, rather than XbaI restriction enzyme and buffer, are used), and 1 $\mu$g of the plasmid pCZ101 ~0.6 kb BamHI-HgiAI fragment of Example 3B are ligated and the resultant plasmid used to transform E. coli K12 RV308 in substantial accordance with the teaching of Example 2D.

Some of the resultant transformants, as conventionally shown by agarose gel electrophoresis (Maniatis et al., 1982) and other tests, contain only the desired ~10.8 kb plasmid. Such a transformant, herein designated as E. coli K12 RV308/pCZ112, is selected, plated on TY agar containing appropriate antibiotics and then cultured using conventional microbiological techniques. The resultant cells are shown, by SDS gel electrophoresis, RIA and other tests, to express the aforementioned MET-ASP-ASP-LYS-bGH derivative at high levels. Because plasmid pCZ112 contains a thermoinducible runaway replicon, maximum expression of

*Medium Salt buffer was prepared with the following composition:
50 mM NaCl
100 mM Tris HCl, pH 7.5
6 mM MgCl$_2$
2 mM $\beta$-mercaptoethanol

the desired bGH derivative product occurs at culture temperatures of about 37° C.

Example 8

## Construction of Plasmid pAT1 and E. coli K12 RV308/-pAT1

The desired constructions are made in substantial accordance with the teaching of Example 7 except that the DNA linker sequence

```
5' CGACA ATG TTC CCA GCT ATG TCT CTA TCT GGT
       ''' ''' ''' ''' ''' ''' ''' ''' ''' '''
3'     TGT TAC AAG GGT CGA TAC AGA GAT AGA CCA

      CTG TTT GCC AAC GCT GTGCT   3'
      ''' ''' ''' ''' '''   '
      GAC AAA CGG TTG CGA C       5'
```

is substituted for the linker sequence of Example 7. The above specified linker sequence can be constructed in substantial accordance with the conventional procedure of Itakura et al., 1977 and Crea et al., 1978. The aforementioned synthesis method is also specifically illustrated in Example 1.

The desired transformants, herein designated as E. coli K12 RV308/pAT1, are plated on TY agar containing appropriate antibiotics and then conventionally cultured for subsequent production and isolation of plasmid pAT1. The transformants are also shown, by SDS gel electrophoresis, RIA and other tests, to express the aforedefined MET-bGH derivative at high levels. Because plasmid pAT1 contains a thermoinducible runaway replicon, maximum expression of the desired bGH derivative product occurs at culture temperatures of about 37° C.

Example 9

## Construction of Plasmid pASP1 and E. coli K12 RV308/-pASP1

The desired constructions are made in substantial accordance with the teaching of Example 7 except that the DNA sequence

```
5' CGACC ATG GAT TTT CCG GCT ATG TCT CTG TCC GGC CTG
       ''' ''' ''' ''' ''' ''' ''' ''' ''' ''' ''' '''
3'     TGG TAC CTA AAA GGC CGA TAC AGC GAC AGG CCG GAC

      TTT GCC AAC GCT GTGCT   3'
      ''' ''' ''' '''   '
      AAA CGG TTG CGA C       5'
```

is substituted for the linker sequence of Example 7. The above specified linker sequence can be constructed in substantial accordance with the conventional procedure of Itakura et al., 1977 and Crea et al., 1978. The aforementioned synthesis method is also specifically illustrated in Example 1.

The desired transformants, herein designated as E. coli K12 RV308/pASP1 are plated on TY agar containing appropriate antibiotics and then conventionally cultured for subsequent production and isolation of plasmid pASP1. The transformants are also shown, by SDS gel electrophoresis, RIA and other tests, to express the aforedefined MET-ASP-bGH derivative at high levels. Because plasmid pASP1 contains a thermoinducible runaway replicon, maximum expression of the desired bGH derivative product occurs at culture temperatures of about 37° C.

Example 10

## Construction of Plasmid pASP2 and E. coli K12 RV308/- pASP2

The desired constructions are made in substantial accordance with the teaching of Example 7 except that the DNA sequence

```
5' CGATC ATG GAT TTT CCG GCT ATG TCT CTG TCC GGC CTG
         ::: ::: ::: ::: ::: ::: ::: ::: ::: ::: ::: :::
3'       TAG TAC CTA AAA GGC CGA TAC AGA GAC AGG CCG GAC

         TTT GCC AAC GCT GTGCT   3'
         ::: ::: ::: :::   :
         AAA CGG TTG CGA  C      5'
```

is substituted for the linker sequence of Example 7. The above specified linker sequence can be constructed in substantial accordance with the conventional procedure of Itakura et al., 1977 and Crea et al., 1978. The aforementioned synthesis method is also specifically illustrated in Example 1.

The desired transformants, herein designated as E. coli K12 RV308/pASP2 are plated on TY agar containing appropriate antibiotics and then conventionally cultured for subsequent production and isolation of plasmid pASP1. The transformants are also shown, by SDS gel electrophoresis, RIA and other tests, to express the aforedefined MET-ASP-bGH derivative at high levels. Because plasmid pASP2 contains a thermoinducible runaway replicon, maximum expression of the desired bGH derivative product occurs at culture temperatures of about 37° C.

Example 11

## Construction of Plasmid pAT2 and E. coli K12 RV308/- pAT2

The desired constructions are made in substantial accordance with the teaching of Example 3 except that the DNA linker sequence

```
5'    CTAGAGGGTATTAATA ATG TTT CCA GCT ATG TCT CTA TCT
      :::::::::::::::: ::: ::: ::: ::: ::: ::: ::: :::
3'     TCCCATAATTAT TAC AAA GGT CGA TAC AGA GAT AGA

      GGT CTG TTT GCC AAC GCT GTGCT    3'
      ::: ::: ::: ::: ::: :::   :
      CCA GAC AAA CGG TTG CGA  C       5'
```

is substituted for the linker sequence of Example 3C. The above-specified linker sequence can be constructed in substantial accordance with the conventional procedure of Itakura et al., 1977 and Crea et al., 1978. The aforementioned synthesis method is also specifically illustrated in Example 1.

The desired transformants, herein designated as E. coli K12 RV308/pAT2, are plated on TY agar containing appropriate antibiotics and then conventionally cultured for subsequent production and isolation of plasmid pAT2. The transformants are also shown, by SDS gel electrophoresis, RIA and other tests, to express the aforedefined MET-bGH derivative at high levels. Because plasmid pAT2 contains a thermoinducible runaway replicon, maximum expression of the desired bGH derivative product occurs at culture temperatures of about 37° C.

Example 12

## Construction of Plasmid pCZ154 and E. coli K12 RV308/- pCZ154

The desired constructions were made in substantial accordance with the teaching of Example 7 except that the DNA linker sequence

```
5'    CGACC ATG GTT TTT CCG GCT ATG TCT CTG TCC GGC CTG
      ||| ||| ||| ||| ||| ||| ||| ||| ||| ||| ||| |||
3'          TGG TAC CAA AAA GGC CGA TAC AGA GAC AGG CCG GAC

      TTT GCC AAC GCT GTGCT     3'
      ||| ||| ||| ||| |
      AAA CGG TTG CGA C         5'
```

was substituted for the linker sequence of Example 7. The above specified linker sequence can be constructed in substantial accordance with the conventional procedure of Itakura et al., 1977 and Crea et al., 1978. The aforementioned synthesis method is also specifically illustrated in Example 1.

The desired transformants, herein designated as E. coli K12 RV308/pCZ154, were plated on TY agar containing appropriate antibiotics and then conventionally cultured for subsequent production and isolation of plasmid pCZ154. The transformants were also shown, by SDS gel electrophoresis, RIA and other tests, to express the aforedefined MET-VAL-bGH derivative at high levels. Because plasmid pCZ154 contains a thermoinducible runaway replicon, maximum expression of the desired bGH derivative product occurs at culture temperatures of about 37° C.

Example 13

## Construction of Plasmid pCZ155 and E. coli K12 RV308/- pCZ155

The desired constructions were made in substantial accordance with the teaching of Example 7 except that the DNA linker sequence

```
5'    CGACC ATG GCT TTT CCG GCT ATG TCT CTG TCC GTC CTG
      ||| ||| ||| ||| ||| ||| ||| ||| ||| ||| ||| |||
3'          TGG TAC CGA AAA GGC CGA TAC AGA GAC AGG CAG GAC

      TTT GCC AAC GCT GTGCT     3'
      ||| ||| ||| ||| |
      AAA CGG TTG CGA C         5'
```

was substituted for the linker sequence of Example 7. The above specified linker sequence can be constructed in substantial accordance with the conventional procedure of Itakura et al., 1977 and Crea et al., 1978. The aforementioned synthesis method is also specifically illustrated in Example 1.

The desired transformants, herein designated as E. coli K12 RV308/pCZ155, were plated on TY agar containing appropriate antibiotics and then conventionally cultured for subsequent production and isolation of plasmid pCZ155. The transformants were also shown, by SDS gel electrophoresis, RIA and other tests, to express the aforedefined MET-ALA-PHE-PRO-ALA-MET-SER-LEU-SER-VAL-b'GH derivative at high levels. Because plasmid pCZ155 contains a thermoinducible runaway replicon, maximum expression of the desired bGH derivative product occurs at culture temperatures of about 37° C.

Example 14

# Construction of Plasmid pCZ156 and E. coli K12 RV308/-pCZ156

The desired constructions were made in substantial accordance with the teaching of Example 7 except that the DNA linker sequence

```
5'    CGATA ATG GAT TTT CCG GCT ATG TCT CTG TCC GGC CTG
            ¦¦¦ ¦¦¦ ¦¦¦ ¦¦¦ ¦¦¦ ¦¦¦ ¦¦¦ ¦¦¦ ¦¦¦ ¦¦¦ ¦¦¦
3'        TAT TAC CTA AAA GGC CGA TAC AGA GAC AGG CCG GAC

      TTT GCC AAC GCT GTGCT     3'
      ¦¦¦ ¦¦¦ ¦¦¦ ¦¦¦ ¦
      AAA CGG TTG CGA C         5'
```

was substituted for the linker sequence of Example 7. The above specified linker sequence can be constructed in substantial accordance with the conventional procedure of Itakura et al., 1977 and Crea et al., 1978. The aforementioned synthesis method is also specifically illustrated in Example 1.

The desired transformants, herein designated as E. coli K12 RV308/pCZ156, were plated on TY agar containing appropriate antibiotics and then conventionally cultured for subsequent production and isolation of plasmid pCZ156. The transformants were also shown, by SDS gel electrophoresis, RIA and other tests, to express the aforedefined MET-ASP-bGH derivative at high levels. Because plasmid pCZ156 contains a thermoinducible runaway replicon, maximum expression of the desired bGH derivative product occurs at culture temperatures of about 37° C.

Example 15

# Construction of Plasmid pCZ103 and E. coli K12 RV308/pCZ103

Ten μg of plasmid pCZ101 were dissolved in 10 μl 10X BstEII reaction buffer (1.5 M NaCl; 60 mM Tris-HCl, pH=7.9; 60 mM MgCl₂; 60 mM 2-mercaptoethanol; and 1 mg/ml BSA), 2 μl (~20 units) restriction enzyme BstEII, and 88 μl of H₂O, and the resulting reaction was incubated at 60° C for two hours. After phenol and chloroform extractions, several ethanol precipitations of the DNA were done from a 0.25 M NaOAc solution.

About 0.1 μg of the BstEII-digested plasmid pCZ101 DNA was suspended in 100 μl of ligase buffer. After adding 100 units of T4 DNA ligase and incubating at 16° C for 2 hours, the ligated DNA was used to conventionally transform E. coli K12 RV308. E. coli K12 RV308/pCZ103 transformants were selected on kanamycin and identified by restriction enzyme analysis of their plasmid DNA. Plasmid pCZ103 was isolated from the transformants in substantial accordance with the procedure of Example 2A.

Example 16

Construction of Plasmid pCZ103-Derived Vectors For Expressing Bovine Growth Hormone Derivatives

32

Since the BstEII deletion done to construct plasmid pCZ103 does not affect the bGH protein-coding sequence, the plasmid pCZ103-derived plasmids of the present invention express the same bGH derivative as their plasmid pCZ101-derived counterparts.

Since the pCZ103-derived plasmids of the present invention were constructed in substantial accordance with the teaching of the Examples in which their pCZ101-derived counterparts were constructed, Table 3 is presented to briefly summarize the construction of the pCZ103-derived plasmids. Table 3 lists the pCZ103-derived plasmid, the corresponding Example teaching the construction of the pCZ101-derived counterpart, and the size of the only different DNA fragment involved in the construction.

Table 3

Construction Of pCZ103-Derived Vectors For Expressing bGH Derivatives

| pCZ103-Derived Vector | Corresponding Example | Different Restriction Fragment Used | Notes |
|---|---|---|---|
| pJR1.3 | 4 | The ~9.3 kb BamHI-XbaI restriction fragment of plasmid pCZ103 was used instead of the ~10.2 kb BamHI-XbaI restriction fragment of plasmid pCZ101. | The other DNA fragments used were the same as those used in the referenced Examples. Note that the ~0.6 kb BamHI-HgiAI restriction fragments of plasmids pCZ101 and pCZ103 are identical. |
| pAT2.3 | 11 | | |
| pAT1.3 | 8 | The ~9.3 kb BamHI-EcoRI restriction fragment of plasmid pCZ103 was used instead of the ~10.2 kb BamHI-EcoRI restriction fragment of plasmid pCZ101. | The other DNA fragments used were the same as those used in the referenced Examples. |
| pASP1.3 | 9 | | |
| pASP2.3 | 10 | | |
| pCZ154.3 | 12 | | |
| pCZ155.3 | 13 | | |
| pCZ156.3 | 14 | | |

The pCZ103-derived plasmids were used to conventionally transform E. coli K12 RV308. The resultant transformants express the aforementioned bGH derivatives. Because the pCZ103-derived plasmids contain a thermoinducible runaway replicon, maximum expression occurs at culture temperatures of about 37° C.

**Claims**

34

**Claims for the following Contracting States: BE, CH, DE, FR, GB, IT, LI, LU, NL, SE**

1. A selectable and autonomously replicating recombinant DNA expression vector which comprises
   a) a runaway replicon and
   b) a transcriptional and translational activating sequence which is in the reading frame of a sequence that codes for a bioactive bovine growth hormone derivative, said sequence being

```
(1)   5'   ATG AAA GGG AAT TCT ATG GCC TTC
           ''' ''' ''' ''' ''' ''' ''' '''
      3'   TAC TTT CCC TTA AGA TAC CGG AAG

           CCA GCC ATG TCC TTG TCC GGC        3'
           ''' ''' ''' ''' ''' ''' '''   1
           GGT CGG TAC AGG AAC AGG CCG-R-R    5',

(2)   5'   ATG GAT GAT AAG TTT CCG GCT ATG
           ''' ''' ''' ''' ''' ''' ''' '''
      3'   TAC CTA CTA TTC AAA GGC CGA TAC

           TCT CTG TCC GGC        3'
           ''' ''' ''' '''   1
           AGA GAC AGG CCG-R-R    5',

(3)   5'   ATG TTT CCA GCC ATG GCT CTA
           ''' ''' ''' ''' ''' ''' '''
      3'   TAC AAA GGT CGG TAC CGA GAT

           TCT GGT        3'
           ''' '''   1
           AGA CCA-R-R    5',

(4)   5'   ATG TTC CCA GCT ATG TCT CTA
           ''' ''' ''' ''' ''' ''' '''
      3'   TAC AAG GGT CGA TAC AGA GAT

           TCT GGT        3'
           ''' '''   1
           AGA CCA-R-R    5',

(5)   5'   ATG GAT TTT CCG GCT ATG TCT
           ''' ''' ''' ''' ''' ''' '''
      3'   TAC CTA AAA GGC CGA TAC AGA

           CTG TCC GGC        3'
           ''' ''' '''   1
           GAC AGG CCG-R-R    5',

  or

(6)   5'   ATG TTT CCA GCT ATG TCT CTA
           ''' ''' ''' ''' ''' ''' '''
      3'   TAC AAA GGT CGA TAC AGA GAT

           TCT GGT        3'
           ''' '''   1
           AGA CCA-R-R    5'
```

wherein
A is deoxyadenyl,
G is deoxyguanyl,
C is deoxycytosyl,
T is thymidyl,

R is a DNA sequence that codes for amino acids 9 (leucine) through 191 (phenylalanine) of bovine growth hormone, and

R¹ is a DNA sequence that codes for a translational stop signal which is

```
5' TAA 3'    5' TAG 3'
   ¦¦¦          ¦¦¦
3' ATT 5',   3' ATC 5',      or

5' TGA 3'
   ¦¦¦
3' ACT 5'    .
```

**2.** The vector of Claim 1 which is a plasmid.

**3.** The vector of Claim 1 or 2 in which the transcriptional activating sequence is the E. coli lactose, bacteriophage λ P$_L$O$_L$, bacteriophage λ P$_R$L$_R$, tac, E. coli lipoprotein or E. coli tryptophan transcriptional activating sequence.

**4.** A vector of claim 1, 2 or 3 which is:

plasmid pCZ1920 prepared by ligating the ~10.2 kb BamHI-XbaI fragment of plasmid pCZ101, a DNA linker having the nucleotide sequence

```
5' CTAGAGGGTATTAATA ATG AAA GGG AAT TCT ATG GCC TTC CCA GCC
   ¦¦¦¦¦¦¦¦¦¦¦¦¦¦¦¦ ¦¦¦ ¦¦¦ ¦¦¦ ¦¦¦ ¦¦¦ ¦¦¦ ¦¦¦ ¦¦¦ ¦¦¦
3'     TCCCATAATTAT TAC TTT CCC TTA AGA TAC CGG AAG GGT CGG

ATG TCC TTG TCC GGC CTG TTT GCC AAC GCT GTGCT  3'
¦¦¦ ¦¦¦ ¦¦¦ ¦¦¦ ¦¦¦ ¦¦¦ ¦¦¦ ¦¦¦ ¦¦¦ ¦¦¦ ¦
TAC AGG AAC AGG CCG GAC AAA CGG TTG CGA C      5'
```

and the ~0.6 kb BamHI-XbaI fragment of plasmid pCZ101,

said plasmid pCZ101 being constructed by ligating the ~0.6 kb XbaI-BamHI fragment of plasmid pNM789B derived from plasmid pKEN111 (obtainable from NRRL B-15011) as described in Figures 1-8 and Example 1, into similarly digested plasmid pIM-I'-A3 (obtainable from NRRL B-15733),

plasmid pJR1 prepared by ligating the ~10.2 kb BamHI-XbaI fragment of plasmid pCZ101, a DNA linker having the nucleotide sequence

```
5' CTAGAGGGTATTAATA ATG TTT CCA GCC ATG GCT CTA TCT GGT
   ¦¦¦¦¦¦¦¦¦¦¦¦¦¦¦¦ ¦¦¦ ¦¦¦ ¦¦¦ ¦¦¦ ¦¦¦ ¦¦¦ ¦¦¦ ¦¦¦ ¦¦¦
3'     TCCCATAATTAT TAC AAA GGT CGG TAC CGA GAT AGA CCA

CTG TTT GCC AAC GCT GTGCT  3'
¦¦¦ ¦¦¦ ¦¦¦ ¦¦¦ ¦¦¦ ¦
GAC AAA CGG TTG CGA C      5'
```

and the ~0.6 kb BamHI-XbaI fragment of plasmid pCZ101,

plasmid pCZ112 prepared by ligating the ~10.2 kb BamHI-EcoRI fragment of plasmid pCZ101, a DNA linker having the nucleotide sequence

```
5'   CGACC ATG GAT GAT AAG TTT CCG GCT ATG TCT CTG
     ¦¦¦ ¦¦¦ ¦¦¦ ¦¦¦ ¦¦¦ ¦¦¦ ¦¦¦ ¦¦¦ ¦¦¦ ¦¦¦ ¦¦¦
3'        TGG TAC CTA CTA TTC AAA GGC CGA TAC AGA GAC

     TCC GGC CTG TTT GCC AAC GCT GTGCT   3'
     ¦¦¦ ¦¦¦ ¦¦¦ ¦¦¦ ¦¦¦ ¦¦¦ ¦¦¦ ¦
     AGG CCG GAC AAA CGG TTG CGA C          5'
```

and the EcoRI-ClaI digest of plasmid pNM608,

said plasmid pNM608 being constructed by ligating the EcoRI-ClaI fragment of plasmid pH17Δ4Δ1 into EcoRI-ClaI-digested plasmid pBR322, plasmid pH17Δ4Δ1 being constructed as described in Example 5,

plasmid pAT1 prepared by ligating the ~10.2 kb BamHI-EcoRI fragment of plasmid pCZ101, a DNA linker having the nucleotide sequence

```
5'   CGACA ATG TTC CCA GCT ATG TCT CTA TCT GGT
     ¦¦¦ ¦¦¦ ¦¦¦ ¦¦¦ ¦¦¦ ¦¦¦ ¦¦¦ ¦¦¦ ¦¦¦ ¦¦¦
3'        TGT TAC AAG GGT CGA TAC AGA GAT AGA CCA

     CTG TTT GCC AAC GCT GTGCT   3'
     ¦¦¦ ¦¦¦ ¦¦¦ ¦¦¦ ¦¦¦ ¦
     GAC AAA CGG TTG CGA C          5'
```

and the EcoRI-ClaI digest of plasmid pNM608,

plasmid pASP1 prepared by ligating the ~10.2 kb BamHI-EcoRI fragment of plasmid pCZ101, a DNA linker having the nucleotide sequence

```
5'   CGACC ATG GAT TTT CCG GCT ATG TCT CTG TCC GGC CTG
     ¦¦¦ ¦¦¦ ¦¦¦ ¦¦¦ ¦¦¦ ¦¦¦ ¦¦¦ ¦¦¦ ¦¦¦ ¦¦¦ ¦¦¦ ¦¦¦
3'        TGG TAC CTA AAA GGC CGA TAC AGC GAC AGG CCG GAC

     TTT GCC AAC GCT GTGCT   3'
     ¦¦¦ ¦¦¦ ¦¦¦ ¦¦¦ ¦
     AAA CGG TTG CGA C          5'
```

and the EcoRI-ClaI digest of plasmid pNM608,

plasmid pAT2 prepared by ligating the ~10.2 kb BamHI-XbaI fragment of plasmid pCZ101, a DNA linker having the nucleotide sequence

```
5' CTAGAGGGTATTAATA ATG TTT CCA GCT ATG TCT CTA TCT GGT
   ¦¦¦¦¦¦¦¦¦¦¦¦¦¦¦¦¦ ¦¦¦ ¦¦¦ ¦¦¦ ¦¦¦ ¦¦¦ ¦¦¦ ¦¦¦ ¦¦¦ ¦¦¦
3'        TCCCATAATTAT TAC AAA GGT CGA TAC AGA GAT AGA CCA

     CTG TTT GCC AAC GCT GTGCT   3'
     ¦¦¦ ¦¦¦ ¦¦¦ ¦¦¦ ¦¦¦ ¦
     GAC AAA CGG TTG CGA C          5'
```

and the ~0.6 kb BamHI-XbaI fragment of plasmid pCZ101,

plasmid pASP2 prepared by ligating the ~10.2 kb BamHI-EcoRI fragment of plasmid pCZ101, a DNA linker having the nucleotide sequence

```
5'   CGATC ATG GAT TTT CCG GCT ATG TCT CTG TCC GGC CTG
     ::: ::: ::: ::: ::: ::: ::: ::: ::: ::: ::: :::
3'       TAG TAC CTA AAA GGC CGA TAC AGA GAC AGG CCG GAC


     TTT GCC AAC GCT GTGCT  3'
     ::: ::: ::: ::: :
     AAA CGG TTG CGA C       5'
```

and the EcoRI-ClaI digest of plasmid pNM608,

plasmid pJR1.3 prepared by ligating the ~9.3 kb BamHI-XbaI fragment of plasmid pCZ103, a DNA linker having the nucleotide sequence

```
5' CTAGAGGGTATTAATA ATG TTT CCA GCC ATG GCT CTA TCT GGT
   '''''''''''''''' ::: ::: ::: ::: ::: ::: ::: ::: :::
3'      TCCCATAATTAT TAC AAA GGT CGG TAC CGA GAT AGA CCA


   CTG TTT GCC AAC GCT GTGCT  3'
   ::: ::: ::: ::: ::: :
   GAC AAA CGG TTG CGA C       5'
```

and the ~0.6 kb BamHI-XbaI fragment of plasmid pCZ101,

said plasmid pCZ103 being constructed by deletion of the ~900 bp BstEII restriction fragment of plasmid pCZ101 and recircularisation,

plasmid pAT1.3 prepared by ligating the ~9.3 kb BamHI-EcoRI fragment of plasmid pCZ103, a DNA linker having the nucleotide sequence

```
5'   CGACA ATG TTC CCA GCT ATG TCT CTA TCT GGT
     ::: ::: ::: ::: ::: ::: ::: ::: ::: :::
3'       TGT TAC AAG GGT CGA TAC AGA GAT AGA CCA


     CTG TTT GCC AAC GCT GTGCT  3'
     ::: ::: ::: ::: ::: :
     GAC AAA CGG TTG CGA C       5'
```

and the EcoRI-ClaI digest of plasmid pNM608,

plasmid pASP1.3 prepared by ligating the ~9.3 kb BamHI-EcoRI fragment of plasmid pCZ103, a DNA linker having the nucleotide sequence

```
5'   CGACC ATG GAT TTT CCG GCT ATG TCT CTG TCC GGC CTG
     ::: ::: ::: ::: ::: ::: ::: ::: ::: ::: ::: :::
3'       TGG TAC CTA AAA GGC CGA TAC AGC GAC AGG CCG GAC


     TTT GCC AAC GCT GTGCT  3'
     ::: ::: ::: ::: :
     AAA CGG TTG CGA C       5'
```

and the EcoRI-ClaI digest of plasmid pNM608,

plasmid pASP2.3 prepared by ligating the ~9.3 kb BamHI-EcoRI fragment of plasmid pCZ103, a DNA linker having the nucleotide sequence

```
5'   CGATC ATG GAT TTT CCG GCT ATG TCT CTG TCC GGC CTG
           ''' ''' ''' ''' ''' ''' ''' ''' ''' ''' '''
3'         TAG TAC CTA AAA GGC CGA TAC AGA GAC AGG CCG GAC


     TTT GCC AAC GCT GTGCT   3'
     ''' ''' ''' ''' '
     AAA CGG TTG CGA C       5'
```

and the EcoRI-ClaI digest of plasmid pNM608,

plasmid pCZ154.3 prepared by ligating the ~9.3 kb BamHI-EcoRI fragment of plasmid pCZ103, a DNA linker having the nucleotide sequence

```
5'   CGACC ATG GTT TTT CCG GCT ATG TCT CTG TCC GGC CTG
           ''' ''' ''' ''' ''' ''' ''' ''' ''' ''' '''
3'         TGG TAC CAA AAA GGC CGA TAC AGA GAC AGG CCG GAC


     TTT GCC AAC GCT GTGCT   3'
     ''' ''' ''' ''' '
     AAA CGG TTG CGA C       5'
```

and the EcoRI-ClaI digest of plasmid pNM608,

plasmid pCZ155.3 prepared by ligating the ~9.3 kb BamHI-EcoRI fragment of plasmid pCZ103, a DNA linker having the nucleotide sequence

```
5'   CGACC ATG GCT TTT CCG GCT ATG TCT CTG TCC GTC CTG
           ''' ''' ''' ''' ''' ''' ''' ''' ''' ''' '''
3'         TGG TAC CGA AAA GGC CGA TAC AGA GAC AGG CAG GAC


     TTT GCC AAC GCT GTGCT   3'
     ''' ''' ''' ''' '
     AAA CGG TTG CGA C       5'
```

and the EcoRI-ClaI digest of pNM608, or

plasmid pCZ156.3 prepared by ligating the ~9.3 kb BamHI-EcoRI fragment of plasmid pCZ103, a DNA linker having the nucleotide sequence

```
5'   CGATA ATG GAT TTT CCG GCT ATG TCT CTG TCC GGC CTG
           ''' ''' ''' ''' ''' ''' ''' ''' ''' ''' '''
3'         TAT TAC CTA AAA GGC CGA TAC AGA GAC AGG CCG GAC


     TTT GCC AAC GCT GTGCT   3'
     ''' ''' ''' ''' '
     AAA CGG TTG CGA C       5'
```

and the EcoRI-ClaI digest of pNM608.

5. The bovine growth hormone derivative coded for by the vector of any of claims 1 to 4 which is MET-PHE-PRO-ALA-MET-ALA-$R^2$, MET-ASP-ASP-LYS-bGH or MET-ASP-bGH
wherein
MET is methionine,
PHE is phenylalanine,
PRO is proline,
ALA is alanine,
ASP is aspartic acid,

LYS is lysine,

R² is the natural amino acid sequence of bovine growth hormone beginning with the sixth amino acid (leucine) from the N-terminus and

bGH is the natural amino acid sequence of bovine growth hormone beginning with the N-terminal (first) phenylalanine.

6. A host cell comprising a recombinant DNA expression vector of any of Claims 1 to 4.

7. The cell of Claim 6 which is E. coli.

8. The cell of Claim 7 which is E. coli K12 RV308.

9. A method for increasing milk production in cows comprising administering to said cows a bovine growth hormone derivative coded for by the vector of any of Claims 1 to 4, exclusive of methionyl bovine growth hormone, in an amount effective for increasing said production.

10. The method of Claim 9 wherein the amount administered is from 0.05 to 100,000 micrograms, preferably from 5 to 25,000 micrograms, most preferably from 500 to 5,000 micrograms per animal per day.

11. Plasmid pCZ103 constructed by deletion of the ~900 bp BstEII restriction fragment of plasmid pCZ101 and recircularization, said plasmid pCZ101 being constructed by ligating the ~0.6 kb XbaI-BamHI fragment of plasmid pNM789B derived from plasmid pKEN111 (obtainable from NRRL 5-15011) as described in Figures 1-8 and Example 1, into similarly digested pIM-I'-A3 (obtainable from NRRL B-15733).

**Claims for the following Contracting State: AT**

1. A process for preparing a selectable and autonomously replicating recombinant DNA expression vector which comprises ligating

   a) a runaway replicon and

   b) a transcriptional and translational activating sequence which is in the reading frame of a sequence that codes for a bioactive bovine growth hormone derivative, said sequence being

```
(1)  5'   ATG AAA GGG AAT TCT ATG GCC TTC
          ||| ||| ||| ||| ||| ||| ||| |||
     3'   TAC TTT CCC TTA AGA TAC CGG AAG

          CCA GCC ATG TCC TTG TCC GGC        3'
          ||| ||| ||| ||| ||| ||| |||-R-R¹
          GGT CGG TAC AGG AAC AGG CCG        5',

(2)  5'   ATG GAT GAT AAG TTT CCG GCT ATG
          ||| ||| ||| ||| ||| ||| ||| |||
     3'   TAC CTA CTA TTC AAA GGC CGA TAC

          TCT CTG TCC GGC        3'
          ||| ||| ||| |||-R-R¹
          AGA GAC AGG CCG        5',

(3)  5'   ATG TTT CCA GCC ATG GCT CTA
          ||| ||| ||| ||| ||| ||| |||
     3'   TAC AAA GGT CGG TAC CGA GAT

          TCT GGT        3'
          ||| |||-R-R¹
          AGA CCA        5',

(4)  5'   ATG TTC CCA GCT ATG TCT CTA
          ||| ||| ||| ||| ||| ||| |||
     3'   TAC AAG GGT CGA TAC AGA GAT

          TCT GGT        3'
          ||| |||-R-R¹
          AGA CCA        5',

(5)  5'   ATG GAT TTT CCG GCT ATG TCT
          ||| ||| ||| ||| ||| ||| |||
     3'   TAC CTA AAA GGC CGA TAC AGA


          CTG TCC GGC        3'
          ||| ||| |||-R-R¹
          GAC AGG CCG        5',

         or

(6)  5'   ATG TTT CCA GCT ATG TCT CTA
          ||| ||| ||| ||| ||| ||| |||
     3'   TAC AAA GGT CGA TAC AGA GAT

          TCT GGT        3'
          ||| |||-R-R¹
          AGA CCA        5'
```

wherein

A is deoxyadenyl,

G is deoxyguanyl,

C is deoxycytosyl,

T is thymidyl,

R is a DNA sequence that codes for amino acids 9 (leucine) through 191 (phenylalanine) of bovine growth hormone, and

$R^1$ is a DNA sequence that codes for a translational stop signal which is

```
5' TAA 3'    5' TAG 3'
   ' ' '        ' ' '
3' ATT 5',   3' ATC 5',      or

5' TGA 3'
   ' ' '
3' ACT 5'    .
```

**2.** The process of Claim 1 in which the transcriptional activating sequence is the E. coli lactose, bacteriophage λ P$_L$O$_L$, bacteriophage λ P$_R$O$_R$, tac, E. coli lipoprotein or E. coli tryptophan transcriptional activating sequence.

**3.** The process of Claim 1 or 2 in which the vector is a plasmid.

**4.** The process of Claim 3 in which the translational activating sequence is the E. coli lipoprotein sequence.

**5.** The process of Claim 4 for preparing plasmid pCZ1920 which comprises ligating the ~10.2 kb BamHI-XbaI fragment of plasmid pCZ101, a DNA linker having the nucleotide sequence

```
5' CTAGAGGGTATTAATA ATG AAA GGG AAT TCT ATG GCC TTC CCA GCC
   ''''''''''''''''' ''' ''' ''' ''' ''' ''' ''' ''' ''' '''
3'     TCCCATAATTAT TAC TTT CCC TTA AGA TAC CGG AAG GGT CGG

ATG TCC TTG TCC GGC CTG TTT GCC AAC GCT GTGCT  3'
''' ''' ''' ''' ''' ''' ''' ''' ''' ''' '
TAC AGG AAC AGG CCG GAC AAA CGG TTG CGA C      5'
```

and the ~0.6 kb BamHI-XbaI fragment of plasmid pCZ101, said plasmid pCZ101 being constructed by ligating the ~0.6 hb XbaI-BamHI fragment of plasmid pNM789B derived from plasmid pKEN111 (obtainable from NRRL B-15011) as described in figures 1-8 and Example 1, into similar digested plasmid pIM-I'-A3 (obtainable from NRRL B-15733 ).

**6.** The process of Claim 4 for preparing plasmid pJR1 which comprises ligating the ~10.2 kb BamHI-XbaI fragment of plasmid pCZ101 as defined in claim 5, a DNA linker having the nucleotide sequence

```
5' CTAGAGGGTATTAATA ATG TTT CCA GCC ATG GCT CTA TCT GGT
   ''''''''''''''''' ''' ''' ''' ''' ''' ''' ''' '''
3'     TCCCATAATTAT TAC AAA GGT CGG TAC CGA GAT AGA CCA

CTG TTT GCC AAC GCT GTGCT  3'
''' ''' ''' ''' ''' '
GAC AAA CGG TTG CGA C      5'
```

and the ~0.6 kb BamHI-XbaI fragment of plasmid pCZ101.

**7.** The process of Claim 4 for preparing plasmid pAT2 which comprises ligating the ~10.2 kb BamHI-XbaI fragment of plasmid pCZ101 as defined in claim 5, a DNA linker having the nucleotide sequence

```
5' CTAGAGGGTATTAATA ATG TTT CCA GCT ATG TCT CTA TCT GGT
   ''''''''''''''''' ''' ''' ''' ''' ''' ''' ''' ''' '''
3'     TCCCATAATTAT TAC AAA GGT CGA TAC AGA GAT AGA CCA

CTG TTT GCC AAC GCT GTGCT  3'
''' ''' ''' ''' ''' '
GAC AAA CGG TTG CGA C      5'
```

and the ~0.6 kb BamHI-XbaI fragment of plasmid pCZ101.

8. The process of Claim 4 for preparing plasmid pJR1.3 which comprises ligating the ~9.3 kb BamHI-XbaI fragment of plasmid pCZ103, a DNA linker having the nucleotide sequence

```
5' CTAGAGGGTATTAATA ATG TTT CCA GCC ATG GCT CTA TCT GGT
   ''''''''''''''''' ''' ''' ''' ''' ''' ''' ''' ''' '''
3'         TCCCATAATTAT TAC AAA GGT CGG TAC CGA GAT AGA CCA

   CTG TTT GCC AAC GCT GTGCT  3'
   ''' ''' ''' ''' '''  '
   GAC AAA CGG TTG CGA C      5'
```

and the ~0.6 kb BamHI-XbaI fragment of plasmid pCZ101 as defined in Claim 5, said pCZ103 being constructed by deletion of the ~900 bp BstEII restriction fragment of plasmid pCZ101 and recircularization.

9. The process of Claim 3 in which the translational activating sequence is the E. coli tryptophan sequence.

10. The process of Claim 9 for preparing plasmid pCZ112 which comprises ligating the ~10.2 kb BamHI-EcoRI fragment of plasmid pCZ101 as defined in Claim 5, a DNA linker having the nucleotide sequence

```
5'  CGACC ATG GAT GAT AAG TTT CCG GCT ATG TCT CTG
    ''' ''' ''' ''' ''' ''' ''' ''' ''' ''' '''
3'     TGG TAC CTA CTA TTC AAA GGC CGA TAC AGA GAC
       '
    TCC GGC CTG TTT GCC AAC GCT GTGCT  3'
    ''' ''' ''' ''' ''' ''' '''  '
    AGG CCG GAC AAA CGG TTG CGA C      5'
```

and the EcoRI-ClaI digest of plasmid NM608, said plasmid pNM603 being constructed by ligating the EcoRI-ClaI fragment of plasmid pH17Δ4Δ1 into EcoRI-ClaI-digested plasmid pBR322, plasmid pH17Δ4Δ1 being constructed as described in Example 5.

11. The process of Claim 9 for preparing plasmid pAT1 which comprises ligating the ~10.2 kb BamHI-EcoRI fragment of plasmid pCZ101 as defined in Claim 5, a DNA linker having the nucleotide sequence

```
5'  CGACA ATG TTC CCA GCT ATG TCT CTA TCT GGT
    ''' ''' ''' ''' ''' ''' ''' ''' ''' '''
3'     TGT TAC AAG GGT CGA TAC AGA GAT AGA CCA

    CTG TTT GCC AAC GCT GTGCT  3'
    ''' ''' ''' ''' '''  '
    GAC AAA CGG TTG CGA C      5'
```

and the EcoRI-ClaI digest of plasmid pNM608 as defined in claim 10.

12. The process of Claim 9 for preparing plasmid pASP1 which comprises ligating the ~10.2 kb BamHI-EcoRI fragment of plasmid pCZ101 as defined in claim 5, a DNA linker having the nucleotide sequence

```
5'   CGACC ATG GAT TTT CCG GCT ATG TCT CTG TCC GGC CTG
     ||| ||| ||| ||| ||| ||| ||| ||| ||| ||| ||| |||
3'     TGG TAC CTA AAA GGC CGA TAC AGC GAC AGG CCG GAC

     TTT GCC AAC GCT GTGCT   3'
     ||| ||| ||| ||| |
     AAA CGG TTG CGA C        5'
```

and the EcoRI-ClaI digest of plasmid pNM608 as defined in claim 10

13. The process of Claim 9 for preparing plasmid pASP2 which comprises ligating the ~10.2 kb BamHI-EcoRI fragment of plasmid pCZ101 as defined in claim 5, a DNA linker having the nucleotide sequence

```
5'   CGATC ATG GAT TTT CCG GCT ATG TCT CTG TCC GGC CTG
     ||| ||| ||| ||| ||| ||| ||| ||| ||| ||| ||| |||
3'     TAG TAC CTA AAA GGC CGA TAC AGA GAC AGG CCG GAC

     TTT GCC AAC GCT GTGCT   3'
     ||| ||| ||| ||| |
     AAA CGG TTG CGA C        5'
```

and the EcoRI-ClaI digest of plasmid NM608 as defined in claim 10

14. The process of Claim 9 for preparing plasmid pCZ154 which comprises ligating the ~10.2 kb BamHI-EcoRI fragment of plasmid pCZ101 as defined in claim 5, a DNA linker having the nucleotide sequence

```
5'   CGACC ATG GTT TTT CCG GCT ATG TCT CTG TCC GGC CTG
     ||| ||| ||| ||| ||| ||| ||| ||| ||| ||| ||| |||
3'     TGG TAC CAA AAA GGC CGA TAC AGA GAC AGG CCG GAC

     TTT GCC AAC GCT GTGCT   3'
     ||| ||| ||| ||| |
     AAA CGG TTG CGA C        5'
```

and the EcoRI-ClaI digest of pNM608 as defined in claim 10

15. The process of Claim 9 for preparing plasmid pCZ155 which comprises ligating the ~10.2 kb BamHI-EcoRI fragment of plasmid pCZ101 as defined in claim 5, a DNA linker having the nucleotide sequence

```
5'   CGACC ATG GCT TTT CCG GCT ATG TCT CTG TCC GTC CTG
     ||| ||| ||| ||| ||| ||| ||| ||| ||| ||| ||| |||
3'     TGG TAC CGA AAA GGC CGA TAC AGA GAC AGG CAG GAC

     TTT GCC AAC GCT GTGCT    3'
     ||| ||| ||| ||| |
     AAA CGG TTG CGA C         5'
```

and the EcoRI-ClaI digest of NM608 as defined in claim 10

16. The process of Claim 9 for preparing plasmid pCZ156 which comprises ligating the ~10.2 kb BamHI-EcoRI fragment of plasmid pCZ101 as defined in claim 5, a DNA linker having the nucleotide sequence

```
5'  CGATA ATG GAT TTT CCG GCT ATG TCT CTG TCC GGC CTG
    ''' ''' ''' ''' ''' ''' ''' ''' ''' ''' ''' '''
3'    TAT TAC CTA AAA GGC CGA TAC AGA GAC AGG CCG GAC


    TTT GCC AAC GCT GTGCT    3'
    ''' ''' ''' ''' '
    AAA CGG TTG CGA C         5'
```

and the EcoRI-ClaI digest of pNM608 as defined in claim 10

17. The process of Claim 9 for preparing plasmid pAT2.3 which comprises ligating the ~9.3 kb BamHI-EcoRI fragment of plasmid pCZ103 a DNA linker having the nucleotide sequence

```
5' CTAGAGGGTATTAATA ATG TTT CCA GCT ATG TCT CTA TCT GGT
   ''''''''''''''''' ''' ''' ''' ''' ''' ''' ''' ''' '''
3'    ''''TCCCATAATTAT TAC AAA GGT CGA TAC AGA GAT AGA CCA


   CTG TTT GCC AAC GCT GTGCT   3'
   ''' ''' ''' ''' ''' '
   GAC AAA CGG TTG CGA C        5'
```

and the ~0.6 kb BamHI-XbaI fragment of plasmid pCZ101, said plasmid pCZ103 being constructed by deletion of the ~900 bp BstEII restriction fragment of plasmid pCZ101 as defined in claim 5, and recircularization.

18. The process of Claim 9 for preparing plasmid pAT1.3 which comprises ligating the ~9.3 kb BamHI-EcoRI fragment of plasmid pCZ103 as defined in claim 17, a DNA linker having the nucleotide sequence

```
5'  CGACA ATG TTC CCA GCT ATG TCT CTA TCT GGT
    ''' ''' ''' ''' ''' ''' ''' ''' ''' '''
3'    TGT TAC AAG GGT CGA TAC AGA GAT AGA CCA


    CTG TTT GCC AAC GCT GTGCT   3'
    ''' ''' ''' ''' ''' '
    GAC AAA CGG TTG CGA C        5'
```

and the EcoRI-ClaI digest of plasmid pNM608 as defined in claim 10

19. The process of Claim 9 for preparing plasmid pASP1.3 which comprises ligating the ~9.3 kb BamHI-EcoRI fragment of plasmid PCZ103 as defined in claim 17, a DNA linker having the nucleotide sequence

```
5'  CGACC ATG GAT TTT CCG GCT ATG TCT CTG TCC GGC CTG
    ''' ''' ''' ''' ''' ''' ''' ''' ''' ''' ''' '''
3'    TGG TAC CTA AAA GGC CGA TAC AGC GAC AGG CCG GAC


    TTT GCC AAC GCT GTGCT    3'
    ''' ''' ''' ''' '
    AAA CGG TTG CGA C         5'
```

and the EcoRI-ClaI digest of plasmid pNM608 as defined in claim 10.

20. The process of Claim 9 for preparing plasmid pASP2.3 which comprises ligating the ~9.3 kb BamHI-EcoRI fragment of plasmid pCZ103 as defined in claim 17, a DNA linker having the nucleotide sequence

```
5'   CGATC ATG GAT TTT CCG GCT ATG TCT CTG TCC GGC CTG
          ||| ||| ||| ||| ||| ||| ||| ||| ||| ||| ||| |||
3'        TAG TAC CTA AAA GGC CGA TAC AGA GAC AGG CCG GAC

     TTT GCC AAC GCT GTGCT   3'
     ||| ||| ||| |||  |
     AAA CGG TTG CGA  C       5'
```

and the EcoRI-ClaI digest of plasmid pNM608 as defined in claim 10.

**21.** The process of Claim 9 for preparing plasmid pCZ154.3 which comprises ligating the ~9.3 kb BamHI-EcoRI fragment of plasmid pCZ103 as defined in claim 17, a DNA linker having the nucleotide sequence

```
5'   CGACC ATG GTT TTT CCG GCT ATG TCT CTG TCC GGC CTG
          ||| ||| ||| ||| ||| ||| ||| ||| ||| ||| ||| |||
3'        TGG TAC CAA AAA GGC CGA TAC AGA GAC AGG CCG GAC

     TTT GCC AAC GCT GTGCT   3'
     ||| ||| ||| |||  |
     AAA CGG TTG CGA  C       5'
```

and the EcoRI-ClaI digest of plasmid pNM608 as defined in claim 10

**22.** The process of Claim 9 for preparing plasmid pCZ155.3 which comprises ligating the ~9.3 kb BamHI-EcoRI fragment of plasmid pCZ103 as defined in claim 17, a DNA linker having the nucleotide sequence

```
5'   CGACC ATG GCT TTT CCG GCT ATG TCT CTG TCC GTC CTG
          ||| ||| ||| ||| ||| ||| ||| ||| ||| ||| ||| |||
3'        TGG TAC CGA AAA GGC CGA TAC AGA GAC AGG CAG GAC

     TTT GCC AAC GCT GTGCT   3'
     ||| ||| ||| |||  |
     AAA CGG TTG CGA  C       5'
```

and the EcoRI-ClaI digest of pNM608 as defined in claim 10.

**23.** The process of Claim 9 for preparing plasmid pCZ156.3 which comprised ligating the ~9.3 kb BamHI-EcoRI fragment of plasmid pCZ103 as defined in claim 17, a DNA linker having the nucleotide sequence

```
5'   CGATA ATG GAT TTT CCG GCT ATG TCT CTG TCC GGC CTG
          ||| ||| ||| ||| ||| ||| ||| ||| ||| ||| ||| |||
3'        TAT TAC CTA AAA GGC CGA TAC AGA GAC AGG CCG GAC

     TTT GCC AAC GCT GTGCT   3'
     ||| ||| ||| |||  |
     AAA CGG TTG CGA  C       5'
```

and the EcoRI-ClaI digest of pNM608 as defined in claim 10.

**24.** A process for preparing a plasmid suitable for use in the process of any of Claims 8 and 17 to 23 which comprises treating plasmid pCZ101 as defined in claim 5 with BstEII restriction enzyme, purifying the DNA thus obtained, and ligating the purified DNA to produce plasmid pCZ103.

**25.** A method for increasing milk production in cows comprising administering to said cows a bovine growth hormone derivative of coded for by the gene comprised in the vector produced by the process of any of Claims 1 to 23, exclusive of methionyl bovine growth hormone, in an amount effective for increasing said production.

26. The method of Claim 25 wherein the effective amount is from about 0.05 to about 100,000 micrograms per animal per day.

27. The method of Claim 26 wherein the effective amount is from about 5 to about 25,000 micrograms per animal per day.

28. The method of Claim 27 wherein the effective amount is from about 500 to about 5,000 micrograms per animal per day.

**Revendications**
**Revendications pour les Etats contractants suivants: BE, CH, DE, FR, GB, IT, LI, LU, NL, SE**

1. Vecteur d'expression d'ADN recombinant sélectionnable et à réplication autonome, qui comprend:
   a) un réplicon incontrollable;
   b) une séquence d'activation de transcription et de traduction qui se situe dans le cadre de lecture d'une séquence qui code pour un dérivé bioactif de l'hormone de croissance bovine, ladite séquence étant :

```
(1)   5'   ATG AAA GGG AAT TCT ATG GCC TTC
           ||| ||| ||| ||| ||| ||| ||| |||
      3'   TAC TTT CCC TTA AGA TAC CGG AAG

           CCA GCC ATG TCC TTG TCC GGC              3'
           ||| ||| ||| ||| ||| ||| |||-R-R¹
           GGT CGG TAC AGG AAC AGG CCG              5'

(2)   5'   ATG GAT GAT AAG TTT CCG GCT ATG
           ||| ||| ||| ||| ||| ||| ||| |||
      3'   TAC CTA CTA TTC AAA GGC CGA TAC

           TCT CTG TCC GGC            3'
           ||| ||| ||| |||-R-R¹
           AGA GAC AGG CCG            5' ,
```

```
(3)   5'   ATG TTT CCA GCC ATG GCT CTA
           ::: ::: ::: ::: ::: ::: :::
      3'   TAC AAA GGT CGG TAC CGA GAT


           TCT GGT         3'
           ::: :::-R-R¹
           AGA CCA         5',


(4)   5'   ATG TTC CCA GCT ATG TCT CTA
           ::: ::: ::: ::: ::: ::: :::
      3'   TAC AAG GGT CGA TAC AGA GAT


           TCT GGT         3'
           ::: :::-R-R¹
           AGA CCA         5',



(5)   5'   ATG GAT TTT CCG GCT ATG TCT
           ::: ::: ::: ::: ::: ::: :::
      3'   TAC CTA AAA GGC CGA TAC AGA


           CTG TCC GGC         3'
           ::: ::: :::-R-R¹
           GAC AGG CCG         5',
   ou


(6)   5'   ATG TTT CCA GCT ATG TCT CTA
           ::: ::: ::: ::: ::: ::: :::
      3'   TAC AAA GGT CGA TAC AGA GAT


           TCT GGT         3'
           ::: :::-R-R¹
           AGA CCA         5'
```

dans laquelle

A représente un groupe désoxyadényle,

G représente un groupe désoxyguanyle,

C représente un groupe désoxycytosyle,

T représente un groupe thymidyle,

R représente une séquence d'ADN qui code pour les acides aminés 9 (leucine) à 191 (phénylalanine) de l'hormone de croissance bovine, et

R¹ est une séquence d'ADN qui code pour un signal de terminaison de traduction, à savoir

```
5'   TAA   3'        5'   TAG   3'
     :::                   :::
3'   ATT   5',       3'   ATC   5',        ou


5'   TGA   3'
     :::
3'   ACT   5'
```

2. Vecteur selon la revendication 1, qui est un plasmide.

3. Vecteur selon la revendication 1 ou 2, dans lequel la séquence d'activation de transcription est la séquence d'activation de transcription du lactose de **E. coli**, du bactériophage λ $P_LO_L$, du bactériophage λ $P_RL_R$, de **tac**, de la lipoprotéine de **E. coli** ou du tryptophane de **E. coli**.

**4.** Vecteur selon la revendication 1, 2 ou 3, à savoir :

le plasmide pCZ1920 préparé par ligation du fragment **Bam**HI-**Xba**I d'environ 10,2 kb du plasmide pCZ101, d'un chaînon d'ADN muni de la séquence nucléotidique

```
5' CTAGAGGGTATTAATA ATG AAA GGG AAT TCT ATG GCC TTC CCA GCC
   ''''''''''''''''' ''' ''' ''' ''' ''' ''' ''' ''' ''' '''
3'      TCCCATAATTAT TAC TTT CCC TTA AGA TAC CGG AAG GGT CGG

ATG TCC TTG TCC GGC CTG TTT GCC AAC GCT GTGCT   3'
''' ''' ''' ''' ''' ''' ''' ''' ''' ''' '
TAC AGG AAC AGG CCG GAC AAA CGG TTG CGA C        5'
```

et du fragment **Bam**HI-**Xba**I d'environ 0,6 kb du plasmide pCZ101,

ledit plasmide pCZ101 étant construit par ligation du fragment **Bam**HI-**Xba**I d'environ 0,6 kb du plasmide pNM789B dérivé du plasmide pKEN111 (que l'on peut obtenir auprès de NRRL B-15011), tel que décrit dans les figures 1-8 et dans l'exemple 1, dans un plasmide pIM-I'-A3 mis à digérer de manière similaire (que l'on peut obtenir auprès de NRRL B-15733),

le plasmide pJR1 préparé par ligation du fragment **Bam**HI-**Xba**I d'environ 10,2 kb du plasmide pCZ101, d'un chaînon d'ADN muni de la séquence nucléotidique

```
5' CTAGAGGGTATTAATA ATG TTT CCA GCC ATG GCT CTA TCT GGT
   ''''''''''''''''' ''' ''' ''' ''' ''' ''' ''' ''' '''
3'      TCCCATAATTAT TAC AAA GGT CGG TAC CGA GAT AGA CCA

CTG TTT GCC AAC GCT GTGCT   3'
''' ''' ''' ''' ''' '
GAC AAA CGG TTG CGA C        5'
```

et du fragment **Bam**HI-**Xba**I d'environ 0,6 kb du plasmide pCZ101,

le plasmide pCZ112 préparé par ligation du fragment **Bam**HI-**Eco**RI d'environ 10,2 kb du plasmide pCZ101, d'un chaînon d'ADN muni de la séquence nucléotidique

```
5' CGACC ATG GAT GAT AAG TTT CCG GCT ATG TCT CTG
         ''' ''' ''' ''' ''' ''' ''' ''' ''' '''
3'       TGG TAC CTA CTA TTC AAA GGC CGA TAC AGA GAC

TCC GGC CTG TTT GCC AAC GCT GTGCT   3'
''' ''' ''' ''' ''' ''' ''' '
AGG CCG GAC AAA CGG TTG CGA C        5'
```

et du produit de digestion par **Eco**RI-**Cla**I du plasmide pNM608,

ledit plasmide pNM608 étant construit par ligation du fragment **Eco**RI-**Cla**I du plasmide pHI7△4△1 dans le plasmide pBR322 digéré par **Eco**RI-**Cla**I, le plasmide pHI7△4△1 étant construit comme décrit à l'exemple 5,

le plasmide pAT1 préparé par ligation du fragment **Bam**HI-**Eco**RI d'environ 10,2 kb du plasmide pCZ101, d'un chaînon d'ADN muni de la séquence nucléotidique

```
5'   CGACA ATG TTC CCA GCT ATG TCT CTA ·TCT GGT
     ::: ::: ::: ::: ::: ::: ::: ::: :::
3'     TGT TAC AAG GGT CGA TAC AGA GAT AGA CCA

       CTG TTT GCC AAC GCT GTGCT   3'
       ::: ::: ::: ::: :::  :
       GAC AAA CGG TTG CGA C       5'
```

et du produit de digestion par **Eco**RI-**Cla**I du plasmide pNM608,

le plasmide pASP1 préparé par ligation du fragment **Bam**HI-**Eco**RI d'environ 10,2 kb du plasmide pCZ101, d'un chaînon d'ADN muni de la séquence nucléotidique le plasmide pJR1.3 préparé par ligation du fragment **Bam**HI-**Xba**I d'environ 9,3 kb du plasmide pCZ103, d'un chaînon d'ADN muni de la séquence nucléotidique

```
5' CTAGAGGGTATTAATA ATG TTT CCA GCC ATG GCT CTA TCT GGT
   ::::::::::::::::: ::: ::: ::: ::: ::: ::: ::: ::: :::
3'     TCCCATAATTAT TAC AAA GGT CGG TAC CGA GAT AGA CCA

       CTG TTT GCC AAC GCT GTGCT   3'
       ::: ::: ::: ::: :::  :
       GAC AAA CGG TTG CGA C       5'
```

et du fragment **Bam**HI-**Xba**I d'environ 0,6 kb du plasmide pCZ101,

ledit plasmide pCZ103 étant construit par délétion du fragment de restriction **Bst**EII d'environ 900 pb du plasmide pCZ101 et par recyclisation,

le plasmide pAT1.3 préparé par ligation du fragment **Bam**HI-**Eco**RI d'environ 9,3 kb du plasmide pCZ103, d'un chaînon d'ADN muni de la séquence nucléotidique

```
5'   CGACA ATG TTC CCA GCT ATG TCT CTA TCT GGT
     ::: ::: ::: ::: ::: ::: ::: ::: :::
3'     TGT TAC AAG GGT CGA TAC AGA GAT AGA CCA

       CTG TTT GCC AAC GCT GTGCT   3'
       ::: ::: ::: ::: :::  :
       GAC AAA CGG TTG CGA C       5'
```

et du produit de digestion par **Eco**RI-**Cla**I du plasmide pNM608,

le plasmide pASP1.3 préparé par ligation du fragment **Bam**HI-**Eco**RI d'environ 9,3 kb du plasmide pCZ103, d'un chaînon d'ADN muni de la séquence nucléotidique

```
5'   CGACC ATG GAT TTT CCG GCT ATG TCT CTG TCC GGC CTG
     ::: ::: ::: ::: ::: ::: ::: ::: ::: ::: ::: :::
3'     TGG TAC CTA AAA GGC CGA TAC AGC GAC AGG CCG GAC

       TTT GCC AAC GCT GTGCT   3'
       ::: ::: ::: :::  :
       AAA CGG TTG CGA C       5'
```

et du produit de digestion par **Eco**RI-**Cla**I du plasmide pNM608,

le plasmide pASP2.3 préparé par ligation du fragment **Bam**HI-**Eco**RI d'environ 9,3 kb du plasmide pCZ103, d'un chaînon d'ADN muni de la séquence nucléotidique

```
5'   CGATC ATG GAT TTT CCG GCT ATG TCT CTG TCC GGC CTG
          ::: ::: ::: ::: ::: ::: ::: ::: ::: ::: ::: :::
3'         TAG TAC CTA AAA GGC CGA TAC AGA GAC AGG CCG GAC

     TTT GCC AAC GCT GTGCT   3'
     ::: ::: ::: ::: :
     AAA CGG TTG CGA C        5'
```

et du produit de digestion par **Eco**RI-**Cla**I du plasmide pNM608,

le plasmide pCZ154.3 préparé par ligation du fragment **Bam**HI-**Eco**RI d'environ 9,3 kb du plasmide pCZ103, d'un chaînon d'ADN muni de la séquence nucléotidique

```
5'   CGACC ATG GTT TTT CCG GCT ATG TCT CTG TCC GGC CTG
          ::: ::: ::: ::: ::: ::: ::: ::: ::: ::: ::: :::
3'         TGG TAC CAA AAA GGC CGA TAC AGA GAC AGG CCG GAC

     TTT GCC AAC GCT GTGCT   3'
     ::: ::: ::: ::: :
     AAA CGG TTG CGA C        5'
```

et du produit de digestion par **Eco**RI-**Cla**I du plasmide pNM608, le plasmide pCZ155.3 préparé par ligation du fragment **Bam**HI-**Eco**RI d'environ 9,3 kb du plasmide pCZ103, d'un chaînon d'ADN muni de la séquence nucléotidique

```
5'   CGACC ATG GCT TTT CCG GCT ATG TCT CTG TCC GTC CTG
          ::: ::: ::: ::: ::: ::: ::: ::: ::: ::: ::: :::
3'         TGG TAC CGA AAA GGC CGA TAC AGA GAC AGG CAG GAC

     TTT GCC AAC GCT GTGCT   3'
     ::: ::: ::: ::: :
     AAA CGG TTG CGA C        5'
```

et du produit de digestion par **Eco**RI-**Cla**I de pNM608, ou

le plasmide pCZ156.3 préparé par ligation du fragment **Bam**HI-**Eco**RI d'environ 9,3 kb du plasmide pCZ103, d'un chaînon d'ADN muni de la séquence nucléotidique

```
5'   CGATA ATG GAT TTT CCG GCT ATG TCT CTG TCC GGC CTG
          ::: ::: ::: ::: ::: ::: ::: ::: ::: ::: ::: :::
3'         TAT TAC CTA AAA GGC CGA TAC AGA GAC AGG CCG GAC

     TTT GCC AAC GCT GTGCT   3'
     ::: ::: ::: ::: :
     AAA CGG TTG CGA C        5'
```

et du produit de digestion par **Eco**RI-**Cla**I de pNM608.

**5.** Dérivé de l'hormone de croissance bovine codée par le vecteur selon l'une quelconque des revendications 1 à 4, à savoir MET-PHE-PRO-ALA-MET-ALA-R², MET-ASP-ASP-LYS-bGH ou MET-ASP-bGH, dans lequel :
MET représente la méthionine,

PHE représente la phénylalanine,
PRO représente la proline,
ALA représente l'alanine,
ASP représente l'acide aspartique,
LYS représente la lysine,
$R^2$ représente la séquence naturelle d'acides aminés de l'hormone de croissance bovine commençant par le sixième acide aminé (leucine) à partir de la terminaison N, et

bGH représente la séquence naturelle d'acides aminés de l'hormone de croissance bovine commençant par la (première) phénylalanine à terminaison N.

6. Cellule hôte comprenant un vecteur d'expression d'ADN recombinant selon l'une quelconque des revendications 1 à 4.

7. Cellule selon la revendication 6, qui est **E. coli.**

8. Cellule selon la revendication 7, qui est **E. coli** K12 RV308.

9. Procédé destiné à augmenter la production de lait chez des vaches, consistant à administrer auxdites vaches un dérivé de l'hormone de croissance bovine codé par le vecteur selon l'une quelconque des revendications 1 à 4, non compris l'hormone de croissance bovine méthionyle, en une quantité efficace pour augmenter ladite production.

10. Procédé selon la revendication 9, dans lequel la quantité administrée est de 0,05 à 100.000 microgrammes, de préférence de 5 à 25.000 microgrammes, de manière la plus préférée de 500 à 5.000 microgrammes par animal et par jour.

11. Plasmide pCZ103 construit par délétion du fragment de restriction **Bst**EII d'environ 900 pb du plasmide pCZ101 et par recyclisation, ledit plasmide pCZ101 étant construit par ligation du fragment **Xbal-Bam**HI d'environ 0,6 kb du plasmide pNM789B dérivé du plasmide pKEN111 (que l'on peut obtenir auprès de NRRL B-15011), comme décrit dans les figures 1 à 8 et à l'exemple 1, dans le pIM-I'-A3 mis à digérer de manière similaire (que l'on peut obtenir auprès de NRRL B-15733).

**Revendications pour l'Etat contractant suivant: AT**

1. Procédé pour préparer un vecteur d'expression d'ADN recombinant sélectionnable et à réplication autonome, qui comprend:

a) un réplicon incontrollable;

b) une séquence d'activation de transcription et de traduction qui se situe dans le cadre de lecture d'une séquence qui code pour un dérivé bioactif de l'hormone de croissance bovine, ladite séquence étant :

```
(1)  5'  ATG AAA GGG AAT TCT ATG GCC TTC
         ||| ||| ||| ||| ||| ||| ||| |||
     3'  TAC TTT CCC TTA AGA TAC CGG AAG

         CCA GCC ATG TCC TTG TCC GGC        3'
         ||| ||| ||| ||| ||| ||| |||-R-R¹
         GGT CGG TAC AGG AAC AGG CCG        5'.

(2)  5'  ATG GAT GAT AAG TTT CCG GCT ATG
         ||| ||| ||| ||| ||| ||| ||| |||
     3'  TAC CTA CTA TTC AAA GGC CGA TAC

         TCT CTG TCC GGC        3'
         ||| ||| ||| |||-R-R¹
         AGA GAC AGG CCG        5',
```

```
(3)  5'  ATG TTT CCA GCC ATG GCT CTA
         ::: ::: ::: ::: ::: ::: :::
     3'  TAC AAA GGT CGG TAC CGA GAT

         TCT GGT            3'
         ::: :::-R-R¹
         AGA CCA            5',

(4)  5'  ATG TTC CCA GCT ATG TCT CTA
         ::: ::: ::: ::: ::: ::: :::
     3'  TAC AAG GGT CGA TAC AGA GAT

         TCT GGT            3'
         ::: :::-R-R¹
         AGA CCA            5',

(5)  5'  ATG GAT TTT CCG GCT ATG TCT
         ::: ::: ::: ::: ::: ::: :::
     3'  TAC CTA AAA GGC CGA TAC AGA

         CTG TCC GGC        3'
  ou     ::: ::: :::-R-R¹
         GAC AGG CCG        5',

(6)  5'  ATG TTT CCA GCT ATG TCT CTA
         ::: ::: ::: ::: ::: ::: :::
     3'  TAC AAA GGT CGA TAC AGA GAT

         TCT GGT            3'
         ::: :::-R-R¹
         AGA CCA            5!
```

dans laquelle

A représente un groupe désoxyadényle,

G représente un groupe désoxyguanyle,

C représente un groupe désoxycytosyle,

T représente un groupe thymidyle,

R représente une séquence d'ADN qui code pour les acides aminés 9 (leucine) à 191 (phénylalanine) de l'hormone de croissance bovine, et

R¹ représente une séquence d'ADN qui code pour un signal de terminaison de traduction, à savoir

```
5'  TAA  3'       5'  TAG  3'
    :::               :::
3'  ATT  5',      3'  ATC  5',       ou


5'  TGA  3'
    :::
3'  ACT  5'
```

2. Procédé selon la revendication 1, dans lequel la séquence d'activation de transcription est la séquence d'activation de transcription du lactose de **E. coli**, du bactériophage λ $P_LO_L$, du bactériophage λ $P_RO_R$, de **tac**, de la lipoprotéine de **E. coli** ou du tryptophane de **E. coli**.

3. Procédé selon la revendication 1 ou 2, dans lequel le vecteur est un plasmide.

**4.** Procédé selon la revendication 3, dans lequel la séquence d'activation de traduction est la séquence de la lipoprotéine de **E. coli.**

**5.** Procédé selon la revendication 4, pour préparer le plasmide pCZ1920, qui consiste à effectuer la ligation du fragment **Bam**HI-**Xba**I d'environ 10,2 kb du plasmide pCZ101, d'un chaînon d'ADN muni de la séquence nucléotidique

```
5' CTAGAGGGTATTAATA ATG AAA GGG AAT TCT ATG GCC TTC CCA GCC
   ''''''''''''''''  ''' ''' ''' ''' ''' ''' ''' ''' ''' '''
3'    TCCCATAATTAT TAC TTT CCC TTA AGA TAC CGG AAG GGT CGG

ATG TCC TTG TCC GGC CTG TTT GCC AAC GCT GTGCT  3'
''' ''' ''' ''' ''' ''' ''' ''' ''' ''' '
TAC AGG AAC AGG CCG GAC AAA CGG TTG CGA C       5'
```

et du fragment **Bam**HI-**Xba**I d'environ 0,6 kb du plasmide pCZ101,

ledit plasmide pCZ101 étant construit par ligation du fragment **Xba**I-**Bam**HI d'environ 0,6 kb du plasmide pNM789B dérivé du plasmide pKEN111 (que l'on peut obtenir auprès de NRRL B-15011), tel que décrit dans les figures 1-8 et dans l'exemple 1, dans le plasmide pIM-I'-A3 mis à digérer de manière similaire (que l'on peut obtenir auprès de NRRL B-15733).

**6.** Procédé selon la revendication 4, pour préparer le plasmide pJR1, qui consiste à effectuer la ligation du fragment **Bam**HI-**Xba**I d'environ 10,2 kb du plasmide pCZ101 tel que défini à la revendication 5, d'un chaînon d'ADN muni de la séquence nucléotidique

```
5' CTAGAGGGTATTAATA ATG TTT CCA GCC ATG GCT CTA TCT GGT
   ''''''''''''''''  ''' ''' ''' ''' ''' ''' ''' ''' '''
3'    TCCCATAATTAT TAC AAA GGT CGG TAC CGA GAT AGA CCA

        CTG TTT GCC AAC GCT GTGCT  3'
        ''' ''' ''' ''' ''' '
        GAC AAA CGG TTG CGA C       5'
```

et du fragment **Bam**HI-**Xba**I d'environ 0,6 kb du plasmide pCZ101.

**7.** Procédé selon la revendication 4, pour préparer le plasmide pAT2, qui consiste à effectuer la ligation **Bam**HI-**Xba**I d'environ 10,2 kb du plasmide pCZ101 tel que défini à la revendication 5, d'un chaînon d'ADN muni de la séquence nucléotidique

```
5' CTAGAGGGTATTAATA ATG TTT CCA GCT ATG TCT CTA TCT GGT
   ''''''''''''''''  ''' ''' ''' ''' ''' ''' ''' ''' '''
3'    TCCCATAATTAT TAC AAA GGT CGA TAC AGA GAT AGA CCA

        CTG TTT GCC AAC GCT GTGCT  3'
        ''' ''' ''' ''' ''' '
        GAC AAA CGG TTG CGA C       5'
```

et du fragment **Bam**HI-**Xba**I d'environ 0,6 kb du plasmide pCZ101.

**8.** Procédé selon la revendication 4, pour préparer le plasmide pJR1.3 qui consiste à effectuer la ligation du fragment **Bam**HI-**Xba**I d'environ 9,3 kb du plasmide pCZ103, d'un chaînon d'ADN muni de la séquence nucléotidique

```
5' CTAGAGGGTATTAATA ATG TTT CCA GCC ATG GCT CTA. TCT GGT
   ''''''''''''''''' ''' ''' ''' ''' ''' ''' ''' ''' '''
3'    TCCCATAATTAT TAC AAA GGT CGG TAC CGA GAT AGA CCA

   CTG TTT GCC AAC GCT GTGCT  3'
   ''' ''' ''' ''' ''' '
   GAC AAA CGG TTG CGA C       5'
```

et du fragment **Bam**HI-**Xba**I d'environ 0,6 kb du plasmide pCZ101 tel que défini à la revendication 5, ledit pCZ103 étant construit par délétion du fragment de restriction **Bst**EII d'environ 900 pb du plasmide pCZ101 et par recyclisation.

9. Procédé selon la revendication 3, dans lequel la séquence d'activation de traduction est la séquence du tryptophane de **E. coli**.

10. Procédé selon la revendication 9, pour préparer le plasmide pCZ112, qui consiste à effectuer la ligation du fragment **Bam**HI-**Eco**RI d'environ 10,2 kb du plasmide pCZ101 tel que défini à la revendication 5, d'un chaînon d'ADN muni de la séquence nucléotidique

```
5'  CGACC ATG GAT GAT AAG TTT CCG GCT ATG TCT CTG
    ''' ''' ''' ''' ''' ''' ''' ''' ''' '''
3'      TGG TAC CTA CTA TTC AAA GGC CGA TAC AGA GAC
    '
    TCC GGC CTG TTT GCC AAC GCT GTGCT  3'
    ''' ''' ''' ''' ''' ''' ''' '
    AGG CCG GAC AAA CGG TTG CGA C        5'
```

et du produit de digestion par **Eco**RI-**Cla**I du plasmide pNM608, ledit plasmide pNM603 étant construit en effectuant la ligation du fragment **Eco**RI-**Cla**I du plasmide pHI7△4△1 dans le plasmide pBR322 mis à digérer par **Eco**RI-**Cla**I, le plasmide pHI7△4△1 étant construit comme décrit à l'exemple 5.

11. Procédé selon la revendication 9, pour préparer le plasmide pAT1, qui consiste à effectuer la ligation du fragment **Bam**HI-**Eco**RI d'environ 10,2 kb du plasmide pCZ101 tel que défini dans la revendication 5, d'un chaînon d'ADN muni de la séquence nucléotidique

```
5'  CGACA ATG TTC CCA GCT ATG TCT CTA TCT GGT
    ''' ''' ''' ''' ''' ''' ''' ''' '''
3'      TGT TAC AAG GGT CGA TAC AGA GAT AGA CCA

    CTG TTT GCC AAC GCT GTGCT  3'
    ''' ''' ''' ''' ''' '
    GAC AAA CGG TTG CGA C       5'
```

et du produit de digestion par **Eco**RI-**Cla**I du plasmide pNM608 tel que défini dans la revendication 10.

12. Procédé selon la revendication 9, pour préparer le plasmide pASP1, qui consiste à effectuer la ligation du fragment **Bam**HI-**Eco**RI d'environ 10,2 kb du plasmide pCZ101 tel que défini dans la revendication 5, d'un chaînon d'ADN muni de la séquence nucléotidique

```
5'  CGACC ATG GAT TTT CCG GCT ATG TCT CTG TCC GGC CTG
    ''' ''' ''' ''' ''' ''' ''' ''' ''' ''' ''' '''
3'      TGG TAC CTA AAA GGC CGA TAC AGC GAC AGG CCG GAC

    TTT GCC AAC GCT GTGCT  3'
    ''' ''' ''' ''' '
    AAA CGG TTG CGA C       5'
```

et du produit de digestion par **Eco**RI-**Cla**I du plasmide pNM608 tel que défini à la revendication 10.

13. Procédé selon la revendication 9, pour préparer le plasmide pASP2, qui consiste à effectuer la ligation du fragment **Bam**HI-**Eco**RI d'environ 10,2 kb du plasmide pCZ101 tel que défini dans la revendication 5, d'un chaînon d'ADN muni de la séquence nucléotidique

```
5'  CGATC ATG GAT TTT CCG GCT ATG TCT CTG TCC GGC CTG
          ::: ::: ::: ::: ::: ::: ::: ::: ::: ::: :::
3'        TAG TAC CTA AAA GGC CGA TAC AGA GAC AGG CCG GAC

    TTT GCC AAC GCT GTGCT  3'
    ::: ::: ::: ::: :
    AAA CGG TTG CGA C      5'
```

et du produit de digestion par **Eco**RI-**Cla**I du plasmide pNM608 tel que défini à la revendication 10.

14. Procédé selon la revendication 9, pour préparer le plasmide pCZ154, qui consiste à effectuer la ligation du fragment **Bam**HI-**Eco**RI d'environ 10,2 kb du plasmide pCZ101 tel que défini dans la revendication 5, d'un chaînon d'ADN muni de la séquence nucléotidique

```
5'    CGACC ATG GTT TTT CCG GCT ATG TCT CTG TCC GGC CTG
            ::: ::: ::: ::: ::: ::: ::: ::: ::: ::: :::
3'          TGG TAC CAA AAA GGC CGA TAC AGA GAC AGG CCG GAC

      TTT GCC AAC GCT GTGCT   3'
      ::: ::: ::: ::: :
      AAA CGG TTG CGA C       5'
```

et du produit de digestion par **Eco**RI-**Cla**I de pNM608 tel que défini à la revendication 10.

15. Procédé selon la revendication 9, pour préparer le plasmide pCZ155, qui consiste à effectuer la ligation du fragment **Bam**HI-**Eco**RI d'environ 10,2 kb du plasmide pCZ101 tel que défini dans la revendication 5, d'un chaînon d'ADN muni de la séquence nucléotidique

```
5'    CGACC ATG GCT TTT CCG GCT ATG TCT CTG TCC GTC CTG
            ::: ::: ::: ::: ::: ::: ::: ::: ::: ::: :::
3'          TGG TAC CGA AAA GGC CGA TAC AGA GAC AGG CAG GAC

      TTT GCC AAC GCT GTGCT    3'
      ::: ::: ::: ::: :
      AAA CGG TTG CGA C        5'
```

et du produit de digestion par **Eco**RI-**Cla**I de pNM608 tel que défini à la revendication 10.

16. Procédé selon la revendication 9, pour préparer le plasmide pCZ156, qui consiste à effectuer la ligation du fragment **Bam**HI-**Eco**RI d'environ 10,2 kb du plasmide pCZ101 tel que défini dans la revendication 5, d'un chaînon d'ADN muni de la séquence nucléotidique

```
5'    CGATA ATG GAT TTT CCG GCT ATG TCT CTG TCC GGC CTG
            ::: ::: ::: ::: ::: ::: ::: ::: ::: ::: :::
3'          TAT TAC CTA AAA GGC CGA TAC AGA GAC AGG CCG GAC

      TTT GCC AAC GCT GTGCT   3'
      ::: ::: ::: ::: :
      AAA CGG TTG CGA C       5'
```

et du produit de digestion par **Eco**RI-**Cla**I de pNM608 tel que défini à la revendication 10.

17. Procédé selon la revendication 9, pour préparer le plasmide pAT2.3, qui consiste à effectuer la ligation du fragment **Bam**HI-**Eco**RI d'environ 9,3 kb du plasmide pCZ103, d'un chaînon d'ADN muni de la séquence nucléotidique

```
5' CTAGAGGGTATTAATA ATG TTT CCA GCT ATG TCT CTA TCT GGT
   '''''''''''''''' ''' ''' ''' ''' ''' ''' ''' ''' '''
3'    'TCCCATAATTAT TAC AAA GGT CGA TAC AGA GAT AGA CCA

   CTG TTT GCC AAC GCT GTGCT  3'
   ''' ''' ''' ''' ''' '
   GAC AAA CGG TTG CGA C       5'
```

et du fragment **Bam**HI-**Xba**I d'environ 0,6 kb du plasmide pCZ101, ledit plasmide pCZ103 étant construit par délétion du fragment de restriction **Bst**EII d'environ 900 pb du plasmide pCZ101 tel que défini à la revendication 5, et par recyclisation.

18. Procédé selon la revendication 9, pour préparer le plasmide pAT1.3, qui consiste à effectuer la ligation du fragment **Bam**HI-**Eco**RI d'environ 9,3 kb du plasmide pCZ103 tel que défini dans la revendication 17, d'un chaînon d'ADN muni de la séquence nucléotidique

```
5' CGACA ATG TTC CCA GCT ATG TCT CTA TCT GGT
   ''' ''' ''' ''' ''' ''' ''' ''' ''' '''
3'   TGT TAC AAG GGT CGA TAC AGA GAT AGA CCA

   CTG TTT GCC AAC GCT GTGCT  3'
   ''' ''' ''' ''' ''' '
   GAC AAA CGG TTG CGA C        5'
```

et du produit de digestion par **Eco**RI-**Cla**I du plasmide pNM608 tel que défini à la revendication 10.

19. Procédé selon la revendication 9, pour préparer le plasmide pASP1.3, qui consiste à effectuer la ligation du fragment **Bam**HI-**Eco**RI d'environ 9,3 kb du plasmide pCZ103 tel que défini dans la revendication 17, d'un chaînon d'ADN muni de la séquence nucléotidique

```
5' CGACC ATG GAT TTT CCG GCT ATG TCT CTG TCC GGC CTG
   ''' ''' ''' ''' ''' ''' ''' ''' ''' ''' ''' '''
3'   TGG TAC CTA AAA GGC CGA TAC AGC GAC AGG CCG GAC

   TTT GCC AAC GCT GTGCT  3'
   ''' ''' ''' ''' '
   AAA CGG TTG CGA C       5'
```

et du produit de digestion par **Eco**RI-**Cla**I du plasmide pNM608 tel que défini à la revendication 10.

20. Procédé selon la revendication 9, pour préparer le plasmide pASP2.3, qui consiste à effectuer la ligation du fragment **Bam**HI-**Eco**RI d'environ 9,3 kb du plasmide pCZ103 tel que défini dans la revendication 17, d'un chaînon d'ADN muni de la séquence nucléotidique

```
5' CGATC ATG GAT TTT CCG GCT ATG TCT CTG TCC GGC CTG.
   ''' ''' ''' ''' ''' ''' ''' ''' ''' ''' ''' '''
3'   TAG TAC CTA AAA GGC CGA TAC AGA GAC AGG CCG GAC

   TTT GCC AAC GCT GTGCT  3'
   ''' ''' ''' ''' '
   AAA CGG TTG CGA C       5'
```

et du produit de digestion par **EcoRI-ClaI** du plasmide pNM608 tel que défini à la revendication 10.

**21.** Procédé selon la revendication 9, pour préparer le plasmide pCZ154.3, qui consiste à effectuer la ligation du fragment **Bam**HI-**Eco**RI d'environ 9,3 kb du plasmide pCZ103 tel que défini dans la revendication 17, d'un chaînon d'ADN muni de la séquence nucléotidique

```
5'    CGACC ATG GTT TTT CCG GCT ATG TCT CTG TCC GGC CTG
            ||| ||| ||| ||| ||| ||| ||| ||| ||| ||| ||| |||
3'          TGG TAC CAA AAA GGC CGA TAC AGA GAC AGG CCG GAC

      TTT GCC AAC GCT GTGCT    3'
      ||| ||| ||| ||| |
      AAA CGG TTG CGA C        5'
```

et du produit de digestion par **EcoRI-ClaI** du plasmide pNM608 tel que défini à la revendication 10.

**22.** Procédé selon la revendication 9, pour préparer le plasmide pCZ155.3, qui consiste à effectuer la ligation du fragment **Bam**HI-**Eco**RI d'environ 9,3 kb du plasmide pCZ103 tel que défini dans la revendication 17, d'un chaînon d'ADN muni de la séquence nucléotidique

```
5'    CGACC ATG GCT TTT CCG GCT ATG TCT CTG TCC GTC CTG
            ||| ||| ||| ||| ||| ||| ||| ||| ||| ||| ||| |||
3'          TGG TAC CGA AAA GGC CGA TAC AGA GAC AGG CAG GAC

      TTT GCC AAC GCT GTGCT    3'
      ||| ||| ||| ||| |
      AAA CGG TTG CGA C        5'
```

et du produit de digestion par **EcoRI-ClaI** de pNM608 tel que défini à la revendication 10.

**23.** Procédé selon la revendication 9, pour préparer le plasmide pCZ156.3, qui consiste à effectuer la ligation du fragment **Bam**HI-**Eco**RI d'environ 9,3 kb du plasmide pCZ103 tel que défini dans la revendication 17, d'un chaînon d'ADN muni de la séquence nucléotidique

```
5'    CGATA ATG GAT TTT CCG GCT ATG TCT CTG TCC GGC CTG
            ||| ||| ||| ||| ||| ||| ||| ||| ||| ||| ||| |||
3'          TAT TAC CTA AAA GGC CGA TAC AGA GAC AGG CCG GAC

      TTT GCC AAC GCT GTGCT    3'
      ||| ||| ||| ||| |
      AAA CGG TTG CGA C        5'
```

et du produit de digestion par **EcoRI-ClaI** de pNM608 tel que défini à la revendication 10.

**24.** Procédé pour préparer un plasmide approprié pour être utilisé dans le procédé selon l'une quelconque des revendications 8 et 17 à 23, qui consiste à traiter le plasmide pCZ101 tel que défini à la revendication 5, avec l'enzyme de restriction **Bst**EII, à purifier l'ADN ainsi obtenu et à effectuer la ligation de l'ADN purifié pour obtenir le plasmide pCZ103.

**25.** Procédé pour augmenter la production de lait chez des vaches, consistant à administrer auxdites vaches un dérivé de l'hormone de croissance bovine codé par le gène compris dans le vecteur obtenu par le procédé selon l'une quelconque des revendications 1 à 23, non compris l'hormone de croissance bovine de méthionyle, en une quantité efficace pour augmenter ladite production.

**26.** Procédé selon la revendication 25, dans lequel la quantité efficace est d'environ 0,05 à environ 100.000 microgrammes par animal et par jour.

**27.** Procédé selon la revendication 26, dans lequel la quantité efficace est d'environ 5 à environ 25.000 microgrammes par animal et par jour.

**28.** Procédé selon la revendication 27, dans lequel la quantité efficace est d'environ 500 à environ 5.000 microgrammes par animal et par jour.

**Patentansprüche**
**Patentansprüche für folgende Vertragsstaaten: BE, CH, DE, FR, GB, IT, LI, LU, NL, SE**

**1.** Selektierbarer und autonom replizierender rekombinanter DNA-Expressionsvektor, umfassend
a) ein unkontrolliertes Replikon und
b) eine die Transkription und Translation aktivierende Sequenz, die im Leseraster einer Sequenz ist, die ein bioaktives Rinderwachstumshormonderivat kodiert, wobei diese Sequenz

```
(1)  5'  ATG AAA GGG AAT TCT ATG GCC TTC
         ||| ||| ||| ||| ||| ||| ||| |||
     3'  TAC TTT CCC TTA AGA TAC CGG AAG

         CCA GCC ATG TCC TTG TCC GGC          3'
         ||| ||| ||| ||| ||| ||| |||-R-R¹
         GGT CGG TAC AGG AAC AGG CCG          5',

(2)  5'  ATG GAT GAT AAG TTT CCG GCT ATG
         ||| ||| ||| ||| ||| ||| ||| |||
     3'  TAC CTA CTA TTC AAA GGC CGA TAC

         TCT CTG TCC GGC          3'
         ||| ||| ||| |||-R-R¹
         AGA GAC AGG CCG          5',

(3)  5'  ATG TTT CCA GCC ATG GCT CTA
         ||| ||| ||| ||| ||| ||| |||
     3'  TAC AAA GGT CGG TAC CGA GAT

         TCT GGT          3'
         ||| |||-R-R¹
         AGA CCA          5',

(4)  5'  ATG TTC CCA GCT ATG TCT CTA
         ||| ||| ||| ||| ||| ||| |||
     3'  TAC AAG GGT CGA TAC AGA GAT

         TCT GGT          3'
         ||| |||-R-R¹
         AGA CCA          5',

(5)  5'  ATG GAT TTT CCG GCT ATG TCT
         ||| ||| ||| ||| ||| ||| |||
     3'  TAC CTA AAA GGC CGA TAC AGA

         CTG TCC GGC          3'
         ||| ||| |||-R-R¹
         GAC AGG CCG          5',

oder

(6)  5'  ATG TTT CCA GCT ATG TCT CTA
         ||| ||| ||| ||| ||| ||| |||
     3'  TAC AAA GGT CGA TAC AGA GAT

         TCT GGT          3'
         ||| |||-R-R¹
         AGA CCA          5'
```

ist, worin A Deoxyadenyl ist, G Deoxyguanyl ist, C, Deoxycytosyl ist, T Thymidyl ist, R eine DNA-Sequenz ist, die die Aminosäuren 9 (Leucin) bis 191 (Phenylalanin) von Rinderwachstumshormon kodiert, und $R^1$ eine DNA-Sequenz ist, die ein Translationsstopsignal kodiert, das

$$5'\ \text{TAA}\ 3'\quad 5'\ \text{TAG}\ 3'$$
$$3'\ \text{ATT}\ 5',\quad 3'\ \text{ATC}\ 5',$$

oder

$$5'\ \text{TGA}\ 3'$$
$$3'\ \text{ACT}\ 5'$$

ist.

2. Vektor nach Anspruch 1, der ein Plasmid ist.

3. Vektor nach Anspruch 1 oder 2, worin die transkriptionsaktivierende Sequenz die E. coli Lactose-, Bakteriophage $\lambda P_L O_L$-Bakteriophage $\lambda P_R L_R$-, tac, E. coli Lipoprotein- oder E. coli Tryptophan-aktivierende Sequenz ist.

4. Vektor nach Anspruch 1, 2 oder 3, der Plasmid pCZ1920 ist, hergestellt durch Ligieren des ~10,2 kb BamHI-XbaI-Fragments von Plasmid pCZ101, einem DNA-Linker mit der Nukleotidsequenz

```
5' CTAGAGGGTATTAATA ATG AAA GGG AAT TCT ATG GCC TTC CCA GCC
3'     TCCCATAATTAT TAC TTT CCC TTA AGA TAC CGG AAG GGT CGG

ATG TCC TTG TCC GGC CTG TTT GCC AAC GCT GTGCT   3'
TAC AGG AAC AGG CCG GAC AAA CGG TTG CGA C       5'
```

und dem ~0,6 kb BamHI-XbaI-Fragment von Plasmid pCZ101,

wobei das Plasmid pCZ101 konstruiert wird, indem das ~0,6 kb XbaI-BamHI-Fragment von Plasmid pNM789B, das abgeleitet ist von Plasmid pKEN111 (erhältlich von NRRL B-15011), wie in den Figuren 1-8 und Beispiel 1 beschrieben, in das ebenso verdaute Plasmid pIM-I'-A3 (erhältlich aus NRRL B-15733) ligiert wird,

Plasmid pJR1 ist, hergestellt durch Ligieren des ~10,2 kb BamHI-XbaI-Fragments von Plasmid pCZ101, einem DNA-Linker mit der Nukleotidsequenz

```
5' CTAGAGGGTATTAATA ATG TTT CCA GCC ATG GCT CTA TCT GGT
3'     TCCCATAATTAT TAC AAA GGT CGG TAC CGA GAT AGA CCA

CTG TTT GCC AAC GCT GTGCT   3'
GAC AAA CGG TTG CGA C       5'
```

und dem ~0,6 kb BamHI-XbaI-Fragment von Plasmid pCZ101,

Plasmid pCZ112 ist, das hergestellt wird durch Ligieren des ~10,2 kb BamHI-EcoRI-Fragments von Plasmid pCZ101, einem DNA-Linker mit der Nukleotidsequenz

```
5'  CGACC ATG GAT GAT AAG TTT CCG GCT ATG TCT CTG
         ::: ::: ::: ::: ::: ::: ::: ::: ::: :::
3'       TGG TAC CTA CTA TTC AAA GGC CGA TAC AGA GAC

    TCC GGC CTG TTT GCC AAC GCT GTGCT    3'
    ::: ::: ::: ::: ::: ::: :::  :
    AGG CCG GAC AAA CGG TTG CGA C        5'
```

und dem EcoRI-ClaI-Verdau von Plasmid pNM608,

wobei Plasmid pNM608 konstruiert wird, indem das EcoRI-ClaI-Fragment von Plasmid pHI7Δ4Δ1 in das mit EcoRI-ClaI verdaute Plasmid pBR322 ligiert wird, wobei Plasmid pHI7Δ4Δ1, wie in Beispiel 5 beschrieben, konstruiert wird,

Plasmid pAT1 ist, hergestellt durch Ligieren des ~10,2 kb BamHI-EcoRI-Fragments von Plasmid pCZ101, einem DNA-Linker mit der Nukleotidsequenz

```
5'  CGACA ATG TTC CCA GCT ATG TCT CTA TCT GGT
         ::: ::: ::: ::: ::: ::: ::: ::: ::: :::
3'       TGT TAC AAG GGT CGA TAC AGA GAT AGA CCA

    CTG TTT GCC AAC GCT GTGCT    3'
    ::: ::: ::: ::: :::  :
    GAC AAA CGG TTG CGA C        5'
```

und dem EcoRI-ClaI-Verdau von Plasmid pNM608,

Plasmid pASP1 ist, hergestellt durch Ligieren des ~10,2 kb BamHI-EcoRI-Fragments von Plasmid pCZ101, einem DNA-Linker mit der Nukleotidsequenz

```
5'  CGACC ATG GAT TTT CCG GCT ATG TCT CTG TCC GGC CTG
         ::: ::: ::: ::: ::: ::: ::: ::: ::: ::: :::
3'       TGG TAC CTA AAA GGC CGA TAC AGC GAC AGG CCG GAC

    TTT GCC AAC GCT GTGCT    3'
    ::: ::: ::: ::: :
    AAA CGG TTG CGA C        5'
```

und dem EcoRI-ClaI-Verdau von Plasmid pNM608,

Plasmid pAT2 ist, hergestellt durch Ligieren des ~10,2 kb BamHI-XbaI-Fragments von Plasmid pCZ101, einem DNA-Linker mit der Nukleotidsequenz

```
5' CTAGAGGGTATTAATA ATG TTT CCA GCT ATG TCT CTA TCT GGT
   :::::::::::::::: ::: ::: ::: ::: ::: ::: ::: ::: :::
3'        TCCCATAATTAT TAC AAA GGT CGA TAC AGA GAT AGA CCA

    CTG TTT GCC AAC GCT GTGCT    3'
    ::: ::: ::: ::: ::: :
    GAC AAA CGG TTG CGA C        5'
```

und dem ~0,6 kb BamHI-XbaI-Fragment von Plasmid pCZ101,

Plasmid pASP2 ist, hergestellt durch Ligieren des ~10,2 kb BamHI-EcoRI-Fragments von Plasmid pCZ101, einem DNA-Linker mit der Nukleotidsequenz

```
5'   CGATC ATG GAT TTT CCG GCT ATG TCT CTG TCC GGC CTG
           ::: ::: ::: ::: ::: ::: ::: ::: ::: ::: :::
3'         TAG TAC CTA AAA GGC CGA TAC AGA GAC AGG CCG GAC

     TTT GCC AAC GCT GTGCT   3'
     ::: ::: ::: ::: :
     AAA CGG TTG CGA C        5'
```

und dem EcoRI-ClaI-Verdau von Plasmid pNM608,

Plasmid pJR1.3 ist, hergestellt durch Ligieren des ~9,3 kb BamHI-XbaI-Fragments von Plasmid pCZ103, einem DNA-Linker mit der Nukleotidsequenz

```
5' CTAGAGGGTATTAATA ATG TTT CCA GCC ATG GCT CTA TCT GGT
   :::::::::::::::: ::: ::: ::: ::: ::: ::: ::: ::: :::
3'      TCCCATAATTAT TAC AAA GGT CGG TAC CGA GAT AGA CCA

     CTG TTT GCC AAC GCT GTGCT   3'
     ::: ::: ::: ::: ::: :
     GAC AAA CGG TTG CGA C        5'
```

und dem ~0,6 kb BamHI-XbaI-Fragment von Plasmid pCZ101,

wobei das Plasmid pCZ103 konstruiert wird durch Deletion des ~900 bp BstEII-Restriktionsfragments von Plasmid pCZ101 und Rezirkularisierung,

Plasmid pAT1.3 ist, hergestellt durch Ligieren des ~9,3 kb BamHI-EcoRI-Fragments von Plasmid pCZ103, einem DNA-Linker mit der Nukleotidsequenz

```
5'   CGACA ATG TTC CCA GCT ATG TCT CTA TCT GGT
           ::: ::: ::: ::: ::: ::: ::: ::: :::
3'         TGT TAC AAG GGT CGA TAC AGA GAT AGA CCA

     CTG TTT GCC AAC GCT GTGCT   3'
     ::: ::: ::: ::: ::: :
     GAC AAA CGG TTG CGA C        5'
```

und dem EcoRI-ClaI-Verdau von Plasmid pNM608,

Plasmid pASP1.3 ist, hergestellt durch Ligieren des ~9,3 kb BamHI-EcoRI-Fragments von pCZ103, einem DNA-Linker mit der Nukleotidsequenz

```
5'   CGACC ATG GAT TTT CCG GCT ATG TCT CTG TCC GGC CTG
           ::: ::: ::: ::: ::: ::: ::: ::: ::: ::: :::
3'         TGG TAC CTA AAA GGC CGA TAC AGC GAC AGG CCG GAC

     TTT GCC AAC GCT GTGCT   3'
     ::: ::: ::: ::: :
     AAA CGG TTG CGA C        5'
```

und dem EcoRI-ClaI-Verdau von Plasmid pNM608,

Plasmid pASP2.3 ist, hergestellt durch Ligieren des ~9,3 kb BamHI-EcoRI-Fragments von Plasmid pCZ103, einem DNA-Linker mit der Nukleotidsequenz

```
5'   CGATC ATG GAT TTT CCG GCT ATG TCT CTG TCC GGC CTG
     ||| ||| ||| ||| ||| ||| ||| ||| ||| ||| ||| |||
3'     TAG TAC CTA AAA GGC CGA TAC AGA GAC AGG CCG GAC

     TTT GCC AAC GCT GTGCT   3'
     ||| ||| ||| ||| |
     AAA CGG TTG CGA C        5'
```

und dem EcoRI-ClaI-Verdau von Plasmid pNM608,

Plasmid pCZ154.3 ist, hergestellt durch Ligieren des ~9,3 kb BamHI-EcoRI-Fragments von Plasmid pCZ103, einem DNA-Linker mit der Nukleotidsequenz

```
5'   CGACC ATG GTT TTT CCG GCT ATG TCT CTG TCC GGC CTG
     ||| ||| ||| ||| ||| ||| ||| ||| ||| ||| ||| |||
3'     TGG TAC CAA AAA GGC CGA TAC AGA GAC AGG CCG GAC

     TTT GCC AAC GCT GTGCT   3'
     ||| ||| ||| ||| |
     AAA CGG TTG CGA C        5'
```

und dem EcoRI-ClaI-Verdau von Plasmid pNM608,

Plasmid pCZ155.3 ist, hergestellt durch Ligieren des ~9,3 kb BamHI-EcoRI-Fragments von Plasmid pCZ103, einem DNA-Linker mit der Nukleotidsequenz

```
5'   CGACC ATG GCT TTT CCG GCT ATG TCT CTG TCC GTC CTG
     ||| ||| ||| ||| ||| ||| ||| ||| ||| ||| ||| |||
3'     TGG TAC CGA AAA GGC CGA TAC AGA GAC AGG CAG GAC

     TTT GCC AAC GCT GTGCT   3'
     ||| ||| ||| ||| |
     AAA CGG TTG CGA C        5'
```

und dem EcoRI-ClaI-Verdau von pNM608, oder

Plasmid pCZ156.3 ist, hergestellt durch Ligieren des ~9,3 kb BamHI-EcoRI-Fragments von Plasmid pCZ103, einem DNA-Linker der Nukleotidsequenz

```
5'   CGATA ATG GAT TTT CCG GCT ATG TCT CTG TCC GGC CTG
     ||| ||| ||| ||| ||| ||| ||| ||| ||| ||| ||| |||
3'     TAT TAC CTA AAA GGC CGA TAC AGA GAC AGG CCG GAC

     TTT GCC AAC GCT GTGCT   3'
     ||| ||| ||| ||| |
     AAA CGG TTG CGA C        5'
```

und dem EcoRI-ClaI-Verdau von pNM608.

**5.** Rinderwachstumshormonderivat kodiert durch den Vektor nach einem der Ansprüche 1 bis 4, das MET-PHE-PRO-ALA-MET-ALA-R[2],
MET-ASP-ASP-LYS-bGH oder MET-ASP-bGH ist, worin
MET Methionin ist,
PHE Phenylalanin ist,

PRO Prolin ist,

ALA Alanin ist,

ASP Asparaginsäure ist,

LYS Lysin ist,

R$^2$ die natürliche Aminosäuresequenz von Rinderwachstumshormon ist, die mit der sechsten Aminosäure (Leucin) vom N-Ende beginnt, und

bGH die natürliche Aminosäuresequenz des Rinderwachstumshormons ist, beginnend mit dem N-terminalen (ersten) Phenylalanin.

6. Wirtszelle, enthaltend einen rekombinanten DNA-Expressionsvektor nach einem der Ansprüche 1 bis 4.

7. Zelle nach Anspruch 6, die E. coli ist.

8. Zelle nach Anspruch 7, die E. coli K12 RV308 ist.

9. Verfahren zur Erhöhung der Milchproduktion bei Kühen, umfassend, daß man den Kühen ein Rinderwachstumshormonderivat, das durch den Vektor nach einem der Ansprüche 1 bis 4 kodiert wird, mit Ausnahme des Methionylrinderwachstumshormons, in einer Menge, die wirksam ist zur Erhöhung der Produktion, verabreicht.

10. Verfahren nach Anspruch 9, worin die verabreichte Menge 0,05 bis 100000 $\mu$g, vorzugsweise 5 bis 25000 $\mu$g, am meisten bevorzugt 500 bis 5000 $\mu$g, pro Tier pro Tag ist.

11. Plasmid pCZ103, konstruiert durch Deletion des ~900 bp BstEII-Restriktionsfragments von Plasmid pCZ101 und Rezirkularisierung, wobei Plasmid pCZ101 konstruiert wird durch Ligieren des ~0,6 kb XbaI-BamHI-Fragmentes von Plasmid pNM789B, das abgeleitet ist von Plasmid pKEN111 (erhältlich von NRRL B-15011), wie in den Figuren 1-8 und Beispiel 1 beschrieben, in das auf gleiche Weise verdaute pIM-I'-A3 (erhältlich aus NRRL B-15733).

**Patentansprüche für folgenden Vertragsstaat: AT**

1. Verfahren zur Herstellung eines selektierbaren autonom replizierenden rekombinanten DNA-Expressionsvektors, umfassend, daß man

 a) ein unkontrolliertes Replikon und

 b) eine die Transkription und die Translation aktivierende Sequenz, die im Leseraster einer Sequenz, die für ein bioaktives Rinderwachstumshormonderivat kodiert, ist, ligiert, wobei diese Sequenz

(1)  5'  ATG AAA GGG AAT TCT ATG GCC TTC
     3'  TAC TTT CCC TTA AGA TAC CGG AAG

         CCA GCC ATG TCC TTG TCC GGC             3'
         GGT CGG TAC AGG AAC AGG CCG-R-R$^1$     5',

(2)  5'  ATG GAT GAT AAG TTT CCG GCT ATG
     3'  TAC CTA CTA TTC AAA GGC CGA TAC

         TCT CTG TCC GGC             3'
         AGA GAC AGG CCG-R-R$^1$     5',

(3)  5'  ATG TTT CCA GCC ATG GCT CTA
     3'  TAC AAA GGT CGG TAC CGA GAT

         TCT GGT             3'
         AGA CCA-R-R$^1$     5',

(4)  5'  ATG TTC CCA GCT ATG TCT CTA
     3'  TAC AAG GGT CGA TAC AGA GAT

         TCT GGT             3'
         AGA CCA-R-R$^1$     5',

(5)  5'  ATG GAT TTT CCG GCT ATG TCT
     3'  TAC CTA AAA GGC CGA TAC AGA

         CTG TCC GGC             3'
         GAC AGG CCG-R-R$^1$     5',

oder

(6)  5'  ATG TTT CCA GCT ATG TCT CTA
     3'  TAC AAA GGT CGA TAC AGA GAT

         TCT GGT             3'
         AGA CCA-R-R$^1$     5'

ist, worin A Deoxyadenyl ist, G Deoxyguanyl ist, C Deoxycytosyl ist, T Thymidyl ist, R eine DNA-Sequenz, die die Aminosäuren 9 (Leucin) bis 191 (Phenylalanin) des Rinderwachstumshormons kodiert, ist, und R$^1$ eine DNA-Sequenz ist, die ein Translationsstopsignal kodiert, das

$$5' \text{ TAA } 3' \qquad 5' \text{ TAG } 3'$$
$$3' \text{ ATT } 5', \qquad 3' \text{ ATC } 5',$$

oder

$$5' \text{ TGA } 3'$$
$$3' \text{ ACT } 5'$$

ist.

2. Verfahren nach Anspruch 1, worin die transkriptionsaktivierende Sequenz die E. coli Lactose-, Bakteriophage λ $P_L O_L$-, Bakteriophage λ $P_R O_R$- tac-, E. coli Lipoprotein- oder E. coli Tryptophan-transkriptionsaktivierende Sequenz ist.

3. Verfahren nach Anspruch 1 oder 2, worin der Vektor ein Plasmid ist.

4. Verfahren nach Anspruch 3, worin die translationsaktivierende Sequenz die E. coli Lipoproteinsequenz ist.

5. Verfahren nach Anspruch 4 zur Herstellung von Plasmid pCZ1920, umfassend, daß man das ~10,2 kb BamHI-XbaI-Fragment von Plasmid pCZ101, einen DNA-Linker mit der Nukleotidsequenz

```
5' CTAGAGGGTATTAATA ATG AAA GGG AAT TCT ATG GCC TTC CCA GCC
3'     TCCCATAATTAT TAC TTT CCC TTA AGA TAC CGG AAG GGT CGG

ATG TCC TTG TCC GGC CTG TTT GCC AAC GCT GTGCT    3'
TAC AGG AAC AGG CCG GAC AAA CGG TTG CGA C         5'
```

und das ~0,6 kb BamHI-Fragment von Plasmid pCZ101 ligiert, wobei das Plasmid pCZ101 konstruiert wird, indem man das ~0,6 kb XbaI-BamHI-Fragment von Plasmid pNM789B, das abgeleitet ist von Plasmid pKEN111 (erhältlich von NRRL B-15011), wie in den Figuren 1 bis 8 und Beispiel 1 beschrieben, mit dem auf gleiche Weise verdauten Plasmid pIM-I'-A3 (erhältlich aus NRRL B-15733) ligiert.

6. Verfahren nach Anspruch 4 zur Herstellung von Plasmid pJR1, umfassend, daß man das ~10,2 kb BamHI-XbaI-Fragment von Plasmid pCZ101, wie in Anspruch 5 definiert, einen DNA-Linker mit der Nukleotidsequenz

```
5' CTAGAGGGTATTAATA ATG TTT CCA GCC ATG GCT CTA TCT GGT
3'     TCCCATAATTAT TAC AAA GGT CGG TAC CGA GAT AGA CCA

CTG TTT GCC AAC GCT GTGCT    3'
GAC AAA CGG TTG CGA C         5'
```

und das ~0,6 kb BamHI-XbaI-Fragment von Plasmid pCZ101 ligiert.

7. Verfahren nach Anspruch 4 zur Herstellung von Plasmid pAT2, umfassend, daß man das ~10,2 kb BamHI-XbaI-Fragment von Plasmid pCZ101, wie in Anspruch 5 definiert, einen DNA-Linker mit der Nukleotidsequenz

```
5' CTAGAGGGTATTAATA ATG TTT CCA GCT ATG TCT CTA TCT GGT
  ''''''''''''''''' ''' ''' ''' ''' ''' ''' ''' ''' '''
3'    TCCCATAATTAT TAC AAA GGT CGA TAC AGA GAT AGA CCA

     CTG TTT GCC AAC GCT GTGCT  3'
     ''' ''' ''' ''' '''  '
     GAC AAA CGG TTG CGA C       5'
```

und das ~0,6 kb BamHI-XbaI-Fragment von Plasmid pCZ101 ligiert.

8. Verfahren nach Anspruch 4 zur Herstellung von Plasmid pJR1.3, umfassend, daß man das ~9,3 kb BamHI-XbaI-Fragment von Plasmid pCZ103, einen DNA-Linker mit der Nukleotidsequenz

```
5' CTAGAGGGTATTAATA ATG TTT CCA GCC ATG GCT CTA TCT GGT
  ''''''''''''''''' ''' ''' ''' ''' ''' ''' ''' ''' '''
3'    TCCCATAATTAT TAC AAA GGT CGG TAC CGA GAT AGA CCA

     CTG TTT GCC AAC GCT GTGCT  3'
     ''' ''' ''' ''' '''  '
     GAC AAA CGG TTG CGA C       5'
```

und das ~0,6 kb BamHI-XbaI-Fragment von Plasmid pCZ101, wie in Anspruch 5 definiert, ligiert, wobei pCZ103 konstruiert wird durch Deletion des ~900 bp BstEII-Restriktionsfragments von Plasmid pCZ101 und Rezirkularisierung.

9. Verfahren nach Anspruch 3, worin die translationsaktivierende Sequenz die E. coli Tryptophansequenz ist.

10. Verfahren nach Anspruch 9 zur Herstellung von Plasmid pCZ112, umfassend, daß man das ~10,2 kb BamHI-EcoRI-Fragment von Plasmid pCZ101, wie in Anspruch 5 definiert, einen DNA-Linker mit der Nukleotidsequenz

```
5'  CGACC ATG GAT GAT AAG TTT CCG GCT ATG TCT CTG
    ''' ''' ''' ''' ''' ''' ''' ''' ''' ''' '''
3'    TGG TAC CTA CTA TTC AAA GGC CGA TAC AGA GAC
       '
     TCC GGC CTG TTT GCC AAC GCT GTGCT  3'
     ''' ''' ''' ''' ''' ''' '''  '
     AGG CCG GAC AAA CGG TTG CGA C       5'
```

und den EcoRI-ClaI-Verdau von Plasmid pNM608 ligiert, wobei Plasmid pNM608 konstruiert wird, indem das EcoRI-ClaI-Fragment von Plasmid pHI7△4△1 in das mit EcoRI-ClaI verdaute Plasmid pBR322 ligiert wird, wobei Plasmid pHI7△4△1 wie in Beispiel 5 beschrieben konstruiert wird.

11. Verfahren nach Anspruch 9 zur Herstellung von Plasmid pAT1, umfassend, daß man das ~10,2 kb BamHI-EcoRI-Fragment von Plasmid pCZ101, wie in Anspruch 5 definiert, einen DNA-Linker mit der Nukleotidsequenz

```
5'  CGACA ATG TTC CCA GCT ATG TCT CTA TCT GGT
    ''' ''' ''' ''' ''' ''' ''' ''' ''' '''
3'    TGT TAC AAG GGT CGA TAC AGA GAT AGA CCA

     CTG TTT GCC AAC GCT GTGCT  3'
     ''' ''' ''' ''' '''  '
     GAC AAA CGG TTG CGA C       5'
```

und den EcoRI-ClaI-Verdau von Plasmid pNM608, wie in Anspruch 10 definiert, ligiert.

**12.** Verfahren nach Anspruch 9 zur Herstellung von Plasmid pASP1, umfassend, daß man das ~10,2 kb BamHI-EcoRI-Fragment von Plasmid pCZ101, wie in Anspruch 5 definiert, einen DNA-Linker mit der Nukleotidsequenz

```
5'  CGACC ATG GAT TTT CCG GCT ATG TCT CTG TCC GGC CTG
    ||| ||| ||| ||| ||| ||| ||| ||| ||| ||| |||
3'    TGG TAC CTA AAA GGC CGA TAC AGC GAC AGG CCG GAC

    TTT GCC AAC GCT GTGCT  3'
    ||| ||| ||| ||| |
    AAA CGG TTG CGA C      5'
```

und den EcoRI-ClaI-Verdau von Plasmid pNM608, wie in Anspruch 10 definiert, ligiert.

**13.** Verfahren nach Anspruch 9 zur Herstellung von Plasmid pASP2, umfassend, daß man das ~10,2 kb BamHI-EcoRI-Fragment von Plasmid pCZ101, wie in Anspruch 5 definiert, einen DNA-Linker mit der Nukleotidsequenz

```
5'  CGATC ATG GAT TTT CCG GCT ATG TCT CTG TCC GGC CTG
    ||| ||| ||| ||| ||| ||| ||| ||| ||| ||| |||
3'    TAG TAC CTA AAA GGC CGA TAC AGA GAC AGG CCG GAC

    TTT GCC AAC GCT GTGCT  3'
    ||| ||| ||| ||| |
    AAA CGG TTG CGA C      5'
```

und den EcoRI-ClaI-Verdau von Plasmid pNM608, wie in Anspruch 10 definiert, ligiert.

**14.** Verfahren nach Anspruch 9 zur Herstellung von Plasmid pCZ154, umfassend, daß man das ~10,2 kb BamHI-EcoRI-Fragment von Plasmid pCZ101, wie in Anspruch 5 definiert, einen DNA-Linker mit der Nukleotidsequenz

```
5'  CGACC ATG GTT TTT CCG GCT ATG TCT CTG TCC GGC CTG
    ||| ||| ||| ||| ||| ||| ||| ||| ||| ||| ||| |||
3'    TGG TAC CAA AAA GGC CGA TAC AGA GAC AGG CCG GAC

    TTT GCC AAC GCT GTGCT   3'
    ||| ||| ||| ||| |
    AAA CGG TTG CGA C       5'
```

und den EcoRI-ClaI-Verdau von pNM608, wie in Anspruch 10 definiert, ligiert.

**15.** Verfahren nach Anspruch 9 zur Herstellung von Plasmid pCZ155, umfassend, daß man das ~10,2 kb BamHI-EcoRI-Fragment von Plasmid pCZ101, wie in Anspruch 5 definiert, einen DNA-Linker mit der Nukleotidsequenz

```
5'  CGACC ATG GCT TTT CCG GCT ATG TCT CTG TCC GTC CTG
    ||| ||| ||| ||| ||| ||| ||| ||| ||| ||| ||| |||
3'    TGG TAC CGA AAA GGC CGA TAC AGA GAC AGG CAG GAC

    TTT GCC AAC GCT GTGCT   3'
    ||| ||| ||| ||| |
    AAA CGG TTG CGA C       5'
```

EP 0 159 123 B1

und den EcoRI-ClaI-Verdau von pNM608, wie in Anspruch 10 definiert, ligiert.

16. Verfahren nach Anspruch 9 zur Herstellung von Plasmid pCZ156, umfassend, daß man das ~10,2 kb BamHI-EcoRI-Fragment von Plasmid pCZ101, wie in Anspruch 5 definiert, einen DNA-Linker mit der Nukleotidsequenz

```
5'    CGATA ATG GAT TTT CCG GCT ATG TCT CTG TCC GGC CTG
      ||| ||| ||| ||| ||| ||| ||| ||| ||| ||| ||| |||
3'        TAT TAC CTA AAA GGC CGA TAC AGA GAC AGG CCG GAC

      TTT GCC AAC GCT GTGCT      3'
      ||| ||| ||| ||| |
      AAA CGG TTG CGA C          5'
```

und den EcoRI-ClaI-Verdau von pNM608, wie in Anspruch 10 definiert, ligiert.

17. Verfahren nach Anspruch 9 zur Herstellung von Plasmid pAT2.3, umfassend, daß man das ~9,3 kb BamHI-EcoRI-Fragment von Plasmid pCZ103, einen DNA-Linker mit der Nukleotidsequenz

```
5' CTAGAGGGTATTAATA ATG TTT CCA GCT ATG TCT CTA TCT GGT
   |||||||||||||||| ||| ||| ||| ||| ||| ||| ||| ||| |||
3'        TCCCATAATTAT TAC AAA GGT CGA TAC AGA GAT AGA CCA

      CTG TTT GCC AAC GCT GTGCT   3'
      ||| ||| ||| ||| ||| |
      GAC AAA CGG TTG CGA C       5'
```

und das ~0,6 kb BamHI-XbaI-Fragment von Plasmid pCZ101 ligiert, wobei Plasmid pCZ103 konstruiert wird durch Deletion des ~900 bp BstEII-Restriktionsfragments von Plasmid pCZ101, wie in Anspruch 5 definiert, und Rezirkularisierung.

18. Verfahren nach Anspruch 9 zur Herstellung von Plasmid pAT1.3, umfassend, daß man das ~9,3 kb BamHI-EcoRI-Fragment von Plasmid pCZ103, wie in Anspruch 17 definiert, einen DNA-Linker mit der Nukleotidsequenz

```
5'    CGACA ATG TTC CCA GCT ATG TCT CTA TCT GGT
      ||| ||| ||| ||| ||| ||| ||| ||| |||
3'        TGT TAC AAG GGT CGA TAC AGA GAT AGA CCA

      CTG TTT GCC AAC GCT GTGCT   3'
      ||| ||| ||| ||| ||| |
      GAC AAA CGG TTG CGA C       5'
```

und den EcoRI-ClaI-Verdau von Plasmid pNM608, wie in Anspruch 10 definiert, ligiert.

19. Verfahren nach Anspruch 9 zur Herstellung von Plasmid pASP1.3, umfassend, daß man das ~9,3 kb BamHI-EcoRI-Fragment von Plasmid pCZ103, wie in Anspruch 17 definiert, einen DNA-Linker mit der Nukleotidsequenz

```
5'    CGACC ATG GAT TTT CCG GCT ATG TCT CTG TCC GGC CTG
      ||| ||| ||| ||| ||| ||| ||| ||| ||| ||| ||| |||
3'        TGG TAC CTA AAA GGC CGA TAC AGC GAC AGG CCG GAC

      TTT GCC AAC GCT GTGCT      3'
      ||| ||| ||| ||| |
      AAA CGG TTG CGA C          5'
```

69

und den EcoRI-ClaI-Verdau von Plasmid pNM608, wie in Anspruch 10 definiert, ligiert.

20. Verfahren nach Anspruch 9 zur Herstellung von Plasmid pASP2.3, umfassend, daß man das ~9,3 kb BamHI-EcoRI-Fragment von Plasmid pCZ103, wie in Anspruch 17 definiert, einen DNA-Linker mit der Nukleotidsequenz

```
5'   CGATC ATG GAT TTT CCG GCT ATG TCT CTG TCC GGC CTG
           ::: ::: ::: ::: ::: ::: ::: ::: ::: ::: ::: :::
3'         TAG TAC CTA AAA GGC CGA TAC AGA GAC AGG CCG GAC

     TTT GCC AAC GCT GTGCT   3'
     ::: ::: ::: ::: :
     AAA CGG TTG CGA C       5'
```

und den EcoRI-ClaI-Verdau von Plasmid pNM608, wie in Anspruch 10 definiert, ligiert.

21. Verfahren nach Anspruch 9 zur Herstellung von Plasmid pCZ154.3, umfassend, daß man das ~9,3 kb BamHI-EcoRI-Fragment von Plasmid pCZ103, wie in Anspruch 17 definiert, einen DNA-Linker mit der Nukleotidsequenz

```
5'   CGACC ATG GTT TTT CCG GCT ATG TCT CTG TCC GGC CTG
           ::: ::: ::: ::: ::: ::: ::: ::: ::: ::: ::: :::
3'         TGG TAC CAA AAA GGC CGA TAC AGA GAC AGG CCG GAC

     TTT GCC AAC GCT GTGCT    3'
     ::: ::: ::: ::: :
     AAA CGG TTG CGA C        5'
```

und den EcoRI-ClaI-Verdau von Plasmid pNM608, wie in Anspruch 10 definiert, ligiert.

22. Verfahren nach Anspruch 9 zur Herstellung von Plasmid pCZ155.3, umfassend, daß man das ~9,3 kb BamHI-EcoRI-Fragment von Plasmid pCZ103, wie in Anspruch 17 definiert, einen DNA-Linker mit der Nukleotidsequenz

```
5'   CGACC ATG GCT TTT CCG GCT ATG TCT CTG TCC GTC CTG
           ::: ::: ::: ::: ::: ::: ::: ::: ::: ::: ::: :::
3'         TGG TAC CGA AAA GGC CGA TAC AGA GAC AGG CAG GAC

     TTT GCC AAC GCT GTGCT    3'
     ::: ::: ::: ::: :
     AAA CGG TTG CGA C        5'
```

und den EcoRI-ClaI-Verdau von pNM608, wie in Anspruch 10 definiert, ligiert.

23. Verfahren nach Anspruch 9 zur Herstellung von Plasmid pCZ156.3, umfassend, daß man das ~9,3 kb BamHI-EcoRI-Fragment von Plasmid pCZ103, wie in Anspruch 17 definiert, einen DNA-Linker mit der Nukleotidsequenz

70

```
5'   CGATA ATG GAT TTT CCG GCT ATG TCT CTG TCC GGC CTG
     ııı ııı ııı ııı ııı ııı ııı ııı ııı ııı ııı ııı
3'      TAT TAC CTA AAA GGC CGA TAC AGA GAC AGG CCG GAC

     TTT GCC AAC GCT GTGCT    3'
     ııı ııı ııı ııı ı
     AAA CGG TTG CGA C        5'
```

und den EcoRI-ClaI-Verdau von pNM608, wie in Anspruch 10 definiert, ligiert.

24. Verfahren zur Herstellung eines Plasmids, das geeignet ist zur Verwendung in dem Verfahren nach einem der Ansprüche 8 und 17 bis 23, umfassend, daß man das Plasmid pCZ101, wie in Anspruch 5 definiert, mit BstEII-Restriktionsenzym behandelt, die so erhaltene DNA reinigt und die gereinigte DNA ligiert zur Herstellung von Plasmid pCZ103.

25. Verfahren zur Erhöhung der Milchproduktion bei Kühen, umfassend, daß man den Kühen ein Rinderwachstumshormonderivat, das von dem Gen, das in dem Vektor, der mit dem Verfahren nach einem der Ansprüche 1 bis 23 hergestellt wird, enthalten ist, kodiert wird, mit Ausnahme von Methionylrinderwachstumshormon, in einer Menge, die wirksam ist zur Erhöhung der Produktion, verabreicht.

26. Verfahren nach Anspruch 25, worin die wirksame Menge etwa 0,05 bis etwa 100000 $\mu$g pro Tier pro Tag ist.

27. Verfahren nach Anspruch 26, worin die wirksame Menge etwa 5 bis etwa 25000 $\mu$g pro Tier pro Tag ist.

28. Verfahren nach Anspruch 27, worin die wirksame Menge etwa 500 bis etwa 5000 $\mu$g pro Tier pro Tag ist.

# FIG.I

# FIG.2

Plasmid 103 (Figure 1)

Hind III

S1 Nuclease

Bam HI Linkers
$T_4$ DNA Ligase

Bam HI

$T_4$ DNA Ligase

Eco RI

Ippρ

Xba I

Eco RI

Bam HI

Sal I

$Ap^R$

104

$Tc^s$

Eco RI Partial Digestion

S1 Nuclease

Hind III Linkers
$T_4$ DNA Ligase

Hind III

$T_4$ DNA Ligase

Hind III

Ippρ

$Ap^R$

105

Xba I

Eco RI

Bam HI

Sal I

# FIG.3

**Plasmid 105 (Figure 2)**

↓ Sal I

↓ Bam HI

↓ Alkaline Phosphatase

**Plasmid pKEN111 (101 in Figure 1)**

↓ Hpa I

↓ Sal I Linkers T₄ DNA Ligase

↓ Sal I

↓ Pvu II

↓ Bam HI Linkers T₄ DNA Ligase

↓ Bam HI

≈ 950bp Fragment Sal I, Bam HI Ends

pKEN021

Hind III

Ipp^p

Ap^R

**106**

Xba I
Eco RI
Bam HI

3' Ipp

Bam HI
Xba I
Alkaline Phosphatase

Sal I

Hind III

Ipp^p

Ap^R

**107**

Xba I

Bam HI

Sal I

# FIG.4

ptrp ED50chGH800

Plasmid pBR322
(102 in Figure 1)

pNM575

# FIG. 5

**Plasmid pNM575**
**(109 in Figure 4)**

↓ **Bam HI**

**691 bp Bam HI**
**Fragment**

↓ **Fnu DII**

**538 bp Bam HI,**
**Fnu DII Fragment**

**110**

Xba I                    Fnu DII

**Synthetic**
**Fragment**

**Plasmid pKEN021**
**Xba I – Bam HI –**
**digested**
**(107 in Figure 3)**

**T₄ DNA Ligase**

pNM702

Hind III

Ipp^p          Xba I

Ap^R

Fnu DII
Pvu II

**111**

Pvu II
Bam HI

Pvu II

Sal I

# FIG.6

pBP348

Pst I

Hpa II

Pvu II

Eco RI

Sma I

Tc$^R$

Pvu II

Pst I

**112**

Plasmid pNM702
(111 in Figure 5)

Pvu II Partial
Digestion

Pvu II

494 bp Fragment

Hind III

Ipp$^p$

Xba I

Ap$^R$

Pvu II

**113**

Pvu II

Pvu II

3'Ipp

Sal I

T$_4$ DNA Ligase

Hind III

Ipp$^p$

Ap$^R$

Xba I

Pvu II

pNM685

**114**

Sma I

Pvu II

Bam HI

Pvu II

3'Ipp

Sal I

# FIG.7

**114 (in Figure 6)**

| Pvu II Partial Digestion
| Xba I
| Alkaline Phosphatase

**116**

Xba I ══════▨▨▨ Hpa II
Synthetic Fragment

Hind III

*Ipp<sub>P</sub>*

Xba I

Ap<sup>R</sup>

**115**

Pvu II

Pvu II

Bovine Growth Hormone

Bam HI

Sal I

pPB348
(112 in Figure 6)

| Pvu II
| Pst I

135 bp Fragment

| Hpa II Partial Digestion

46 bp Fragment

pNM789

Hind III

*Ipp<sub>P</sub>*

Ap<sup>R</sup>

**117**

Xba I
Hpa II
Pvu II

Pvu II
Bam HI

3' Ipp

Pvu II

Sal I

# FIG.8

Plasmid pNM789
(117 in Figure 7)

↓ Pvu II Partial Digestion

↓ Bam HI

↓ Alkaline Phosphatase

118

Pvu II    Bam HI

Synthetic Fragment

Hind III

Ippₚ

Xba I

Pvu II

Apᴿ

119

Pvu II

3'Ipp   Bam HI

Sal I

T₄ DNA Ligase

Hind III

Ippₚ

Xba I

Apᴿ

120

Pvu II

Coding Sequence for Bovine Growth Hormone

Pvu II

Bam HI

3'Ipp

pNM789B

Sal I

# FIG. 9
## Restriction Site Map of Plasmids pCZ1920 and pJR1

pCZ 1920

# FIG. 10
## Restriction Site Map of Plasmid pCZ112

# FIG. 11
## Thymosin Alpha I Gene

|  | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 |
|---|---|---|---|---|---|---|---|---|---|----|----|----|----|----|----|----|----|----|----|----|----|----|----|----|----|----|----|----|
| Met | Ser | Asp | Ala | Ala | Val | Asp | Thr | Ser | Ser | Glu | Ile | Thr | Thr | Lys | Asp | Leu | Lys | Glu | Lys | Lys | Glu | Val | Val | Glu | Glu | Ala | Glu | Asn |

Eco RI

stop stop

$\leftarrow$T$_1$$\rightarrow$  $\leftarrow$T$_2$$\rightarrow$  $\leftarrow$T$_3$$\rightarrow$  $\leftarrow$T$_4$$\rightarrow$  $\leftarrow$T$_9$$\rightarrow$  $\leftarrow$T$_{10}$$\rightarrow$  $\leftarrow$T$_{11}$$\rightarrow$  $\leftarrow$T$_{12}$$\rightarrow$

AATTCATGTCTGATGCTGCTGTTGATACTTCTTCTGAGATTACTACTAAAGATCTTAAGGAGAAGAAGGAAGTTGTCGAAGAGGCTGAGAACTAATAG

GTACAGACTACGACGACAACTATGAAGAAGACTCTAATGATGATTTCTAGAATTCCTCTTCTTCCTTCAACAGCTTCTCCGACTCTTGATTATCCTAG

$\leftarrow$T$_5$$\rightarrow$  $\leftarrow$T$_6$$\rightarrow$  $\leftarrow$T$_7$$\rightarrow$  $\leftarrow$T$_8$$\rightarrow$  $\leftarrow$T$_{13}$$\rightarrow$  $\leftarrow$T$_{14}$$\rightarrow$  $\leftarrow$T$_{15}$$\rightarrow$  $\leftarrow$T$_{16}$$\rightarrow$

Bgl II

Bam HI

EP 0 159 123 B1

# FIG.12
## Synthesis Procedure for Fragment T₁₅

EP 0 159 123 B1

# FIG.13
## Construction Route for Plasmid pTh α 1

EP 0 159 123 B1

# FIG.14
## Proinsulin Synthetic Gene

|  | 1 |  |  |  |  |  |  |  |  | 10 |  |  |  |  |  |  |  |  | 20 |
|---|---|---|---|---|---|---|---|---|---|---|---|---|---|---|---|---|---|---|---|
| met | phe | val | asn | gln | his | leu | cys | gly | ser | his | leu | val | glu | ala | leu | tyr | leu | val | cys | gly |

```
    ┌──────H1──────┐  ┌────────H2────────┐  ┌─────────H3─────────┐  ┌─────────H4─────────┐  ┌────────B1────────┐  ┌────────B2────────┐
  AATTC  ATG  TTC  GTC  AAT  CAG  CAC  CTT  TGT  GGT  TCT  CAC  CTC  GTT  GAA  GCT  TTG  TAC  CTT  GTT  TGC  GGT
     G   TAC  AAG  CAG  TTA  GTC  GTG  GAA  ACA  CCA  AGA  GTG  GAG  CAA  CTT  CGA  AAC  ATG  GAA  CAA  ACG  CCA
    └──────────H5──────────┘  └────────H6────────┘  └────────H7────────┘  └────────H8────────┘  └────────B6────────┘
```

|  |  |  |  |  |  |  |  |  | 30 |  |  |  |  |  |  |  |  |  | 40 |  |  |  |
|---|---|---|---|---|---|---|---|---|---|---|---|---|---|---|---|---|---|---|---|---|---|---|---|
| glu | arg | gly | phe | phe | tyr | thr | pro | lys | thr | arg | arg | glu | ala | glu | asp | leu | gln | val | gly | gln | val | glu |

```
  ┌──────B3──────┐  ┌────────B4────────┐  ┌──────B5'──────┐
  GAA  CGT  GGT  TTC  TTC  TAC  ACT  CCT  AAG  ACT  CGC │CGG  GAG  GCA  GAG  GAC  CTG  CAG  GTG  GGG  CAG  GTG  GAG
  CTT  GCA  CCA  AAG  AAG  ATG  TGA  GGA  TTC  TGA  GCG  GCC│ CTC  CGT  CTC  CTG  GAC  GTC  CAC  CCC  GTC  CAC  CTC
  ──B7──┘  └────B8────┘  └────────B9────────┘  └──B10'──┘
```

|  |  |  |  | 50 |  |  |  |  |  |  |  |  |  |  |  | 60 |  |  |  |  |  |  |
|---|---|---|---|---|---|---|---|---|---|---|---|---|---|---|---|---|---|---|---|---|---|---|
| leu | gly | gly | gly | pro | gly | ala | gly | ser | leu | gln | pro | leu | ala | leu | glu | gly | ser | leu | gln | lys | arg | gly |

```
  CTG  GGC  GGG  GGC  CCT  GGT  GCA  GGC  AGC  CTG  CAG  CCC  TTG  GCC  CTG  GAG  GGG  TCC  CTG  CAG  AAG  CGT  GGC
  GAC  CCG  CCC  CCG  GGA  CCA  CGT  CCG  TCG  GAC  GTC  GGG  AAC  CGG  GAC  CTC  CCC  AGG  GAC  GTC  TTC  GCA  CCG
```

|  |  |  | 70 |  |  |  |  |  |  |  |  |  | 80 |  |  |  |  |  |  |
|---|---|---|---|---|---|---|---|---|---|---|---|---|---|---|---|---|---|---|---|
| ile | val | glu | gln | cys | cys | thr | ser | ile | cys | ser | leu | tyr | gln | leu | glu | asn | tyr | cys | asn |

```
  ATT  GTG  GAA  CAA  TGC  TGT  ACC  AGC  ATC  TGC  TCC  CTC  TAC  CAG  CTG  GAG  AAC  TAC  TGC  AAC  TAG  ACGCAGC
  TAA  CAC  CTT  GTT  ACG  ACA  TGG  TCG  TAG  ACG  AGG  GAG  ATG  GTC  GAC  CTC  TTG  ATG  ACG  TTG  ATC  TGCGTCG
```

```
CCGCAGGCAGCCCCACACCCGCCGCCTCCTGCACCGAGAGAGATGGAATAAAGCCCTTGAACCAGC · poly A · CCG
GGCGTCCGTCGGGGTGTGGGCGGCGGAGGACGTGGCTCTCTCTACCTTATTTCGGGAACTTGGTCG · poly T · GGCCTAG
```

Amino acids 1-30 are the B chain; 31-65 are the C chain; and 66-86 are the A chain.

# FIG. 15
## Construction of Plasmid pHI 7△4△1

# FIG.16

## Construction of Plasmid pHI104

EP 0 159 123 B1